(19) Europäisches Patentamt
European Patent Office
Office européen des brevets

(11) EP 2 925 884 B1

(12) **EUROPEAN PATENT SPECIFICATION**

(45) Date of publication and mention of the grant of the patent:
**31.01.2018 Bulletin 2018/05**

(21) Application number: **13802567.1**

(22) Date of filing: **27.11.2013**

(51) Int Cl.:
***C12Q 1/68*** *(2018.01)*

(86) International application number:
**PCT/EP2013/074907**

(87) International publication number:
**WO 2014/083081 (05.06.2014 Gazette 2014/23)**

(54) **COMPOSITIONS AND METHODS FOR EVALUATING HEART FAILURE**

ZUSAMMENSETZUNGEN UND VERFAHREN ZUR BEWERTUNG VON HERZFEHLERN

COMPOSITIONS ET PROCÉDÉS D'ÉVALUATION D'UNE INSUFFISANCE CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2012 LU 92106**
**27.11.2012 US 201261730459 P**

(43) Date of publication of application:
**07.10.2015 Bulletin 2015/41**

(73) Proprietor: **Luxembourg Institute of Health**
**1445 Strassen (LU)**

(72) Inventors:
- **DEVAUX, Yvan**
  **F-57330 Zoufftgen (FR)**
- **VAUSORT, Mélanie**
  **L-7481 Tuntange (LU)**
- **ZHANG, Lu**
  **F-57000 Metz (FR)**
- **WAGNER, Daniel**
  **L-8083 Bertrange (LU)**
- **SQUIRE, Iain**
  **Newton Harcourt**
  **Leicestershire LE8 9FH (GB)**

(74) Representative: **Paemen, Liesbet R.J.**
**De Clercq & Partners cvba**
**Edgard Gevaertdreef 10 a**
**9830 Sint-Martens-Latem (BE)**

(56) References cited:
**WO-A2-2008/042231** **WO-A2-2008/043521**

- **CREEMERS ESTHER E ET AL: "Circulating MicroRNAs Novel Biomarkers and Extracellular Communicators in Cardiovascular Disease?", CIRCULATION RESEARCH, vol. 110, no. 3, February 2012 (2012-02), pages 483-495, XP009166249,**
- **DI STEFANO VALERIA ET AL: "microRNAs as peripheral blood biomarkers of cardiovascular disease", VASCULAR PHARMACOLOGY, vol. 55, no. 4, Sp. Iss. SI, October 2011 (2011-10), pages 111-118, XP055049884,**
- **TALWAR S ET AL: "Profile of plasma N-terminal proBNP following acute myocardial infarction: Correlation with left ventricular systolic dysfunction", EUROPEAN HEART JOURNAL, vol. 21, no. 18, September 2000 (2000-09), pages 1514-1521, XP055049880, ISSN: 0195-668X cited in the application**
- **YVAN DEVAUX ET AL: "A Panel of 4 microRNAs Facilitates the Prediction of Left Ventricular Contractility after Acute Myocardial Infarction", PLOS ONE, vol. 8, no. 8, 13 August 2013 (2013-08-13) , page e70644, XP55097670, DOI: 10.1371/journal.pone.0070644**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the invention

The present invention relates to compositions and kits comprising probes for detecting miRNAs useful for monitoring the diagnosis or progression of heart disease in an individual. In particular the compositions of the invention can be used for the prognosis of patients having suffered from an acute myocardial infarction.

### Introduction to the invention

Heart disease encompasses a family of disorders, such as cardiomyopathies, and is a leading cause of morbidity and mortality in the industrialized world. Disorders within the heart disease spectrum are understood to arise from pathogenic changes in distinct cell types, such as cardiomyocytes, via alterations in a complex set of biochemical pathways. Left ventricular (LV) remodelling develops after acute myocardial infarction (AMI) in a significant proportion of patients [1]. Associated mortality and morbidity are important and may be prevented or at least alleviated by personalized health care. To achieve this goal, however, it is critical to identify new tools to accurately predict the development of LV remodelling. Whereas N-terminal pro-brain natriuretic peptide (Nt-pro-BNP) is known to be associated with LV dysfunction after AMI, it fluctuates after AMI and better predicts poor outcome when measured 3-5 days after AMI [2]. Talwar S et al (2000) Eur. Heart J. 21:1514-1521 showed that Nt-pro-BNP was an independent predictor of wall motion index score (WMIS), an indicator of LV contractility and remodelling.

Since the discovery of their stability in the bloodstream [3, 4], microRNAs (miRNAs), short oligonucleotides which down-regulate gene expression, have been the focus of a plethora of biomarker studies. Their potential to diagnose AMI has been suggested by multiple reports [5],[6]. However, their prognostic value has received much less attention, and only cardiomyocytes-enriched miRNAs have been evaluated [7] and WO2008042231. Interestingly, the temporal profile of circulating miRNAs is related to the development of LV remodelling after AMI[8], which suggests their usefulness as prognostic biomarkers.

Creemers, Esther E. et al: Circulation Research, Vol. 110, no. 3, February 2012 (2012-02), pages 483-495 discloses various miRNAs in connection with evaluating various cardiovascular diseases.

WO 2008/043521 discloses a large number of miRNAs, including those of the present invention, for evaluating and treating a cardiac disease.

Di Stefano, Valeria et al: Vascular Pharmacology, Vol. 55, no. 4, sp. ISS. Si, (2011-10), pages 111-118 discloses various miRNAs as markers.

WO 2008/042231 discloses a list of microRNAs, including miR-101 and miR-27a, as suitable markers for evaluating heart diseases.

### Summary of the invention

In the present invention, we have shown that a group of 4 miRNAs, miR-16 encoded by SEQ ID NO:1, miR-27a encoded by SEQ ID NO:2, miR-101, encoded by SEQ ID NO:3, miR-150 encoded by SEQ ID NO:4, (i.e. further indicated as the miRNA panel of the invention) can add to the predictive value (or prognostic value) of the existing marker, i.e. Nt-pro-BNP, in a prospective cohort of AMI patients. The potential of the miRNA panel was shown to aid in the prognostication of patients having suffered from acute myocardial infarction.

The four miRNAs of the present invention were selected from a pool of 695 possible miRNAs and, surprisingly, it has been found that only these four, in specific combination, are able to enhance the prognosis of left ventricular remodelling, preferably in combination with Nt-pro-BNP.

In a particular aspect, the present invention shows an added value of the 4 miRNA panel to Nt-pro-BNP as shown in SEQ ID NO:5, to classify patients which have suffered from myocardial infarction. In particular, the sensitivity of the prediction was improved, and the specificity was preserved.In a particular aspect the invention provides a kit for monitoring the prognosis of a patient having suffered from acute myocardial ischemia consisting of (1) probes for measuring a panel of miRNA biomarkers in a sample of bodily fluid of said patient, said panel of miRNA biomarkers consisting of: (a) miR-16 encoded by SEQ ID NO:1, (b) miR-27a encoded by SEQ ID NO:2, (c) miR-101 encoded by SEQ ID NO:3, and (d) miR-150 encoded by SEQ ID NO:4, and optionally (2) detection reagents for measuring Nt-pro-BNP as shown in SEQ ID NO:5 in a sample of bodily fluid of said patient. In particular embodiments, said probes for measuring said panel of miRNAs are suitable for detection by quantitative PCR and said detection reagents for measuring said Nt-proBNP level are suitable for detection by immunoassay.

In a particular aspect the invention provides said kit for detecting said biomarker panel consisting of miR-16, miR-27a, miR-101 and miR-150 for monitoring the prognosis of a patient having suffered from acute myocardial ischemia.

In yet another aspect the invention provides the kit for detecting said biomarker panel consisting of miR-16, miR-27a, miR-101 and miR-150 and Nt-pro-BNP for monitoring the prognosis of a patient having suffered from acute myocardial ischemia.

In yet another aspect the invention provides the use of a kit for detecting a biomarker panel consisting of miR-16, miR-27a, miR-101 and miR-150 for monitoring the prognosis of a patient having suffered from acute myocardial ischemia.

In yet another aspect the invention provides the use of a kit for detecting said biomarker panel consisting of miR-16, miR-27a, miR-101 and miR-150 and Nt-pro-BNP for monitoring the prognosis of a patient having suffered from acute myocardial ischemia.

In still another aspect the invention provides a method for predicting and/or monitoring the prognosis of a patient having suffered from an acute myocardial infarction comprising determining the levels of miR-16, miR-27a, miR-101 and miR-150 in a body fluid of said patient and correlating the levels of said miRNAs with a previously established classification model wherein said model was developed by fitting data from a study of a population of patients and said fitted data comprises levels of said biomarkers and conversion to the development of left ventricular remodeling in said selected population of patients and optionally also determining an increase in levels of Nt-pro-BNP by comparison with the control and wherein a prognostic score is obtained for being at risk of developing left ventricular modeling by establishing the odds ratios of only said miRNAs optionally in combination with levels of Nt-pro-BNP.

In still another aspect the invention provides a method for predicting and/or monitoring the prognosis of a patient having suffered from an acute myocardial infarction comprising determining the levels of miR-16, miR-27a, miR-101, miR-150 and Nt-pro-BNP in a body fluid of said patient and correlating the levels of said miRNAs and Nt-pro-BNP with a previously established classification model wherein said model was developed by fitting data from a study of a population of patients and said fitted data comprises levels of said biomarkers and conversion to the development of left ventricular remodeling in said selected population of patients and optionally also determining an increase in levels of Nt-pro-BNP by comparison with the control and wherein a prognostic score is obtained for being at risk of developing left ventricular modeling by establishing the odds ratios of only said miRNAs optionally in combination with levels of Nt-pro-BNP.

In particular aspect the patient having suffered from an acute myocardial infarction has a WMIS score between 1 and 1.4.

In yet another aspect the invention provides a method for assessing the efficacy of a treatment for a patient having suffered from an acute myocardial infarction and having a likelihood of developing a reduced LV contractility wherein the method comprises i) determining the levels of miR-16, miR-27a, miR-101 and miR-150 in a body fluid of said patient, ii) determining the Nt-pro-BNP level in a body fluid of said patient, iii) determining the levels of miR-16, miR-27a, miR-101 and miR-150 and the level of Nt-pro-BNP in a body fluid of said patient after treatment, iv) comparing the results of i) and ii) with the results of iii), wherein a difference between the results of i), ii) and iii) indicates an effect of the treatment.

In a particular aspect a patient has a WMIS score between 1 and 1.4.

In still other particular aspects the body fluid is blood, plasma or serum.

The application further discloses a composition of i) at least one short interfering nucleic acid capable of encoding a miRNA selected from the list consisting of miR-101 and miR-150 and at least one short interfering nucleic acid capable of inhibiting a miRNA selected from the list consisting of miR-16 and miR-27a or ii) short interfering nucleic acids capable of encoding miR-101 and miR-150 or iii) short interfering nucleic acids capable of inhibiting miR-16 and miR-27a for the treatment of left ventricular remodeling.

The application further discloses pharmaceutical formulations comprising the previous compositions.

Figures

Figure 1: Risk estimates for clinical parameters, Nt-pro-BNP and miRNAs. Nt-pro-BNP and miRNAs were measured at discharge from the hospital and LV contractility was evaluated by WMIS at 6 months follow-up. Censored regression models were built to determine the risk of impaired LV contractility. A. Model 1 is a multivariable model including indicated clinical parameters and Nt-pro-BNP. B. Model 2 is a multivariable model including the variables of model 1 and the expression values of miR-16/27a/101/150. CI: confidence interval; OR: odd ratio.

Figure 2: Bootstrap internal validation (censored regression). Represented is the number of times that a combination of miRNAs was selected as providing the best improvement of the prediction of model 1. Data are expressed as percentage of the number of selection relative to 150 iterations.

Figure 3: Risk estimates obtained by logistic regression. Nt-pro-BNP and miRNAs were measured at discharge and LV contractility was evaluated by WMIS at follow-up. Patients were dichotomized according to WMIS using a threshold value of 1.2. Patients with WMIS ≤ 1.2 had preserved LV contractility (n=79) and patients with WMIS > 1.2 had impaired LV contractility (n=71). Logistic regression models were built to determine the risk of impaired LV contractility. A. Model 3 is a multivariable model including indicated clinical parameters and Nt-pro-BNP. B. Model 4 is a multivariable model including the variables of model 1 and the expression values of miR-16/27a/101/150. CI: confidence interval; OR: odd ratio. Note: X axis is in log scale.

Figure 4: Bootstrap internal validation (logistic regression). Represented is the number of times that a combination of miRNAs was selected as providing the best improvement of the prediction of model 3. Data are expressed as percentage of the number of selection relative to 150 iterations.

Figure 5 shows systems-based identification of candidate miRNAs. A. Network of interactions between proteins known to be associated with LV remodelling in humans (dark grey nodes) and 26 interacting proteins (light grey).

Figure 6 shows expression of differentiation-related genes in early endothelial progenitor cells treated by anti-miR-16.

## Detailed description of the invention

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., Current Protocols in Molecular Biology (Supplement 100), John Wiley & Sons, New York (2012), for definitions and terms of the art. The definitions provided herein should not be construed to have a scope less than understood by a person of ordinary skill in the art.

In the present invention we demonstrate the prognostic value of an assay comprising a panel of 4 different miRNAs in AMI patients. In particular, the combination of a panel of 4 specific miRNAs (i.e. miR-16, miR-27a, miR-101 and miR-150) and the determination of Nt-pro-BNP is found to improve the prognostic value of the gold standard Nt-pro-BNP as a stand-alone prognostic marker. The method of the invention increases the sensitivity from 48 to 60%, while maintaining the specificity at 75%. In addition, the positive predictive value was increased form 67% to 71%, and the negative predictive value was increased form 58% to 64%. One particular advantage of the invention is that the method for prognosis also improves the classification of patients with intermediate phenotypes, particularly dyskinetic patients, which are difficult to classify using existing biomarkers.

Accordingly, the application discloses a biomarker panel consisting of miR-16, miR-27a, miR-101 and miR-150 and the invention provides in a first embodiment a kit comprising the probes detecting said panel for monitoring the prognosis of a patient having suffered from acute myocardial ischemia.

Also disclosed herein a biomarker panel is provided consisting of miR-16, miR-27a, miR-101, miR-150 and Nt-pro-BNP and a further embodiment of the invention provides a kit comprising probes and/or reagents detecting said panel for monitoring the prognosis of a patient having suffered from acute myocardial ischemia.

In yet another embodiment the invention provides the use of said kits for detecting a biomarker panel consisting of miR-16, miR-27a, miR-101 and miR-150 for monitoring the prognosis of a patient having suffered from acute myocardial ischemia.

In yet another embodiment the invention provides the use of said kits for detecting a biomarker panel consisting of miR-16, miR-27a, miR-101, miR-150 and Nt-pro-BNP for monitoring the prognosis of a patient having suffered from acute myocardial ischemia.

In yet another embodiment the invention provides a method for predicting and/or monitoring the prognosis of a patient having suffered from an acute myocardial infarction comprising determining the levels of miR-16, miR-27a, miR-101 and miR-150 in a body fluid of said patient and correlating the levels of said miRNAs with a previously established classification model wherein said model was developed by fitting data from a study of a population of patients and said fitted data comprises levels of said biomarkers and conversion to the development of left ventricular remodeling in said selected population of patients, and optionally also determining an increase in levels of Nt-pro-BNP by comparison with the control, and wherein a prognostic score is obtained for being at risk of developing left ventricular modeling by establishing the odds ratios of only said miRNAs optionally in combination with levels of Nt-pro-BNP.

In yet another embodiment the invention provides a method for predicting and/or monitoring the prognosis of a patient having suffered from an acute myocardial infarction comprising determining the levels of miR-16, miR-27a, miR-101 and miR-150 in a body fluid of said patient and correlating the levels of said miRNAs with a previously established classification model wherein said model was developed by fitting data from a study of a population of patients and said fitted data comprises levels of said biomarkers and conversion to the development of left ventricular remodeling in said selected population of patients, and optionally also determining an increase in levels of Nt-pro-BNP by comparison with the control, and wherein a prognostic score is obtained for being at risk of developing left ventricular modeling by establishing the odds ratios of only said miRNAs optionally in combination with levels of Nt-pro-BNP and wherein a practitioner may start a treatment plan based on the prognostic score.

In a particular embodiment the treatment plan involves the administration of a drug, such as an ACE inhibitor, an angiotensin II receptor blocker, a Beta-blocker, a vasodilator, a pro-angiogenic factor, a cardiac glycoside, an antiarrhythmic agent, a diuretic, a statin, or an anticoagulant, an inotropic agent; an immunosuppressive agent, use of a pacemaker, defibrillator, mechanical circulatory support, surgery, or therapy with stem cells (bone marrow derived stem cells, mesenchymal stem cells, cardiac stem cells, muscle derived stem cells).

The wording "for being at risk of developing left ventricular modeling" is equivalent to the wording "for being at risk of developing a reduced left ventricular contractility".

In yet another embodiment the invention provides a method for predicting and/or monitoring the prognosis of a patient having suffered from an acute myocardial infarction comprising: i) determining the levels of miR-16, miR-27a, miR-101 and miR-150 in a body fluid of said patient, ii) determining the Nt-pro-BNP level in a body fluid of said patient wherein the levels of said miRNAs and said Nt-pro-BNP level is correlated with a previously established classification model wherein said model was developed by fitting data from a study of a population of patients and said fitted data comprises levels of said biomarkers and conversion to the development of left ventricular remodeling in said selected population of patients and wherein a prognostic score is obtained for being at risk of developing left ventricular modeling.

In a particular embodiment the body fluid for measuring the levels of Nt-pro-BNP and the body fluid for measuring the levels of miR-16, miR-27a, miR-101 and miR-150 are different body fluids.

In a particular embodiment a body fluid is blood, serum, plasma, Cerebro Spinal Fluid (CSF), saliva or urine.

In a preferred embodiment the body fluid is blood, serum or plasma.

In particular embodiments the body fluid of a patient having suffered from an acute myocardial infarction is sampled after 5 minutes, 10 minutes, 60 minutes, 2 hours, 12 hours, 1 day, 2 days, 3 days, 4 days, 5 days or after even a longer period. In a particular embodiment the body fluid is sampled at any time point between 5 minutes and 4 weeks after the acute myocardial infarction.

Methods of plasma and serum preparation are well known in the art. Either "fresh" blood plasma or serum, or frozen (stored) and subsequently thawed plasma or serum may be used. Frozen (stored) plasma or serum should optimally be maintained at storage conditions of -20 to -70°C until thawed and used. "Fresh" plasma or serum should be refrigerated or maintained on ice until used, with nucleic acid extraction being performed as soon as possible. Blood can be drawn by standard methods into a collection tube, typically siliconized glass, either without anticoagulant for preparation of serum, or with EDTA, sodium citrate, heparin, or similar anticoagulants for preparation of plasma. When preparing plasma or serum for storage, although not an absolute requirement, is that plasma or serum is first fractionated from whole blood prior to being frozen. This reduces the burden of extraneous intracellular RNA released from lysis of frozen and thawed cells which might reduce the sensitivity of the amplification assay or interfere with the amplification assay through release of inhibitors to PCR such as porphyrins and hematin. "Fresh" plasma or serum may be fractionated from whole blood by centrifugation, using gentle centrifugation at 300-800 times gravity for five to ten minutes, or fractionated by other standard methods. High centrifugation rates capable of fractionating out apoptotic bodies should be avoided. Since heparin may interfere with RT-PCR, use of heparinized blood may require pretreatment with heparanase, followed by removal of calcium prior to reverse transcription. Imai, H. et al (1992) J. Virol. Methods 36:181-184.

An AMI patient is a patient who has suffered from an acute myocardial infarction.

In the present invention the classification model is established with patients who have suffered from an acute myocardial infarction. Typically, for establishing the model patients are recruited who developed left ventricular remodeling and patients who did not develop left ventricular remodeling.

The wording "a method for predicting and/or monitoring the prognosis" as used herein refers to methods by which the skilled artisan can predict the course or outcome of a condition in a patient. The term "prognosis" does not refer to the ability to predict the course or outcome of a condition with 100% accuracy, or even that a given course or outcome is more likely to occur than not. Instead, the skilled artisan will understand that the term "prognosis" refers to an increased probability that a certain course or outcome will occur; that is, that a course or outcome is more likely to occur in a patient exhibiting a given characteristic, such as the presence or level of a prognostic indicator, when compared to those individuals not exhibiting the characteristic. For example, as described hereinafter, an AMI patient exhibiting a high level of miR-16 and mi-R27a and a low level of miR-150 and miR-101 and an increased level of Nt-pro-BNP, as compared to a mean value determined in a population of patients included in the classification model, may be more likely to suffer or to progress towards a patient with an impaired LV contractility. In preferred embodiments, a prognosis is about a 5% chance of a given outcome, about a 7% chance, about a 10% chance, about a 12% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/- 1%.

The skilled artisan will understand that associating a prognostic indicator with a predisposition to an outcome of reduced LV contractility is a statistical analysis. For example, changes in the miRNA panel as described herein in combination with a change in the amount of Nt-pro-BNP may signal that a patient, in particular an AMI patient, is more likely to suffer from an adverse outcome than patients with different levels, as determined by a level of statistical significance. Common tests for evaluating statistical significance include but are not limited to ANOVA, Kniskal-Wallis, t-test and odds ratio (OR). Statistical significance is often determined by comparing two or more populations, and determining a confidence interval (CI) and/or a p value. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001. Exemplary statistical tests for associating a prognostic indicator with a predisposition to an adverse outcome are described hereinafter.

The term "correlating," as used herein in reference to the use of prognostic indicators to determine a prognosis, refers to comparing the presence or amount of the prognostic indicator in a patient to its presence or amount in persons known to suffer from, or known to be at risk of, a given condition; or in persons known to be free of a given condition. For example, the miRNA panel levels in a patient can be compared to a level known to be associated with an increased disposition of developing an impaired left ventricular contractility. The patient's miRNA panel levels are said to have been correlated with a prognosis; that is, the skilled artisan can use the patient's miRNA panel levels, optionally in combination with the determination of the Nt-pro-BNP levels, to determine the likelihood that the patient is at risk for developing impaired LV contractility or dyskinesia, and respond accordingly. Alternatively, the patient's miRNA panel levels can be compared to a miRNA panel level known to be associated with a good outcome (e.g., no impaired LV contractility, no risk for sudden death, etc.), and determine if the patient's prognosis is predisposed to the good outcome.

As used herein, expression pattern refers to the combination of occurrences or levels in a set of miRNAs of a sample. In assessing the similarity of two expression patterns, for example, a test expression pattern and a reference expression pattern, a comparison is made between the occurrences or levels of the same miRNAs in the test and reference (or control) expression patterns for each of the four miRNA pairs. In one embodiment the classification scheme involves building or constructing a statistical model also referred to as a classifier or predictor, that can be used to classify samples to be tested (test samples) based on miRNA levels or occurrences. The model is built using reference samples (control samples) for which the classification has already been ascertained, referred to herein as a reference dataset comprising reference expression patterns. Hence, reference expression patterns are levels or occurrences of a set of one or more miRNAs in a reference sample (e.g. a reference blood or plasma or serum sample). Once the model (classifier) is built, then a test expression pattern obtained from a test sample is evaluated against the model (e.g. classified as a function of relative miRNAs expression of the sample with respect to that of the model). In some embodiments, evaluation involves identifying the reference expression pattern that most closely resembles the expression pattern of the test sample and associating the known reduced left ventricular contractility class or type of the reference expression pattern with the test expression pattern, thereby classify (categorizing) the risk towards developing a reduced left ventricular contractility associated with the test expression pattern. The number of relevant miRNAs to be used for building the model can be determined by one of skill in the art. In one embodiment, a greedy search method (backward selection) with Support Vector Machine is used to determine a subset of miRNAs that can be chosen to build a model (e.g., Naive Bayes and Logistic regression) for prediction of the presence of left ventricular contractility reduction. A class prediction strength can also be measured to determine the degree of confidence with which the model classifies a sample to be tested. The prediction strength conveys the degree of confidence of the classification of the sample and evaluates when a sample cannot be classified. There may be instances in which a sample is tested, but does not belong to a particular class. This is done by utilizing a threshold wherein a sample which scores below the determined threshold is not a sample that can be classified (e.g., a "no call"). The prediction strength threshold can be determined by the skilled artisan based on known factors, including, but not limited to the value of a false positive classification versus a "no call." Once a model is built, the validity of the model can be tested using methods known in the art. One way to test the validity of the model is by cross-validation of the dataset. To perform cross-validation, one of the samples is eliminated and the model is built, as described above, without the eliminated sample, forming a "cross-validation model." The eliminated sample is then classified according to the model, as described herein. This process is done with all the samples of the initial dataset and an error rate is determined. The accuracy of the model is then assessed. This model classifies samples to be tested with high accuracy for classes that are known, or classes that have been previously ascertained or established through class discovery as discussed herein. Another way to validate the model is to apply the model to an independent data set, such as a new unknown test plasma or blood or serum sample. Other standard biological or medical research techniques, known or developed in the future, can be used to validate class discovery or class prediction.

Classification of the sample gives a healthcare provider information about a classification to which the sample belongs, based on the analysis of the levels of the miRNA panel of the invention, optionally including the determination of Nt-pro-BNP levels. The information provided by the present invention, alone or in conjunction with other test results, aids the healthcare provider in diagnosing the individual. Also, the present invention provides methods for determining a treatment plan. Once the health care provider knows to which disease class (i.e. being at risk for developing LV remodeling or not) the sample, and therefore, the individual belongs, the health care provider can determine an adequate treatment plan for the individual. For example, different assessments of left ventricular contractility reduction often require differing treatments. Properly diagnosing and understanding the seriousness of left ventricular remodeling of an individual allows for a better, more successful treatment and prognosis. Other applications of the invention include classifying persons who are likely to have successful treatment with a particular drug or therapeutic regiment. Those interested in determining the efficacy of a drug for reducing left ventricular remodeling can utilize the methods of the present invention.

In yet another embodiment the invention relates to a method of assessing the efficacy of a treatment for a patient having suffered from an acute myocardial infarction and is at risk for developing a reduced LV contractility wherein the method comprises i) determining the levels of miR-16, miR-27a, miR-101 and miR-150 in a body fluid of said patient, ii) determining the Nt-pro-BNP level in a body fluid of said patient, iii) determining the levels of miR-16, miR-27a, miR-101 and miR-150 and the level of Nt-pro-BNP in a body fluid of said patient after treatment, iv) comparing the results of i) and ii) with the results of iii), wherein a difference between the results of i), ii) and iii) indicates an effect of the treatment.

In certain embodiments, the treatment is the administration of a drug, such as an ACE inhibitor, an angiotensin II receptor blocker, a Beta-blocker, a vasodilator, a cardiac glycoside, an antiarrhythmic agent, a diuretic, statins, or an anticoagulant, an inotropic agent; an immunosuppressive agent, use of a pacemaker, defibrillator, mechanical circulatory support, or surgery.

Assay measurement strategies: numerous methods and devices are well known to the skilled artisan for measuring the prognostic indicators of the instant invention. With regard to polypeptides, such as Nt-pro-BNP, in patient samples, immunoassay devices and methods are often used. See, e.g. US6143576, US6113855 and US6019944. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. See, e.g. US5631171 and US5955377.

With regard to the determination of the 4 miRNAs of the invention, the expression of these 4 miRNAs can be measured separately or simultaneously. The miRNA expression levels are obtained, e.g. by using a quantitative RT-PCR or a bead-based system. In a particular embodiment a suitable array-based system (e.g. miRMAX microarray, GeneXpert System Cepheid, MDx platform Biocartis) can be developed and the extent of hybridization of the miRNAs in the sample to the beads or the probes on the microarray is determined. Once the miRNA expression levels of the sample are obtained, the levels are compared or evaluated against the model and the patient sample is classified.

In another particular embodiment the levels of miR-150 and miR101 in the body fluid derived from a patient having suffered from an AMI and being at risk for developing an impaired LV contractility are lower than the corresponding miRNA levels in the corresponding body fluid of a group of control patients. A control patient is typically a patient having suffered from an AMI and having preserved LV contractility. In a particular embodiment a control patient is a patient who has not suffered from an AMI and is also an individual with a preserved LV contractility. In a particular embodiment the levels of miR-150 and miR101 in the body fluid derived from a patient having suffered from an AMI and being at risk for developing an impaired LV contractility are at least 2-fold lower, at least 3-fold lower, at least 4-fold lower, at least 5-fold lower than the levels of the corresponding miRNA levels in the corresponding body fluid of a control patient.

In yet another particular embodiment the levels of miR-16 and miR27a in the body fluid derived from a patient having suffered from an AMI and being at risk for developing an impaired LV contractility are higher than the corresponding miRNA levels in the corresponding body fluid of a control patient. In a particular embodiment the levels of miR-16 and miR27a in the body fluid derived from a patient having suffered from an AMI and being at risk for developing an impaired LV contractility are at least 2-fold higher, at least 3-fold higher, at least 4-fold higher, at least 5-fold higher than the levels of the corresponding miRNA levels in the corresponding body fluid of a control patient.

In yet another embodiment for each increase of 1 unit of miR-16 in the patient body fluid the likelihood of classifying said patient into the category (or class) of developing a reduced left ventricular contractility is increased by 4.2 fold and for each increase of 1 unit of miR-27a in the patient body fluid the likelihood of classifying said patient into the category of developing a reduced left ventricular contractility is increased by 15.9 fold and for each increase of 1 unit of miR-101 in a patient body fluid the likelihood of classifying said patient into the high risk category of developing a reduced left ventricular contractility is decreased by 5.2 fold and for each increase of 1 unit of miR-150 in a patient body fluid, the likelihood of classifying said patient into the high risk category of developing a reduced left ventricular contractility is decreased by 12.1 fold and for an at least 3-fold increase of Nt-pro-BNP in a patient body fluid there is a high likelihood that a patient will develop a reduced left ventricular contractility.

In yet another embodiment the invention provides a kit for determining the prognosis of a patient diagnosed with an acute myocardial infarction. These kits preferably comprise devices and reagents for measuring the Nt-pro-BNP level in a patient sample, and devices and reagents for measuring the panel of 4 miRNAs of the invention and instructions for performing the assays. Optionally, the kits may contain one or more means for converting the Nt-pro-BNP levels and miRNA panel levels to a prognosis.

The application further discloses methods for the treatment of left ventricular modeling. In a specific embodiment the treatment is conditional to the value of the prognostic score obtained through the method for predicting and/or monitoring the prognosis of a patient having suffered from an acute myocardial infarction of the present invention.

Accordingly, disclosed herein are methods useful for the treatment of left ventricular remodeling (or reduced left ventricular cardiac contractility) based on the supplementation of miR-150 and/or miR-101 and/or in combination with the inhibition of miR-16 and/or miR-27a.

The application further describes a composition of

i) at least one short interfering nucleic acid capable of encoding a miRNA selected from the list consisting of miR-101 and miR-150 and at least one short interfering nucleic acid capable of inhibiting a miRNA selected from the list consisting of miR-16 and miR-27a or

ii) ii) short interfering nucleic acids capable of encoding miR-101 and miR-150 or

iii) iii) short interfering nucleic acids capable of inhibiting miR-16 and miR-27a for the treatment of left ventricular remodeling.

The wording 'at least one short interfering nucleic acid capable of encoding a miRNA selected from the list consisting of miR-101 and miR-150' refers to the supplementation of the miRNA expression of miR-150 or miR-101 which is downregulated in patients predicted with a prognosis of developing cardiac left ventricular remodeling. A short interfering nucleic acid capable of encoding a miRNA can for example be a microRNA, a short interfering RNA, a double-stranded RNA or a short hairpin RNA. A short interfering nucleic acid of the present invention can be chemically synthesized, expressed from a vector or enzymatically synthesized. The use of chemically-modified short interfering nucleic acids improves various properties of native short interfering nucleic acid molecules through, for example, increased resistance to nuclease degradation *in vivo* and/or through improved cellular uptake. Chemically synthesizing nucleic acid molecules with modifications (base, sugar and/or phosphate) that prevent their degradation by serum ribonucleases can increase their potency. There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups. In one embodiment the short interfering nucleic acid molecule is double stranded and each strand of the short interfering nucleic acid molecule comprises about 19 to about 23 nucleotides, and each strand comprises at least about 19 nucleotides that are complementary to the nucleotides of the other strand.

Also described herein is an embodiment of the composition wherein each strand of the short interfering nucleic acids comprises about 16 to about 25 nucleotides.

Also described herein are embodiments of the composition wherein a short interfering nucleic acid sequence is substantially similar to the sequence of the selected miRNA, or is a short interfering nucleic acid sequence which is identical to the selected miRNA sequence at all but 1, 2, 3, 4, 5, 6, 7, 8, 9, 10, 11, 12, 13, 14, 15, 16, 17, or 18 bases. In some embodiments, the short interfering nucleic acid sequence is a sequence that is substantially similar to the sequence of an miRNA, or is a short interfering nucleic acid sequence that is different than the miRNA sequence at all but up to one base. In some embodiments, a miRNA is supplemented by delivering an siRNA having a sequence that comprises the sequence, or a substantially similar sequence, of the miRNA. In still other embodiments, miRNAs are supplemented by delivering miRNAs encoded by shRNA vectors. Such technologies for delivery exogenous microRNAs to cells are well known in the art.

The wording "a short interfering nucleic acid capable of inhibiting a miRNA" refers to the inhibition of a selected miRNA function. A miRNA in itself inhibits the function of the mRNAs it targets and, as a result, inhibits expression of the polypeptides encoded by the mRNAs. Thus, blocking (partially or totally) or inhibiting the activity of a selected miRNA can effectively induce, or restore, expression of a polypeptide whose expression is inhibited (derepress the polypeptide). In one embodiment, derepression of polypeptides encoded by mRNA targets of a selected miRNA is accomplished by inhibiting the miRNA activity in cells through any one of a variety of methods. For example, blocking the activity of a miRNA can be accomplished by hybridization with a short interfering nucleic acid that is complementary, or substantially complementary to, the miRNA, thereby blocking interaction of the miRNA with its target mRNA. As used herein, a short interfering nucleic acid that is substantially complementary to a miRNA is a short interfering nucleic acid that is capable of hybridizing with a selected miRNA, thereby blocking the miRNA's activity. In some embodiments, a short interfering nucleic acid that is substantially complementary to a miRNA is a short interfering nucleic acid that is complementary with the miRNA at all but 1, 2, 3, 4, 5, 6, 7, 8, 9, 10, 11, 12, 13, 14, 15, 16, 17, or 18 bases. In some embodiments, a short interfering nucleic acid sequence is a sequence that is substantially complementary to a miRNA, or is a short interfering nucleic acid sequence that is complementary with the miRNA at, at least, one base. In particular embodiments antisense oligonucleotides, including chemically modified antisense oligonucleotides - such as 2' O-methyl, locked nucleic acid (LNA) - inhibit miRNA activity by hybridization with guide strands of mature miRNAs, thereby blocking their interactions with target mRNAs. In further particular embodiments 'antagomirs' are phosphorothioate modified oligonucleotides that can specifically block a selected miRNA in vivo (see for example Kurtzfeldt, J. et al. (2005) Nature 438, 685-689). In still other particular embodiments microRNA inhibitors, termed miRNA sponges, can be expressed in cells from transgenes (see for example Ebert, M.S. (2007) Nature Methods, 12). These miRNA sponges specifically inhibit selected miRNAs through a complementary heptameric seed sequence and even an entire family of miRNAs can be silenced using a single sponge sequence. Other methods for silencing miRNA function in cells will be apparent to one of ordinary skill in the art.

Also described herein is the use of the compositions as described herein before for the treatment of a human subject which may be a pediatric, an adult or a geriatric subject, wherein said human subject is predicted to develop left ventricular remodeling. As used herein treatment, or treating, includes amelioration, cure of a left ventricular remodeling. In specific embodiments the invention provides a pharmaceutical pack or kit comprising one or more containers filled with one or more of the ingredients of the pharmaceutical compositions of the invention. Associated with such container(s) can be various written materials (written information) such as instructions (indicia) for use, or a notice in the form prescribed by a governmental agency regulating the manufacture, use or sale of pharmaceuticals or biological products, which notice reflects approval by the agency of manufacture, use or sale for human administration. The pharmaceutical compositions of the present invention preferably contain a pharmaceutically acceptable carrier or excipient suitable for rendering the compound or mixture administrable orally as a tablet, capsule or pill, or parenterally, intravenously, intradermally, intramuscularly or subcutaneously, or transdermally. The active ingredients may be admixed or compounded with any conventional, pharmaceutically acceptable carrier or excipient. As used herein, the term "pharmaceutically acceptable carrier" includes any and all solvents, dispersion media, coatings, antibacterial and antifungal agents, isotonic agents, absorption delaying agents, and the like. The use of such media and agents for pharmaceutically active substances is well known in the art. Except insofar as any conventional media or agent is incompatible with the compositions of this invention, its use in the therapeutic formulation is contemplated. Supplementary active ingredients can also be incorporated into the pharmaceutical formulations. A composition is said to be a "pharmaceutically acceptable carrier" if its administration can be tolerated by a recipient patient. Sterile phosphate-buffered saline is one example of a pharmaceutically acceptable carrier. Other suitable carriers are well-known in the art. It will be understood by those skilled in the art that any mode of administration, vehicle or carrier conventionally employed and which is inert with respect to the active agent may be utilized for preparing and administering the pharmaceutical compositions of the present invention. Illustrative of such methods, vehicles and carriers are those described, for example, in Remington's Pharmaceutical Sciences, 21st ed. (2012), the disclosure of which is incorporated herein by reference. Those skilled in the art, having been exposed to the principles of the invention, will experience no difficulty in determining suitable and appropriate vehicles, excipients and carriers or in compounding the active ingredients therewith to form the pharmaceutical compositions of the invention. An effective amount, also referred to as a therapeutically effective amount, of a short interfering nucleic acid as described herein before is an amount sufficient to ameliorate at least one adverse effect associated with expression, or reduced expression, of the selected microRNA in a cell (for example a myocardial cell) or in an individual in need of such inhibition or supplementation. The therapeutically effective amount of the short interfering nucleic acid (active agent) to be included in pharmaceutical compositions depends, in each case, upon several factors, e.g. the type, size and condition of the patient to be treated, the intended mode of administration, the capacity of the patient to incorporate the intended dosage form, etc. Generally, an amount of active agent is included in each dosage form to provide from about 0.1 to about 250 mg/kg, and preferably from about 0.1 to about 100 mg/kg. One of ordinary skill in the art would be able to determine empirically an appropriate therapeutically effective amount. Use of the small interfering nucleic acid-based molecules of the invention can lead to better treatment of the disease progression by affording, for example, the possibility of combination therapies with known drugs, or intermittent treatment with combinations of small interfering nucleic acids and/or other chemical or biological molecules). In some embodiments therapeutic short interfering nucleic acids of the invention delivered exogenously are optimally stable within cells until translation of the target mRNA has been inhibited long enough to reduce the levels of the protein. This period of time varies between hours to days depending upon the disease state. These nucleic acid molecules should be resistant to nucleases in order to function as effective intracellular therapeutic agents. Improvements in the chemical synthesis of nucleic acid molecules described in the instant invention and in the art have expanded the ability to modify nucleic acid molecules by introducing nucleotide modifications to enhance their nuclease stability as described above. The administration of the herein described small interfering nucleic acid molecules to a patient can be intravenous, intraarterial, intraperitoneal, intramuscular, subcutaneous, intrapleural, intrathecal, by perfusion through a regional catheter, or by direct intralesional injection. When administering these small interfering nucleic acid molecules by injection, the administration may be by continuous infusion, or by single or multiple boluses. The dosage of the administered nucleic acid molecule will vary depending upon such factors as the patient's age, weight, sex, general medical condition, and previous medical history. Typically, it is desirable to provide the recipient with a dosage of the molecule which is in the range of from about 1 pg/kg to 10 mg/kg (amount of agent/body weight of patient), although a lower or higher dosage may also be administered. In some embodiments, it may be desirable to target delivery of a therapeutic to the heart, while limiting delivery of the therapeutic to other organs. This may be accomplished by any one of a number of methods known in the art. The delivery to the heart of a pharmaceutical formulation described herein comprises coronary artery infusion. The coronary artery infusion involves inserting a catheter through the femoral artery and passing the catheter through the aorta to the beginning of the coronary artery. The targeted delivery of a therapeutic to the heart involves using antibody-protamine fusion proteins, such as those previously described (Song E et al. (2005) Nature Biotechnology Vol. 23(6), 709-717) to deliver the small interfering nucleic acids disclosed herein. While it is possible for the agents to be administered as the raw substances, it is preferable, in view of their potency, to present them as a pharmaceutical formulation. The formulations for human use comprise the agent, together with one or more acceptable carriers therefor and optionally other therapeutic ingredients. The carrier(s) must be "acceptable" in the sense of being compatible with the other ingredients of the formulation and not deleterious to the recipient thereof or deleterious to the inhibitory function of the active agent. Desirably, the formulations should not include oxidizing agents and other substances with which the agents are known to be incompatible. The formulations may conveniently be presented in unit dosage form and may be prepared by any of the methods well known in the art of pharmacy. All methods include the step of bringing into association the agent with the carrier, which constitutes one or more accessory ingredients. In general, the formulations are prepared by uniformly and intimately bringing into association the agent with the carrier(s) and then, if necessary, dividing the product into unit dosages thereof. Formulations suitable for parenteral administration conveniently comprise sterile aqueous preparations of the agents, which are preferably isotonic with the blood of the recipient. Suitable such carrier solutions include phosphate buffered saline, saline, water, lactated ringers or dextrose (5% in water). Such formulations may be conveniently prepared by admixing the agent with water to produce a solution or suspension, which is filled into a sterile container and sealed against bacterial contamination. Preferably, sterile materials are used under aseptic manufacturing conditions to avoid the need for terminal sterilization. Such formulations may optionally contain one or more additional ingredients among which may be mentioned preservatives, such as methyl hydroxybenzoate, chlorocresol, metacresol, phenol and benzalkonium chloride. Such materials are of special value when the formulations are presented in multidose containers. Buffers may also be included to provide a suitable pH value for the formulation. Suitable such materials include sodium phosphate and acetate. Sodium chloride or glycerin may be used to render a formulation isotonic with the blood. If desired, the formulation may be filled into the containers under an inert atmosphere such as nitrogen or may contain an antioxidant, and are conveniently presented in unit dose or multidose form, for example, in a sealed ampoule.

The following examples are provided to better illustrate particular embodiments, and they should not be considered limiting the application. The application is limited only by the claims.

## Examples

### Example 1

1.Patient characteristics

Table 1 shows the demographic features of patients of the studied population. Among the 150 patients enrolled 79 patients evidenced preserved LV contractility at follow-up (WMIS ≤ 1.2) and 71 patients had a loss of LV contractility. Comparisons between these 2 groups of patients revealed that patients with impaired contractility had higher levels of troponin I, creatine kinase and Nt-pro-BNP at discharge than patients with preserved contractility. Diuretics were more often prescribed in these patients, and they had a higher risk of developing chronic heart failure.

**Table 1. Demographic and clinical features of AMI patients**

| | All (N=150) | WMIS ≤ 1.2 (N=79) | WMIS > 1.2 (N=71) | *P*[1] |
|---|---|---|---|---|
| Age, y (median-range) | 64 (24-87) | 61 (37-86) | 65 (24-87) | 0.56 |
| Male, n (%) | 116 (77%) | 63 (80%) | 53 (75%) | 0.89 |
| **Cardiovascular history/ risk factors, n (%)** | | | | |

(continued)

| | All (N=150) | WMIS ≤ 1.2 (N=79) | WMIS > 1.2 (N=71) | *P*[1] |
|---|---|---|---|---|
| Smoker | 60 (40%) | 33 (42%) | 27 (38%) | 0.88 |
| FH | 59 (39%) | 31 (42%) | 28 (35%) | 0.89 |
| Angina | 14 (28%) | 5 (6%) | 9 (13%) | 0.35 |
| Diabetes | 24 (16%) | 12 (15%) | 12 (17%) | 1 |
| Hypertension | 52 (35%) | 26 (33%) | 26 (37%) | 1 |
| Hypercholesterolaemia | 40 (27%) | 18 (23%) | 22 (31%) | 0.49 |
| MI | 12 (8%) | 3 (4%) | 9 (13%) | 0.12 |
| PCI | 3 (2%) | 3 (4%) | 0 (0%) | 0.30 |
| CABG | 1 (1%) | 0 (0%) | 1 (1%) | 0.96 |
| **Presentation, n (%)** | | | | |
| STEMI | 127 (85%) | 62 (78%) | 65 (92%) | 0.60 |
| Anterior infarct | 59 (39%) | 24 (30%) | 35 (49%) | 0.16 |
| Thrombo lysis | 75 (50%) | 42 (53%) | 33 (46%) | 0.74 |
| **Serum markers during admission (median-range)** | | | | |
| Troponin I (ng/mL) | 9.83 (0.08-150) | 5.90 (0.08-150) | 19.95 (0.09-150) | 0.001 |
| CK (units/L) | 985 (56-7384) | 625 (56-3925) | 1614 (123-7384) | <0.001 |
| Nt-pro-BNP (ng/L) | 2.80 (0.26-3.98) | 2.53 (0.26-3.55) | 3.16 (0.94-3.98) | <0.001 |
| **Medications at admission, n(%)** | | | | |
| Aspirin | 21 (14%) | 9 (11%) | 12 (17%) | 0.54 |
| Clopidogrel | 4 (3%) | 3 (4%) | 1 (1%) | 0.71 |
| Beta-blockers | 24 (16%) | 13 (16%) | 11 (15%) | 0.93 |
| Calcium antagonists | 22 (15%) | 7 (9%) | 15 (21%) | 0.11 |
| ACE inhibitors | 17 (11%) | 6 (8%) | 11 (15%) | 0.27 |
| Angiotensin receptor blocker | 9 (6%) | 6 (8%) | 3 (4%) | 0.64 |
| Statins | 28 (19%) | 13 (16%) | 15 (21%) | 0.69 |
| **Medications at discharge, n(%)** | | | | |
| Aspirin | 134 (89%) | 73 (92%) | 61 (86%) | 0.85 |
| Clopidogrel | 36 (24%) | 23 (29%) | 13 (18%) | 0.30 |
| Beta-blocker | 142 95%) | 75 (95%) | 67 (94%) | 0.93 |
| ACE inhibitor | 134 (89%) | 71 (90%) | 63 (89%) | 0.95 |
| Angiotensin receptor blocker | 11 (7%) | 5 (6%) | 6(8%) | 0.88 |
| Diuretic | 15 (10%) | 2 (3%) | 13 (18%) | 0.008 |
| Statin | 148 (99%) | 78 (99%) | 70 (99%) | 0.91 |
| **Endpoints at 6-months** | | | | |
| Reinfarction, n (%) | 15 (10%) | 5 (6%) | 10 (14%) | 0.25 |
| CHF, n (%) | 11 (7%) | 1 (1%) | 10 (14%) | 0.01 |
| Death, n (%) | 4 (3%) | 1 (1%) | 3(4%) | 0.56 |

[1] For comparison between WMIS ≤ 1.2 and WMIS > 1.2.

ACE: angiotensin-converting enzyme; BNP: brain natriuretic peptide; CABG: coronary artery bypass grafting; CHF: congestive heart failure; CK: creatine kinase; FH: familial hypercholesterolemia; MI: myocardial infarction; PCI: percutaneous coronary intervention; STEMI: ST-elevation myocardial infarction.

Table 2 shows the parameters of LV function as assessed by echocardiography, at discharge from the hospital and at 6-months follow-up. Patients who subsequently developed a loss of LV contractility had lower EF and higher LV volumes and diameters compared to patients with preserved LV contractility, at discharge from the hospital as well as after 6 months.

**Table 2. Echo parameters of AMI patients**

| | All (N=150) | WMIS ≤ 1.2 (N=79) | WMIS > 1.2 (N=71) | *P*[1] |
|---|---|---|---|---|
| **Pre-discharge echo/MRI (median-range)** | | | | |
| LVEF (%) | 44 (15-75) | 50 (22-75) | 37(15-61) | <0.001 |
| LVEDV (mL) | 89 (36-201) | 83 (36-159) | 94 (36-201) | 0.005 |
| LVESV (mL) | 46 (20-132) | 39 (21-86) | 56 (20-132) | <0.001 |
| LVIDd (mm) | 4.8 (2.9-6.6) | 4.6 (2.9-6) | 5.2 (3.7-8.1) | 0.006 |
| LVIDs (mm) | 3.6 (1.8-5.5) | 3.35 (1.8-5.2) | 4.1 (1.7-7) | <0.001 |
| WMIS | 1.31 (1-2.38) | 1.06 (1-1.81) | 1.74 (1-2.38) | <0.001 |
| **Follow-up echo/MRI (median-range)** | | | | |
| LVEF (%) | 48 (16-74) | 52 (37-74) | 40 (16-69) | <0.001 |
| LVEDV (mL) | 87 (40-208) | 80 (45-163) | 96 (40-208) | <0.001 |
| LVESV (mL) | 45 (17-141) | 38 (17-89) | 58 (17-141) | <0.001 |
| LVIDd (mm) | 4.9(3-8.1) | 4.8 (3-6.3) | 5.2 (3.7-8.1) | <0.001 |
| LVIDs (mm) | 3.6 (1.6-7) | 3.4 (1.6-4.9) | 4.1 (1.7-7) | <0.001 |
| WMIS | 1.19 (1-2.38) | 1 (1-1.2) | 1.5 (1.25-2.38) | <0.001 |

[1] For comparison between WMIS ≤ 1.2 and WMIS > 1.2.
LVEDV: end-diastolic volume; LVESV: end-systolic volume; LVEF: ejection fraction;
LVIDd: end-diastolic diameter; LVIDs: end-systolic diameter.

2.Estimation of risk of impaired LV contractility

Levels of miR-16/27a/101/150 were measured in blood samples obtained at discharge from the hospital. Nt-pro-BNP levels at discharge were also determined in each patient. LV contractility at follow-up was assessed by echocardiographic determination of WMIS. High WMIS indicates an impairment of LV contractility. 55 patients had a WMIS equal to 1, indicating a fully preserved contractility in these patients. Due to this left censoring of WMIS values at 1, censored regression (aka "Tobit regression") was used for prediction analysis. To compare the predictive value of miRNAs over classical markers, 2 multivariable models were built. The first model (=model 1) included the following parameters: age, gender, smoking habit, diabetes, hypertension, hypercholesterolemia, antecedent of MI, infarct type (STEMI vs NSTEMI), infarct territory (anterior vs inferior), and Nt-pro-BNP level at discharge. The second model (= model 2) included all the parameters of model 1 and the expression values of a panel of 4 miRNAs, miR-16/27a/101/150, measured in plasma samples obtained at discharge.

Figure 1A shows the odds ratios (OR) of each variable in model 1. Infarct type, infarct territory and Nt-pro-BNP were significant predictors of WMIS. Patients with anterior STEMI and elevated Nt-pro-BNP were at higher risk of impaired contractility. Figure 1B shows that miR-27a and miR-150 significantly contributed to the predictive value of model 2. The predictive values of miR-16 and miR-101 were of borderline significance.

3.Added value of combinations of miRNAs

We determined the ability of each miRNA and of combinations of several miRNAs to improve the predictive value of model 1 (Table 3). The AIC was used in this analysis since this criteria is adjusted by the number of variables, contrarily to AUC which is biased by variable number. This allows avoiding the chance of having a better prediction only by increasing the number of variables included in the model. Low AIC is indicative of accurate prediction. No single miRNA was able to add to the predictive value of model 1. All 4 miRNAs were necessary to significantly improve the model.

**Table 3. Added value of combinations of miRNAs (censored regression)**

| miRNA added to model 1 | Wald chi square test P-value | AIC | LRT P-value |
|---|---|---|---|
| None | 4.35E-07 | 205.386 | |
| miR-16 | 8.47E-07 | 206.853 | 0.465 |
| miR-27a | 2.66E-07 | 204.351 | 0.081 |
| miR-101 | 7.38E-07 | 206.655 | 0.392 |

(continued)

| miRNA added to model 1 | Wald chi square test P-value | AIC | LRT P-value |
|---|---|---|---|
| miR-150 | 9.69E-07 | 207.332 | 0.817 |
| miR-16+miR-27a | 6.02E-07 | 206.35 | 0.219 |
| miR-16+miR-101 | 1.61E-06 | 208.536 | 0.654 |
| miR-16+miR-150 | 1.10E-06 | 207.643 | 0.418 |
| miR-27a+miR-101 | 3.82E-07 | 205.303 | 0.130 |
| miR-27a+miR-150 | 1.68E-07 | 203.816 | 0.062 |
| miR-101+miR-150 | 1.20E-06 | 208.074 | 0.519 |
| miR-16+miR-27a+miR-101 | 8.31E-07 | 207.232 | 0.245 |
| miR-16+miR-27a+miR-150 | 1.98E-07 | 204.178 | 0.066 |
| miR-16+miR-101+miR-150 | 1.72E-06 | 208.95 | 0.487 |
| miR-27a+miR-101+miR-150 | 2.49E-07 | 204.826 | 0.087 |
| miR-16+miR-27a+miR-101+miR-150 | 1.51E-07 | 203.752 | 0.047 |

Shown are the results of all combinations of miRNAs added to model 1. The Wald chi square test indicates the significance of the model. The likelihood ratio test (LRT) compares the predictive value of a model with miRNAs to model 1. AIC: Akaike information criteria.

4.Model validation

Bootstrap internal validation was used to test the strength of the models with combinations of miRNAs (Figure 2). The principle of this test is to calculate the predictive value of the model after resampling patient from the original sample. The model including the 4 miRNAs was the best predictor in 29% of the 150 iterations performed. MiR-27a was selected in 13% of cases and was included in all top models, showing its significant contribution to the prediction.

5.Logistic regression analyses

So far, WMIS was used as a continuous variable. To investigate whether miRNAs are valuable to predict whether a patient will or will not have impaired LV contractility, we dichotomized the population using a WMIS value of 1.2 as threshold, and we performed logistic regression analyses. These analyses were performed to model the probability (P) of belonging to the group of patients that will have impaired LV contractility (WMIS >1.2) over the probability of belonging to the group of patients that will not have impaired LV contractility (WMIS ≤ 1.2). The logistic regression output can thereafter be used as a classifier by prescribing that a sample will be classified in the group of patient that will have impaired LV contractility if P is greater than 0.5, or 50%. Predicting variables included the expression levels of the four miRNAs (in log-scale), the level of Nt-pro-BNP and the other clinical variables. As for censored regression (model 1 and model 2), 2 multivariable models were built: model 3 includes all clinical variables and Nt-pro-BNP, and model 4 includes all variables of model 3 and the 4 miRNAs panel. Odds ratio (OR) for each variable in both models are shown in Figure 3.

Probability (P) calculation of risk of impaired contractility from model 4 is done according to the following formulas:

$$P = \exp(X) / (1+\exp(X))$$

Whereas X= variable 1 x ln OR (var 1) + variable 2 x ln OR (var2) + ....variable X x ln OR (var x) + intercept

If P > 0.5 then high risk of remodeling (WMIS >1.2)

If P <= 0.5 then low or null risk of remodeling (WMIS <=1.2)

For miRNAs, "variable" in the formula indicates the expression values of this particular miRNA in the patient as determined by quantitative RT-PCR as described in Material and Methods.

For Nt-pro-BNP, "variable" in the formula indicates the concentration of Nt-pro-BNP in the patient blood as determined by immune-assay as described in Material and Methods.

For other clinical parameters, "variables" in the formula are binary, except for age which was considered as a continuous variable.

Odds ratio (OR) indicates the contribution of each variable to the prediction. OR below 1 indicates a negative association between a considered variable and the outcome of the patient; OR above 1 indicates a positive association between a considered variable and the outcome of the patient. OR were obtained with R version 2.13.1 with Hmisc, pROC, aod, lmtest and AER packages.

Each OR is associated with 95% CI and P value indicating the statistical significance of the variable in the model.

As an example, for model 4:

X= miR150 x ln 0.08 + miR101 x ln 0.19 + miR27a x ln 15.9 + miR16 x In 4.18 + Nt-pro-BNP x ln 3.97 + territory x ln 2.29 + STEM/NSTEMI x ln 1.68 + Prior MI x ln 8.87 + Hypercholesterolemia x ln 1.63 + Hypertension x ln 1.00 + Diabetes x ln 0.70 + Smoking habit x ln 1.49 + Gender x ln 1.29 + Age x ln 1.00 + ln 8.51x10E-5

Each value of the odd ratio as mentioned in the formula can be replaced by any value within its corresponding 95% CI. As an example from model 4, considering miR-150, OR value can be from 0.01 to 0.48. However, the intercept (ln 8.51x10E-5) is a constant.

Patients with anterior STEMI and elevated Nt-pro-BNP were at high risk of dyskinesia (Figure 3A). All 4 miRNAs were significantly associated with WMIS group. Patients with low levels of miR-150/101 and elevated levels of miR-16/27a were at high risk of dyskinesia (Figure 3B).

We next determined the added value of combinations of miRNAs. As shown in Table 4, the 4 miRNA panel had an additive value to the model with clinical parameters and Nt-pro-BNP (model 3). Adding the 4 miRNAs decreased the AIC from 188.269 to 181.432 (P=0.005). miR-27a/150 was the smallest combination of miRNAs which generated a significant added value.

**Table 4. Added value of combinations of miRNAs (logistic regression)**

| miRNA added to model 3 | Wald chi square test P-value | AIC | LRT P-value |
|---|---|---|---|
| None | 0.003 | 188.269 | |
| miR-16 | 0.003 | 188.381 | 0.169 |
| miR-27a | 0.003 | 186.591 | 0.055 |
| miR-101 | 0.004 | 189.476 | 0.373 |
| miR-150 | 0.004 | 190.261 | 0.931 |
| miR-16+miR-27a | 0.005 | 188.245 | 0.134 |
| miR-16+miR-101 | 0.006 | 190.332 | 0.380 |
| miR-16+miR-150 | 0.003 | 187.753 | 0.105 |
| miR-27a+miR-101 | 0.005 | 186.842 | 0.066 |
| miR-27a+miR-150 | 0.004 | 186.117 | 0.046 |
| miR-101+miR-150 | 0.006 | 191.080 | 0.552 |
| miR-16+miR-27a+miR-101 | 0.007 | 187.837 | 0.092 |
| miR-16+miR-27a+miR-150 | 0.003 | 183.838 | 0.015 |
| miR-16+miR-101+miR-150 | 0.005 | 189.380 | 0.180 |
| miR-27a+miR-101+miR-150 | 0.006 | 186.389 | 0.049 |
| miR-16+miR-27a+miR-101+miR-150 | 0.003 | 181.432 | 0.005 |

Shown are the results of all combinations of miRNAs added to model 3. The Wald chi square test indicates the significance of the model. The likelihood ratio test (LRT) compares the predictive value of a model with miRNAs to model 1. AIC: Akaike information criteria.

Bootstrap cross validation confirmed that the 4 miRNA panel provided the optimal improvement of prediction (Figure 4).

6.Reclassification analyses

The continuous version of the Net Reclassification Index and the Integrated Discrimination Improvement were computed to determine the ability of miRNAs to correctly reclassify patients misclassified by model 3 (Table 5). These are indexes of the change in classification of patients from one category of WMIS to another category (≤ 1.2 or > 1.2). The 4 miRNA panel was able to reclassify a significant proportion of patients, as attested by a NRI of 66 %(P=5x10E-5) and an IDI of 8 % (P=0.001). Several combinations of miRNAs also provided significant reclassifications, such as miR-16/150, miR-27a/150, miR-16/27a/150, or miR-27a/101/150. However, no single miRNA had a significant reclassification capability.

**Table 5. Reclassification analyses (logistic regression)**

| miRNA added to model 3 | NRI | 95% CI | NRI P-value | | IDI | 95% CI | IDI P-value |
|---|---|---|---|---|---|---|---|
| miR-16 | 0.179 | -0.142-0.499 | 0.275 | | 0.010 | -0.007-0.027 | 0.243 |
| miR-27a | 0.263 | -0.057-0.584 | 0.108 | | 0.017 | -0.007-0.040 | 0.162 |
| miR-101 | 0.181 | -0.139-0.502 | 0.267 | | 0.004 | -0.007-0.015 | 0.453 |
| miR-150 | 0.120 | -0.201-0.440 | 0.464 | | 1.53E-04 | -0.001-0.001 | 0.774 |
| miR-16+miR-27a | 0.314 | -0.007-0.634 | 0.055 | | 0.019 | -0.005-0.044 | 0.125 |
| miR-16+miR-101 | 0.125 | -0.195-0.446 | 0.444 | | 0.010 | -0.007-0.027 | 0.232 |
| miR-16+miR-150 | 0.331 | 0.010-0.651 | 0.043 | | 0.028 | 0.002-0.053 | 0.033 |
| miR-27a+miR-101 | 0.379 | 0.058-0.699 | 0.021 | | 0.024 | -0.004-0.053 | 0.087 |
| miR-27a+miR-150 | 0.646 | 0.326-0.967 | 7.78E-05 | | 0.031 | 0.001-0.061 | 0.046 |
| miR-101+miR-150 | 0.213 | -0.108-0.533 | 0.194 | | 0.007 | -0.006-0.021 | 0.296 |
| miR-16+miR-27a+miR-101 | 0.474 | 0.154-0.795 | 0.004 | | 0.030 | -0.001-0.060 | 0.054 |
| miR-16+miR-27a+miR-150 | 0.415 | 0.095-0.736 | 0.011 | | 0.056 | 0.018-0.095 | 0.004 |
| miR-16+miR-101+miR-150 | 0.257 | -0.063-0.578 | 0.115 | | 0.030 | 0.004-0.056 | 0.025 |
| miR-27a+miR-101+miR-150 | 0.514 | 0.193-0.834 | 0.002 | | 0.039 | 0.005-0.072 | 0.023 |
| miR-16+miR-27a+ miR-101+miR-150 | 0.663 | 0.342-0.983 | 5.05E-05 | | 0.077 | 0.032-0.122 | 0.001 |

Shown are the results of all combinations of miRNAs added to model 3. The continuous version of the net reclassification index (NRI) was used in these analyses. CI: confidence interval. IDI: integrated discrimination improvement.

7.Classification of patients with ambiguous phenotype

A main advantage of new biomarkers is to improve the classification of patients with intermediate phenotypes, which are difficult to classify using existing biomarkers. To test the accuracy of the 4 miRNA panel to improve the classification of patients with ambiguous phenotype, we considered borderline patients (1<WMIS<1.4, n=49). Among these, 25 patients had moderate LV dysfunction (1.2<WMIS<1.4) and 24 had no LV dysfunction (1<WMIS≤1.2). Logistic regression and leave-one-out cross validation were used in these analyses. Two models were built, one with clinical variables and Nt-pro-BNP and one with clinical variables, Nt-pro-BNP and the 4 miRNA panel. The model with clinical variables and Nt-pro-BNP had a specificity of 75%, but also a poor sensitivity of 48%. The 4 miRNAs panel increased the sensitivity to 60%, while maintaining the specificity at 75%. With miRNAs, the positive predictive value was increased from 67% to 71%, and the negative predictive value was increased from 58% to 64%. Therefore, the 4 miRNAs panel improved the prognostication of patients with ambiguous phenotype, particularly dyskinetic patients.

Materials and Methods

1.Patients

One hundred and fifty patients with ST-elevation AMI (STEMI) (Table 1) were enrolled in this study. The diagnosis of AMI was based on presentation with appropriate symptoms of myocardial ischemia, dynamic ST segment elevation, and increase in markers of myocyte necrosis [creatine kinase (CK) and troponin I (TnI)] to above twice the upper limit of the normal range. Venous blood samples were collected in EDTA-aprotinin tubes, immediately prior to discharge (day 3-4 after AMI). Samples were centrifuged within 30 minutes and plasma stored in aliquots at -80°C.

The protocol for both cohorts was approved by the local research ethics committee and written informed consent was obtained from all subjects. The conduct of the study was in accordance with the Declaration of Helsinki.

Patients were admitted to Glenfield Hospital, Leicester, UK between September 2004 and March 2005, and were enrolled in a prospective study of LV remodelling after AMI[9]. Half of these patients were treated by thrombolysis. None received primary percutaneous coronary intervention (PPCI), which was not in routine use at this centre at the time. Cardiac function was assessed by echocardiography, as described [9], conducted by a single operator (DK) at discharge and at a median of 176 days (range 138-262 days) after AMI. LV contractility was evaluated by the LV wall motion index score (WMIS), using a standard 16-segment model from para-sternal long- and short-axis and apical two- and four-chamber views. Each LV segment was scored as 0, hyperkinetic; 1, normal; 2, hypokinetic; 3, akinetic; 4, dyskinetic.

The total was divided by the number of segments analysed to give an overall score with higher values indicating more impaired LV contractility. In some analyses, patients were dichotomized into impaired LV contractility group (WMIS > 1.2 at follow-up) and preserved LV contractility group (WMIS ≤ 1.2 at follow-up).

2.Plasma miRNAs determination

Total RNA was extracted from plasma samples using the mirVana PARIS kit (Ambion, Applied Biosystem, Lennik, Belgium) without enrichment for small RNAs. A mix of 3 spiked-in synthetic *C. elegans* miRNAs (Qiagen, Venlo, The Netherlands), lacking sequence homology to human miRNAs, was added to plasma samples for correction of extraction efficiency. Potential genomic DNA contamination was eliminated using DNase (Qiagen). Reverse transcription of RNA was performed with the miScript reverse transcription kit (Qiagen). The resulting cDNA was diluted 10-fold before quantitative PCR using the miScript SYBR-green PCR kit (Qiagen). miRNA-specific miScript primer sets were obtained from Qiagen. Expression values were normalized using the mean Ct obtained from the spiked-in controls [calculation formula: 2 exp (mean Ct spiked-in controls - Ct target miRNA)] and log-transformed. The detection limit of the PCR assay was -7.2, which is the log transformation of the minimum expression detected divided by 10.

3.Nt-pro-BNP assay

Peptides corresponding to the N-terminal (amino acids 1 to 12) and C-terminal (amino acids 65 to 76) of the human Nt-pro-BNP were used to raise rabbit polyclonal antibodies. IgG from the sera was purified on protein A sepharose columns. The C-terminal-directed antibody (0.5 μg in 100 μL for each well) was immobilized onto ELISA plates. The N-terminal antibody was affinity purified and biotinylated using biotin-X-N-hydroxysuccinimide ester (Calbiochem). Aliquots (20 μL) of samples or Nt-pro-BNP standards were incubated in the C-terminal antibody coated wells with the biotinylated antibody for 24 hours at 4°C. ELISA plates were washed with 0.1% Tween in PBS, and streptavidin (Chemicon International Ltd) labeled with methyl-acridinium ester (5×106 relative light units/mL) was added to each well. Plates were read on a Dynatech MLX Luminometer, with sequential injections of 100 μL of 0.1 mol/L nitric acid (with H2O2) and then 100 μL of NaOH (with cetyl ammonium bromide).13 The lower limit of detection was 14.4 fmol/mL of unextracted plasma. Within and between assays, coefficients of variation were acceptable at 2.3% and 4.8%, respectively. There was no cross-reactivity with ANP, BNP, or CNP.

4. Statistical analysis

*Patient characteristics*

Analysis of demographic features and echo parameters were carried out using SigmaPlot v 11.0. For all comparisons, a P-value <0.05 was considered significant. For categorical data, comparisons were by Chi-square test. Comparisons between groups of continuous data were performed with t-test for Gaussian data and the Mann-Whitney test on ranks for non-normally distributed data. Normality was assessed with the Shapiro-Wilk test.

*Prediction analyses*

Prediction analyses were performed with R version 2.13.1 with Hmisc, pROC, aod, lmtest and AER packages. A P-value was considered significant when lower than 0.05. Clinical features were coded as 1 for presence and 0 for absence. Male was chosen as the reference level for sex in regression models. No data were missing thus no imputation method was performed.

Model fitting

WMIS was first treated as a continuous variable (models 1 and 2). Since more than a third of the patients add a value that equalled one (the remaining patients having greater values), a left censored Tobit regression [11] was performed to model WMIS with different sets of predictors. WMIS was then dichotomized into two groups (WMIS <=1.2 and WMIS >1.2), which were analysed by logistic regression (models 3 and 4).

Model parameter estimates were tested for nullity using a Z test in censored regression and a Wald Chi-square test in logistic regression. Residuals were analysed graphically both to detect nonlinear relationships between each variable in a model and WMIS, and to check normality assumptions for Tobit regression. For logistic regression, odd ratios (OR) and 95% confidence intervals (CI) were obtained by exponential transformation, and are shown in Figures 1 to 3.

Best model selection

To determine which miRNA or combination of miRNAs had the maximal added value, all 15 possible combinations of miRNAs among the 4 miRNAs measured were generated and successively added to the reference model containing clinical parameters and Nt-pro-BNP.

For each model, a Wald Chi-square test was used to assess the global effect of miRNAs on WMIS. The added value of miRNAs was tested for significance using the likelihood ratio test (LRT). In the dichotomous case, continuous net reclassification index (NRI) and integrated discrimination improvement (IDI) [12] were evaluated and tested for nullity. The final model was finally selected by minimizing Akaike Information Criterion (AIC) which is penalized by the number of variables added in the model to avoid overfitting.

Model validation

Bootstrap internal validation was used to correct all measures of model performance for optimisation. For each bootstrap sample (i.e. a random sample of individuals with the same size as the original sample where a given patient can appear several times), the whole model selection process was performed again to select the best model according to AIC criterion; the original sample was then tested with this model. In order to evaluate optimisation, NRI and IDI were computed with the test (i.e. original) set and subtracted to the same measures computed with the bootstrap sample. Afterwards optimisation was averaged across 150 bootstrap replications and finally subtracted to the measures obtained with the original sample as a training set.

Borderline patients classification

Borderline patients were defined as having 1 < WMIS < 1.4. To determine whether miRNAs improved the classification of these patients, cross-validation was performed by successively leaving only those patients out one by one during logistic regression. A patient was classified as WMIS > 1.2 when its probability was greater or equal to 0.5 and as WMIS <= 1.2 otherwise. Sensitivity, specificity, positive and negative predictive values were then computed and compared exclusively for those patients between both models.

The present invention refers to the following nucleotide and amino acid sequences:

SEQ ID No. 1:

Nucleotide sequence encoding microRNA 16 (miRNA). (Accession Number of NCBI Reference Sequence: NR_029486.1)

gtcagcagtg ccttagcagc acgtaaatat tggcgttaag attctaaaat tatctccagt attaactgtg ctgctgaagt aaggttgac

SEQ ID No. 2:

Nucleotide sequence encoding microRNA 27a (miRNA). (Accession Number of NCBI Reference Sequence: NR_029501.1) ctgaggagca gggcttagct gcttgtgagc agggtccaca ccaagtcgtg ttcacagtgg ctaagttccg ccccccag

SEQ ID No. 3:

Nucleotide sequence encoding microRNA 101 (miRNA). (Accession Number of NCBI Reference Sequence: NR_029516.1)
tgccctggct cagttatcac agtgctgatg ctgtctattc taaaggtaca gtactgtgat aactgaagga tggca

SEQ ID No. 4:

Nucleotide sequence encoding microRNA 150 (miRNA). (Accession Number of NCBI Reference Sequence:

NR_029703.1)

ctccccatgg ccctgtctcc caacccttgt accagtgctg ggctcagacc ctggtacagg cctgggggac agggacctgg ggac

SEQ ID No. 5:

Amino acid sequence encoding Nt-pro-BNP (Accession Number of NCBI Reference Sequence: NP_002512.1)mdpqtapsra lllllflhla flggrshplg spgsasdlet sglqeqrnhl qgklselqve qtsleplqes prptgvwksr evategirgh rkmvlytlra prspkmvqgs gcfgrkmdri ssssglgckv lrrh

References

1. Torabi A, Cleland JG, Khan NK, Loh PH, Clark AL, Alamgir F, Caplin JL, Rigby AS, Goode K. The timing of development and subsequent clinical course of heart failure after a myocardial infarction. Eur Heart J 2008;29:859-870.

2. Talwar S, Squire IB, Downie PF, McCullough AM, Campton MC, Davies JE, Barnett DB, Ng LL. Profile of plasma N-terminal proBNP following acute myocardial infarction; correlation with left ventricular systolic dysfunction. Eur Heart J 2000;21:1514-1521.

3. Gilad S, Meiri E, Yogev Y, Benjamin S, Lebanony D, Yerushalmi N, Benjamin H, Kushnir M, Cholakh H, Melamed N, Bentwich Z, Hod M, Goren Y, Chajut A. Serum microRNAs are promising novel biomarkers. PLoS One 2008;3:e3148.

4. Mitchell PS, Parkin RK, Kroh EM, Fritz BR, Wyman SK, Pogosova-Agadjanyan EL, Peterson A, Noteboom J, O'Briant KC, Allen A, Lin DW, Urban N, Drescher CW, Knudsen BS, Stirewalt DL, Gentleman R, Vessella RL, Nelson PS, Martin DB, Tewari M. Circulating microRNAs as stable blood-based markers for cancer detection. Proc Natl Acad Sci U S A 2008;105:10513-10518.

5. Creemers EE, Tijsen AJ, Pinto YM. Circulating MicroRNAs: Novel Biomarkers and Extracellular Communicators in Cardiovascular Disease? Circ Res 2012;110:483-495.

6. Devaux Y, Vausort M, Goretti E, Nazarov PV, Azuaje F, Gilson G, Corsten MF, Schroen B, Lair ML, Heymans S, Wagner DR. Use of Circulating MicroRNAs to Diagnose Acute Myocardial Infarction. Clin Chem 2012;58:559-567.

7. Widera C, Gupta SK, Lorenzen JM, Bang C, Bauersachs J, Bethmann K, Kempf T, Wollert KC, Thum T. Diagnostic and prognostic impact of six circulating microRNAs in acute coronary syndrome. J Mol Cell Cardiol 2011;51:872-875.

8. Zile MR, Mehurg SM, Arroyo JE, Stroud RE, Desantis SM, Spinale FG. Relationship Between The Temporal Profile of Plasma microRNA and Left Ventricular Remodeling In Patients Following Myocardial Infarction. Circ Cardiovasc Genet 2011;4:614-619.

9. Kelly D, Cockerill G, Ng LL, Thompson M, Khan S, Samani NJ, Squire IB. Plasma matrix metalloproteinase-9 and left ventricular remodelling after acute myocardial infarction in man: a prospective cohort study. Eur Heart J 2007;28:711-718.

10. Omland T, Persson A, Ng L, O'Brien R, Karlsson T, Herlitz J, Hartford M, Caidahl K. N-terminal pro-B-type natriuretic peptide and long-term mortality in acute coronary syndromes. Circulation 2002;106:2913-2918.

11. Tobin J. Estimation of relationships for limited dependent variables. Econometrica 1958;26:24-36.

12. Pencina MJ, D'Agostino RB, Sr., D'Agostino RB, Jr., Vasan RS. Evaluating the added predictive ability of a new marker: from area under the ROC curve to reclassification and beyond. Stat Med 2008;27:157-172; discussion 207-112.

13. Zampetaki A, Willeit P, Tilling L, Drozdov I, Prokopi M, Renard JM, Mayr A, Weger S, Schett G, Shah A, Boulanger CM, Willeit J, Chowienczyk PJ, Kiechl S, Mayr M. Prospective Study on Circulating MicroRNAs and Risk of Myocardial Infarction. J Am Coll Cardiol 2012;60:290-299.

14. Zhu H, Fan GC. Role of microRNAs in the reperfused myocardium towards post-infarct remodelling. Cardiovasc Res 2012;94:284-292.

15. Abdellatif M. Differential Expression of MicroRNAs in Different Disease States. Circ Res 2012;110:638-650.

16. Da Costa Martins PA, De Windt LJ. MicroRNAs in control of cardiac hypertrophy. Cardiovasc Res 2012;93:563-572.

17. Yamamoto K, Ohki R, Lee RT, Ikeda U, Shimada K. Peroxisome Proliferator-Activated Receptor y Activators Inhibit Cardiac Hypertrophy in Cardiac Myocytes. Circulation 2001;104:1670-1675.

18. Belevych AE, Sansom SE, Terentyeva R, Ho H-T, Nishijima Y, Martin MM, Jindal HK, Rochira JA, Kunitomo Y, Abdellatif M, Carnes CA, Elton TS, Györke S, Terentyev D. MicroRNA-1 and -133 Increase Arrhythmogenesis in Heart Failure by Dissociating Phosphatase Activity from RyR2 Complex. PLoS ONE 2011;6:e28324.

19. Yang B, Lin H, Xiao J, Lu Y, Luo X, Li B, Zhang Y, Xu C, Bai Y, Wang H, Chen G, Wang Z. The muscle-specific microRNA miR-1 regulates cardiac arrhythmogenic potential by targeting GJA1 and KCNJ2. Nat Med 2007;13:486-491.

20. Aurora AB, Mahmoud AI, Luo X, Johnson BA, van Rooij E, Matsuzaki S, Humphries KM, Hill JA, Bassel-Duby R, Sadek HA, Olson EN. MicroRNA-214 protects the mouse heart from ischemic injury by controlling Ca2+ overload and cell death. The Journal of Clinical Investigation 2012;122:1222-1232.

21. Nishi H, Ono K, Horie T, Nagao K, Kinoshita M, Kuwabara Y, Watanabe S, Takaya T, Tamaki Y, Takanabe-Mori R, Wada H, Hasegawa K, Iwanaga Y, Kawamura T, Kita T, Kimura T. MicroRNA-27a Regulates Beta Cardiac Myosin Heavy Chain Gene Expression by Targeting Thyroid Hormone Receptor β1 in Neonatal Rat Ventricular Myocytes. Molecular and Cellular Biology 2011;31:744-755.

22. Sadegh MK, Ekman M, Rippe C, Uvelius B, Sward K, Albinsson S. Deletion of Dicer in Smooth Muscle Affects Voiding Pattern and Reduces Detrusor Contractility and Neuroeffector Transmission. PLoS ONE 2012;7:e35882.

23. Pan Z, Sun X, Shan H, Wang N, Wang J, Ren J, Feng S, Xie L, Lu C, Yuan Y, Zhang Y, Wang Y, Lu Y, Yang B. miR-101 Inhibited Post-Infarct Cardiac Fibrosis and Improved Left Ventricular Compliance via FOS/TGFβ1 Pathway. Circulation 2012.

24. Tijsen AJ, Pinto YM, Creemers EE. Non-cardiomyocyte microRNAs in heart failure. Cardiovasc Res 2012;93:573-582.

25. Senn S, Julious S. Measurement in clinical trials: a neglected issue for statisticians? Stat Med 2009;28:3189-3209.

26. Fedorov V, Mannino F, Zhang R. Consequences of dichotomization. Pharm Stat 2009;8:50-61.

## Example 2

## Identification of miRNAs of the Invention

The selection of the 4 miRNAs of the present invention was a long process, starting from an initial hypothesis that circulating miRNAs may be associated with remodelling post MI. The first step was to perform microarray experiments in blood samples from 2 small groups of MI patients, one with, and one without, remodelling. From the 695 miRNAs represented on the microarrays, we isolated 271 miRNAs that were differentially expressed between patients with and without remodelling. The complete data are in Table S1. To isolate from these 271 those with potential link to remodelling, we used a systems-based approach with interaction networks. This permitted the identification of 10 miRNAs with the highest probability of association with remodelling. From these, we selected miR-27a/-101/-150 because of their high level of expression and differential expression between remodelers and non-remodelers. Also included, and that were not derived from the systems-based approach, were miR-16/- 92a/486, because of their high expression and differential expression in microarrays, and because they had been noted in Goretti et al., J. Leukoc. Biol. (2013). We then measured these 6 miRNAs in 150 MI patients and observed that the 4 miRNAs of the invention, miR-16/27a/101/150 provide a predictive value over and above BNP.

In more detail:

**Procedure used to select the miRNAs of the Invention**

The working hypothesis was that circulating miRNAs are useful to predict left ventricular remodelling and clinical outcome after myocardial infarction (MI).

Multi-step procedure.

Microarray experiments ((Devaux, Y., Vausort, M, McCann, G. P., Zangrando, J., Kelly, D., Razvi, N., Zhang, L., Ng, L.L., Wagner, D.R:, Squire, I.B. (2013) MicroRNA-150. A novel marker of left ventricular remodeling after acute myocardial infarction. Circ. Cardiovasc Genet. 6:290-298)

Circulating miRNA expression profiles were established by using blood samples obtained at hospital discharge from 2 groups of 30 MI patients enrolled at the Leicester Hospital in UK (=derivation cohort). Patients were classified depending on whether they showed left ventricular (LV) remodelling at 6-month follow-up. LV function was assessed by echocardiographic analysis, and LV remodelling was assessed by the change (ΔEDV) in left ventricular end diastolic volume (LVEDV) between discharge and follow-up. LV remodelling was defined as any increase in LVEDV during follow-up. Patients with diabetes or a prior MI event were excluded. Patients with or without LV remodelling were matched by age, and had comparable cardiovascular risk factors. The rationale for this selection procedure was to avoid any bias due to a potential effect of confounding factors on circulating levels of miRNAs. Venous blood was collected in tubes citrated prior to hospital discharge [median of 176 (range 138-262) days after MI]. Plasma was harvested by centrifugation and stored at -80°C until assayed. Identical volumes of plasma samples from each of the 30 patients of a specific group were pooled to reach a final volume of 400 μL for each group. This pooling strategy has been described elsewhere [1]. The two pools were processed conjointly. Total RNA was extracted from these 2 pools of plasma using miRVana PARIS isolation kit (Applied Biosystems, Lennik, Belgium), dephosphorylated and labelled using miRNA Complete Labelling and hybridisation kit (Agilent Technologies, Massy, France). Hybridisation was performed on microarrays covering 695 miRNAs (Human Microarray Release 12.0 slides from Agilent Technologies, Santa Clara, CA). Four arrays were hybridised per group. Scanning was achieved with the Genepix 4000B Scanner (Molecular Devices, Sunnyvale, USA). Raw data were acquired with the Genepix Pro software (Molecular Devices). Spots flagged as absent and having a signal to noise ratio less than 3 were removed. Median values of replicate probes were background subtracted. Normalisation was performed by using normalizeQuantile function from the Bioconductor *limma* package [2]. The miRNAs detected on at least 2 arrays of each group were selected for further analysis. Microarray data are presented in Table S1.

Of the 695 miRNAs represented on microarrays, 160 miRNAs were detected in 1 or both groups of patients. Twenty-nine miRNAs were detected only in patients without remodelling. Overall, 271 miRNAs were differentially expressed between patients with and without remodelling (false discovery rate <0.05 using Statistical Analysis of Microarrays software; See Table S1). To isolate among these 271 miRNAs those with the highest probability of being associated with remodelling post MI, we used a systems-based approach.

Systems-based approach (Devaux, Y., Vausort, M, McCann, G. P., Zangrando, J., Kelly, D., Razvi, N., Zhang, L., Ng, L.L., Wagner, D.R:, Squire, I.B. (2013) MicroRNA-150. A novel marker of left ventricular remodeling after acute myocardial infarction. Circ. Cardiovasc Genet. 6:290-298)

1. We searched for seed genes known to be related to remodelling from NCBI gene database using the keywords: "ventricular remodelling" and "myocardial infarction". The search was limited to human genes. Proteins known to interact with the proteins encoded by seed genes were identified from the IntAct [7], DIP [8] an MINT [9] databases. Only the interactions found in at least two databases were selected for further analysis. Then all seed and interacting proteins were used as inputs to TargetScan [3], PicTar [4] and MicroCosm [5] databases to retrieve miRNAs predicted to have binding sites in the genes encoding these proteins. Gene annotation and identification of enriched KEGG (Kyoto Encyclopedia of Genes and Genomes) pathways were performed using the Database for Annotation, Visualisation and Integrated Discovery (DAVID) [6]. Only the miRNA-target pairs found in at least two databases were selected to build interaction networks. Networks were visualised with CytoScape [10] and Polar Mapper [11]. Node traffic estimation was computed with Polar Mapper. High traffic nodes can represent "cross-communication" hotspots or "bottlenecks" in the network.

Then we used these 13 seed proteins to retrieve from protein-protein interaction databases a list of 26 proteins (=interactors) known to interact with the 13 remodelling genes. See Table 6 and Figure 5A.

Then, we retrieved from miRNA-target databases a list of 265 miRNAs (Table 7) predicted to target the 13 seed genes or the genes encoding the 26 interacting proteins (Table 6). Using these genes and miRNAs, we built an interaction network (Figure 5B and 5C) in which nodes represent either proteins or miRNAs and edges represent either protein-protein interactions or miRNA-target pairs. Bioinformatic analysis of this network allowed isolation of the top 10 high-traffic miRNAs (hsa-miR-27a/-133a/-625/-296-3p/-31/-23a/-204/-19a/-101/-150). These miRNAs were predicted to regulate the expression of a high number of genes encoding proteins involved in remodelling and therefore potentially interesting as predictors of outcome after MI.

From these 10 high-traffic miRNAs, we selected miR-27a/-101/-150 because of their high level of expression and differential expression between remodelers and non-remodelers in the microarray experiments from 1. Differential expression between remodelers and non-remodelers was confirmed by quantitative PCR (Figure 5D).

Figure 5 shows systems-based identification of candidate miRNAs. Fig. 5A. Network of interactions between proteins known to be associated with LV remodelling in humans (dark grey nodes) and 26 interacting proteins (light grey). From the 13 proteins associated with LV remodelling, only 11 had known protein-protein interactions in at least 2 queried databases. B. Network of interactions between the 11 proteins associated with LV remodelling (dark grey), their 26 interactors light grey) and their 265 target miRNAs (medium grey). This network was built with CytoScape. C. Global view of the network containing 15 modules. This view was built with Polar Mapper. D. Discharge plasma levels of miR-27a/101/150 in 60 AMI patients of the derivation cohort, as measured by quantitative PCR. Patients with decreased end-diastolic volume between discharge and follow-up ($\Delta$EDV <0, n=30) had higher levels of miRNAs compared to patients with increased EDV ($\Delta$EDV >0, n=30). Means $\pm$ 95% CI are shown.

2. Selection of miR-16/-92a/486

We also selected miR-16/-92a/486, which were not derived from the systems-based approach, for the following reasons:

- high level of expression in microarrays from 1 (Table S1)
- strong differential expression between remodelers and non-remodelers in microarrays from 1 (Table S1)
- we previously showed that miR-16 played a role in the differentiation of endothelial progenitor cells, thus having a potential effect on remodelling post MI (Figure 6 and Goretti et al J Leukoc Biol 2013 May; 93(5):645-55).

Figure 6 shows expression of differentiation-related genes in early endothelial progenitor cells treated by anti-miR-16. Early endothelial progenitor cells obtained 4 days after plating PBMCs onto human fibronectin-coated plates were transfected with 30 nmol/l anti-miR-16, and expression of endothelial markers was assessed by flow cytometry after 24 h. Results are mean $\pm$ SD (n=3). #P < 0.05. Comparisons are between control and anti-miR-16 conditions.

3. Assessment of miR-16/-27a/-92a/-101/-150/486

We measured the plasma levels of these 6 miRNAs at hospital discharge in 150 MI patients from the Leicester Hospital and we evaluated their ability to predict remodelling, as assessed by the wall motion index score (index of cardiac contractility) obtained by echocardiography at 6-month follow-up. Nt-pro-BNP, a known predictor of outcome post MI, was also measured. Using multiple logistic regression, we deduced that LV contractility (dependent variable 1=WMIS<=1,2) can be predicted from a linear combination of the 4 miRNAs miR-16/27a/101/150 and Nt-pro-BNP as demonstrated below.

**Backward Stepwise**

**Regression:**

**Data source:** Data 1 in Log reg WMIS miRs BNP 150 UK echo

Dependent Variable:1=WMIS<=1,2

F-to-Enter: 4,000 P = 0,047

F-to-Remove: 3,900 P = 0,050

Step 0:

Standard Error of Estimate = 0,442

Analysis of Variance:

| Group | DF | SS | MS | F | P |
|---|---|---|---|---|---|
| Regression | 7 | 9,897 | 1,414 | 7,247 | <0,001 |
| Residual | 148 | 28,872 | 0,195 | | |

Variables in Model

| Group | Coef. | Std. Coeff. | Std. Error | F-to-Remove | P |
|---|---|---|---|---|---|
| Constant | 1,704 | | 0,408 | | |
| Log NTproBNP dis | -0,216 | -0,312 | 0,0524 | 16,996 | <0,001 |
| miR-92a | 0,119 | 0,115 | 0,318 | 0,140 | 0,708 |
| miR-16 | -0,227 | -0,271 | 0,167 | 1,844 | 0,176 |
| miR-27a | -0,598 | -0,559 | 0,168 | 12,748 | <0,001 |
| miR-150 | 0,506 | 0,424 | 0,148 | 11,620 | <0,001 |
| miR-486 | -0,236 | -0,228 | 0,256 | 0,848 | 0,359 |
| miR-101 | 0,345 | 0,349 | 0,142 | 5,908 | 0,016 |

Variables not in Model

| Group | F-to-Enter | P |
|---|---|---|

Step 1: miR-92a Removed

R = 0,505 Rsqr = 0,255 Adj Rsqr = 0,225

Standard Error of Estimate = 0,440

Analysis of Variance:

| Group | DF | SS | MS | F | P |
|---|---|---|---|---|---|
| Regression | 6 | 9,870 | 1,645 | 8,481 | <0,001 |
| Residual | 149 | 28,900 | 0,194 | | |

Variables in Model

| Group | Coef. | Std. Coeff. | Std. Error | F-to-Remove | P |
|---|---|---|---|---|---|
| Constant | 1,687 | | 0,404 | | |
| Log NTproBNP dis | -0,213 | -0,308 | 0,0517 | 17,007 | <0,001 |
| miR-16 | -0,194 | | | | |

(continued)

| **Group** | **Coef.** | **Std. Coeff.** | **Std. Error** | **F-to-** | |
|---|---|---|---|---|---|
| **Remove** | **P** | | | | |
| 0,232 | 0,142 | 1,873 | 0,173 | | |
| miR-27a | -0,574 | | | | |
| 0,537 | 0,155 | 13,776 | <0,001 | | |
| miR-150 | 0,509 | 0,427 | 0,148 | 11,894 | <0,001 |
| miR-486 | -0,166 | | | | |
| 0,160 | 0,174 | 0,905 | 0,343 | | |
| miR-101 | 0,336 | 0,340 | 0,140 | 5,802 | 0,017 |

Variables not in Model

| **Group** | **F-to-Enter** | **P** |
|---|---|---|
| miR-92a | 0,140 | 0,708 |

Step 2: miR-486 Removed

R = 0,500 Rsqr = 0,250 Adj Rsqr = 0,225

Standard Error of Estimate = 0,440

Analysis of Variance:

| **Group** | **DF** | **SS** | **MS** | **F** | **P** |
|---|---|---|---|---|---|
| Regression | 5 | 9,694 | 1,939 | 10,002 | <0,001 |
| Residual | 150 | 29,075 | 0,194 | | |

Variables in Model

| **Group** | | **Coef.** | **Std. Coeff.** | **Std. Error** | **F-to-** | |
|---|---|---|---|---|---|---|
| **Remove** | **P** | | | | | |
| Constant | | 1,824 | | 0,377 | | |
| Log NTproBNP dis | | -0,222 | - | | | |
| 0,321 | 0,0509 | | 19,104 | <0,001 | | |
| miR-16 | | -0,295 | - | | | |
| 0,354 | 0,0933 | | 10,024 | 0,002 | | |
| miR-27a | | -0,526 | - | | | |
| 0,492 | 0,146 | | 12,955 | <0,001 | | |
| miR- | | | | | | |
| 150 | | 0,474 | 0,398 | 0,143 | 11,006 | 0,001 |
| miR- | | | | | | |
| 101 | | 0,297 | 0,300 | 0,133 | 4,966 | 0,027 |

Variables not in Model

| **Group** | **F-to-Enter** | **P** |
|---|---|---|
| miR-92a | 0,192 | 0,662 |
| miR-486 | 0,905 | 0,343 |

Summary Table

| Step # | Vars. Entered | Vars. Removed | R | RSqr | Delta RSqr | Vars in Model |
|---|---|---|---|---|---|---|
| 1 | | miR- | | | | |
| 92a | 0,505 0,255 | 0,255 | 6 | | | |
| 2 | | miR-486 | 0,500 | 0,250 | - | |
| 0,00453 | 5 | | | | | |

The dependent variable 1=WMIS<=1,2 can be predicted from a linear combination of the independent variables:

| | **P** |
|---|---|
| Log NTproBNP dis | <0,001 |
| miR-16 | 0,002 |
| miR-27a | <0,001 |
| miR-150 | 0,001 |
| miR-101 | 0,027 |

The following variables did not significantly add to the ability of the equation to predict 1=WMIS<=1,2 and were not included in the final equation: miR-92a miR-486

| | |
|---|---|
| Normality Test (Shapiro-Wilk) | Failed (P = <0,001) |
| Constant Variance Test: | Passed (P = 0,352) |
| Power of performed test with alpha = 0,050: | 1,000 |

In the following Table S1, the abbreviation RMDG is used for 'remodelling'.

**Table S1**

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00010 086 | hsa-let-7a | 2348.98 | 1813.55 | 1951.14 | 1864.58 | 1483.1 4 | - | - | - | |
| A_25_P00011 584 | hsa-let-7a | 8741.11 | 5195.03 | 4958.22 | 4485.24 | 1797.6 1 | 1776.4 4 | 1665.9 8 | 1362.3 7 | 0.28 |
| A_25_P00010 070 | hsa-let-7b | 29444.14 | 22846.62 | 23211.94 | 25074.20 | 4104.8 1 | 2747.0 5 | 3027.1 3 | 2761.0 4 | 0.13 |
| A_25_P00010 071 | hsa-let-7b | 21619.43 | 14962.94 | 15872.33 | 15779.70 | 2173.0 1 | 1363.6 5 | 1846.2 1 | 1552.0 0 | 0.10 |
| A_25_P00010 072 | hsa-let-7c | 6851.21 | 3505.88 | 5691.86 | 5314.67 | - | 1217.7 3 | - | - | |
| A_25_P00010 073 | hsa-let-7c | 1466.53 | - | 1436.06 | - | - | - | - | 790.67 | |
| A_25_P00011 981 | hsa-let-7d | 4149.71 | 2870.29 | 2733.51 | 2595.09 | - | - | - | - | |
| A_25_P00013 126 | hsa-let-7d* | 1794.51 | 1636.57 | 1587.80 | 1439.40 | - | - | - | - | |
| A_25_P00013 127 | hsa-let-7d* | 3714.96 | 3209.46 | 2753.13 | 2796.02 | 1990.3 5 | - | 1625.8 3 | 1776.9 7 | 0.57 |
| A_25_P00010 088 | hsa-let-7f | 6214.62 | 4253.34 | 3421.54 | 3243.53 | 2063.0 5 | 1560.5 9 | - | 1713.7 9 | 0.44 |
| A_25_P00010 089 | hsa-let-7f | 1391.82 | 970.73 | 1429.16 | 923.72 | - | - | - | - | |
| A_25_P00012 141 | hsa-let-7g | 4936.85 | 3405.47 | 2446.58 | 2247.11 | - | - | - | - | |
| A_25_P00012 142 | hsa-let-7g | 24703.11 | 19365.82 | 13713.56 | 13196.24 | 2343.2 1 | 1793.6 9 | 1868.1 9 | 1483.6 2 | 0.11 |
| A_25_P00012 145 | hsa-let-7i | 15147.29 | 11074.96 | 9018.23 | 9361.91 | 2035.8 4 | 1624.4 8 | 1888.5 5 | 1843.6 3 | 0.17 |
| A_25_P00012 146 | hsa-let-7i | 28531.77 | 24780.89 | 18496.01 | 19146.15 | 4466.4 5 | 3135.3 0 | 3718.1 8 | 3643.0 7 | 0.16 |
| A_25_P00012 038 | hsa-miR-101 | 2735.74 | 1997.93 | 1712.68 | 1670.16 | - | - | - | - | |
| A_25_P00012 039 | hsa-miR-101 | 4982.77 | 3882.79 | 2885.33 | 2925.49 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00011 004 | hsa-miR-103 | 3862.08 | 3135.30 | 2188.26 | 2197.61 | - | - | - | - | |
| A_25_P00011 005 | hsa-miR-103 | 1826.47 | 1802.20 | 1367.90 | 1400.14 | - | - | - | - | |
| A_25_P00010 433 | hsa-miR-106b | 11412.79 | 9951.53 | 7114.67 | 6760.01 | 1076.7 9 | 2799.0 7 | 1592.1 9 | - | 0.21 |
| A_25_P00010 434 | hsa-miR-106b | 4320.39 | 3180.05 | 2869.00 | 2615.21 | - | - | - | - | |
| A_25_P00011 068 | hsa-miR-107 | 7748.12 | 5516.95 | 5584.48 | 4967.67 | 1734.6 3 | - | 1920.6 2 | 1795.8 5 | 0.30 |
| A_25_P00011 069 | hsa-miR-107 | 25772.17 | 21370.36 | 16326.74 | 16550.84 | 6495.3 8 | 5157.0 9 | 5323.5 3 | 5481.5 1 | 0.28 |
| A_25_P00015 059 | hsa-miR-1181 | 2614.28 | 2441.73 | 1738.60 | 1826.04 | - | - | - | - | |
| A_25_P00015 060 | hsa-miR-1181 | 1729.27 | 1523.81 | 1402.67 | 1373.74 | - | - | - | - | |
| A_25_P00015 061 | hsa-miR-1182 | 2719.90 | 2230.77 | 1803.18 | 1850.66 | - | - | - | - | |
| A_25_P00015 062 | hsa-miR-1182 | 2434.84 | 2006.95 | 1862.48 | 1777.42 | - | - | - | - | |
| A_25_P00015 063 | hsa-miR-1183 | 4540.49 | 4390.77 | 3767.45 | 3552.81 | - | - | - | - | |
| A_25_P00015 064 | hsa-miR-1183 | 4189.01 | 4020.32 | 3887.94 | 3764.22 | - | - | 1230.4 6 | - | |
| A_25_P00015 075 | hsa-miR-1202 | 3240.56 | 2418.10 | 3025.69 | 2866.50 | 938.71 | 1170.0 7 | 1246.3 6 | 1109.9 2 | 0.39 |
| A_25_P00015 076 | hsa-miR-1202 | 3104.27 | 2506.86 | 2866.50 | 3025.69 | 825.38 | 919.91 | 1123.5 4 | 1053.1 3 | 0.34 |
| A_25_P00015 087 | hsa-miR-1207-5p | 3487.71 | 2720.90 | 3475.83 | 3475.83 | 1092.8 9 | 1618.8 6 | 1363.9 2 | 1217.6 6 | 0.40 |
| A_25_P00015 088 | hsa-miR-1207-5p | 2982.72 | 2301.64 | 2697.50 | 3190.24 | 1039.7 5 | 1407.9 0 | 961.59 | 955.03 | 0.39 |
| A_25_P00012 153 | hsa-miR-122 | 1593.40 | 1292.34 | 1227.08 | 1216.36 | 2621.7 8 | 2293.5 7 | 2290.8 9 | 2246.7 8 | 1.77 |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00012 154 | hsa-miR-122 | 2808.00 | 2364.15 | 2135.90 | 1984.81 | 4607.6 1 | 3351.4 8 | 4166.2 9 | 4043.8 4 | 1.74 |
| A_25_P00014 906 | hsa-miR-1224-5p | 3523.91 | 3089.34 | 2697.20 | 2523.95 | - | - | - | - | |
| A_25_P00014 907 | hsa-miR-1224-5p | 3066.92 | 2587.07 | 2297.90 | 2310.16 | - | 1167.4 7 | - | - | |
| A_25_P00014 920 | hsa-miR-1225-5p | 47509.43 | 38955.59 | 39596.53 | 35820.80 | 25235. 45 | 32707. 22 | 23978. 31 | 23709. 73 | 0.65 |
| A_25_P00014 921 | hsa-miR-1225-5p | 36379.03 | 35625.04 | 33608.27 | 34647.35 | 21680. 51 | 29327. 63 | 22019. 22 | 20110. 79 | 0.66 |
| A_25_P00015 003 | hsa-miR-1226* | 2528.64 | 2121.39 | 1771.38 | 1610.52 | - | - | - | - | |
| A_25_P00015 004 | hsa-miR-1226* | 2228.36 | 2067.72 | 1339.53 | 1386.57 | - | - | - | - | |
| A_25_P00014 936 | hsa-miR-1228 | 1535.41 | 1316.02 | 1989.08 | 1954.01 | 1750.3 2 | - | - | 1371.1 7 | 0.91 |
| A_25_P00014 937 | hsa-miR-1228 | 1203.63 | - | 1625.02 | 1511.94 | - | - | - | - | |
| A_25_P00014 952 | hsa-miR-1234 | 1298.36 | - | - | 1168.64 | - | - | - | - | |
| A_25_P00014 953 | hsa-miR-1234 | - | - | 1237.89 | 1413.51 | - | - | - | 1147.7 1 | |
| A_25_P00015 142 | hsa-miR-1246 | 4372.21 | 2780.17 | 2061.17 | 2084.74 | - | - | - | - | |
| A_25_P00015 143 | hsa-miR-1246 | - | - | 49827.70 | 49732.79 | 13789. 26 | 14503. 64 | 13879. 65 | 11642. 95 | 0.30 |
| A_25_P00015 148 | hsa-miR-1249 | 1954.22 | 1711.48 | 2380.33 | 2286.38 | - | - | 1436.3 4 | 1413.0 7 | 0.72 |
| A_25_P00015 149 | hsa-miR-1249 | 1720.73 | 1722.63 | 2214.96 | 2143.35 | - | - | - | - | |
| A_25_P00015 158 | hsa-miR-1254 | 1106.16 | - | - | 1010.82 | - | - | - | - | |
| A_25_P00012 212 | hsa-miR-125a-3p | 1503.71 | 1644.45 | 1592.97 | 1724.45 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00012 213 | hsa-miR-125a-3p | 1785.97 | 1652.03 | 1564.09 | 1592.06 | - | 181.00 | - | - | |
| A_25_P00013 941 | hsa-miR-125a-3p | 1435.95 | 1574.28 | 1647.87 | 1547.01 | - | - | - | - | |
| A_25_P00012 215 | hsa.miR-126 | 2876.51 | 2404.97 | 2008.88 | 2002.34 | 1420.9 4 | - | - | - | |
| A_25_P00012 216 | hsa-miR-126 | 6308.96 | 5133.04 | 3817.96 | 3815.63 | 2731.7 3 | 1544.1 6 | 2340.1 0 | 1915.9 3 | 0.45 |
| A_25_P00010 600 | hsa-miR-126* | 1802.16 | 1409.89 | 1193.34 | 1195.03 | - | - | - | - | |
| A_25_P00015 194 | hsa-miR-1268 | 16459.56 | 15725.31 | 13092.60 | 13009.74 | 3862.8 5 | 2869.0 0 | 3177.9 5 | 3090.5 7 | 0.22 |
| A_25_P00015 195 | hsa-miR-1268 | 19770.13 | 16803.38 | 14066.09 | 13627.82 | 6109.7 4 | 4557.0 8 | 4697.6 3 | 4595.5 8 | 0.31 |
| A_25_P00015 230 | hsa-miR-1274b | 1887.56 | 1927.88 | 1839.01 | 1771.53 | - | - | - | - | |
| A_25_P00015 231 | hsa-miR-1274b | 2461.53 | 2380.33 | 2408.19 | 2225.83 | 1765.9 6 | - | 1407.2 0 | 1384.2 7 | 0.65 |
| A_25_P00015 209 | hsa-miR-1275 | 2278.19 | 1885.28 | 1847.10 | 1741.16 | - | - | - | - | |
| A_25_P00015 210 | hsa-miR-1275 | 20928.98 | 16442.00 | 15605.04 | 15476.14 | 4156.3 8 | 3904.4 1 | 4006.8 2 | 3444.4 8 | 0.23 |
| A_25_P00012 162 | hsa-miR-128 | 1480.98 | 1462.17 | 1008.33 | 901.17 | - | - | - | - | |
| A_25_P00015 239 | hsa-miR-1288 | 2079.08 | 1664.83 | - | - | - | - | - | - | |
| A_25_P00015 107 | hsa-miR-1290 | 11072.99 | 7793.28 | 6144.44 | 6097.09 | 3546.8 8 | 3064.9 4 | 3312.7 9 | 2793.7 8 | 0.41 |
| A_25_P00015 133 | hsa-miR-1305 | 4814.10 | 4348.32 | 1784.47 | 1924.95 | - | - | - | - | |
| A_25_P00015 134 | hsa-miR-1305 | 4039.18 | 3574.61 | 1612.25 | 1582.14 | - | - | - | - | |
| A_25_P00015 249 | hsa-miR-1308 | 8086.66 | 6639.02 | 4031.20 | 3358.41 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00015 250 | hsa-miR-1308 | 22943.53 | 18577.83 | 11143.82 | 9857.05 | - | - | - | - | |
| A_25_P00010 439 | hsa-miR-130a | 4480.22 | 3671.84 | 3226.44 | 3198.42 | 1852.0 5 | 1727.9 5 | 2042.9 7 | 1882.5 6 | 0.51 |
| A_25_P00010 440 | hsa-miR-130a | 3049.68 | 2712.43 | 2603.38 | 2462.61 | 1370.5 8 | 2160.8 0 | 1833.8 0 | 1464.2 4 | 0.63 |
| A_25_P00010 437 | hsa-miR-130b | 2143.21 | 1954.47 | 1755.21 | 1718.18 | - | - | - | - | |
| A_25_P00010 963 | hsa-miR-133b | 1941.14 | 1829.98 | 1580.06 | 1712.13 | - | - | 1393.1 1 | - | |
| A_25_P00012 230 | hsa-miR-134 | 11828.89 | 9820.55 | 11547.73 | 9986.73 | 4933.7 5 | 4122.0 0 | 4455.6 6 | 3538.9 | 0.39 |
| A_25_P00012 231 | hsa-miR-134 | 12295.71 | 10094.00 | 10495.47 | 9139.55 | 4466.4 5 | 4197.8 8 | 3842.7 1 | 3759.9 | 0.39 |
| A_25_P00013 406 | hsa-miR-135a* | 4081.86 | 3320.38 | 3298.33 | 3318.56 | 1901.0 0 | - | 1543.6 3 | - | 0.49 |
| A_25_P00013 407 | hsa-miR-135a* | 4886.05 | 4431.99 | 4563.75 | 4442.80 | 2380.3 3 | 1852.7 8 | 1642.9 4 | 1620.3 | 0.41 |
| A_25_P00012 074 | hsa-miR-139-3p | 2614.28 | 2345.86 | - | 1274.00 | - | - | - | - | |
| A_25_P00012 176 | hsa-miR-140-3p | 1419.91 | - | 1514.09 | 1646.89 | - | - | - | - | |
| A_25_P00012 177 | hsa-miR-140-3p | 3018.34 | 2696.10 | 2908.26 | 2817.82 | - | - | - | - | |
| A_25_P00011 016 | hsa-miR-142-3p | 2826.21 | 1983.36 | 1358.49 | - | - | - | - | - | |
| A_25_P00013 937 | hsa-miR-142-3p | 1672.63 | 1388.96 | - | - | - | - | - | - | |
| A_25_P00014 844 | hsa-miR-142-5p | 1609.72 | 1451.08 | - | 1369.71 | - | - | - | - | |
| A_25_P00012 188 | hsa-miR-144 | 6700.29 | 5025.98 | 2845.03 | 2746.06 | - | - | - | - | |
| A_25_P00012 189 | hsa-miR-144 | 8611.71 | 5743.56 | 3364.71 | 3155.81 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00010 078 | hsa-miR-146a | 4730.33 | 4103.21 | 3113.65 | 3101.88 | 2772.9 8 | 2109.2 6 | 2832.7 9 | 2669.2 3 | 0.69 |
| A_25_P00010 079 | hsa-miR-146a | 1509.19 | 1241.16 | 1285.45 | 1461.14 | - | - | - | - | |
| A_25_P00015 286 | hsa-miR-1471 | 3351.97 | 3017.41 | 3203.26 | 2972.58 | - | 1096.1 3 | 1368.8 8 | 1206.7 0 | 0.43 |
| A_25_P00015 287 | hsa-miR-1471 | 2981.03 | 2767.75 | 2774.29 | 2863.75 | 1199.3 5 | 2254.0 3 | - | - | 0.59 |
| A_25_P00010 131 | hsa-miR-148a | 1190.32 | 1401.30 | 1145.62 | 1066.57 | - | - | - | - | |
| A_25_P00010 132 | hsa-miR-148a | 1833.06 | 1211.48 | 1500.40 | 1392.65 | - | - | - | - | |
| A_25_P00010 133 | hsa-miR-148b | 1768.82 | 1353.55 | 1423.13 | 1420.13 | - | - | - | - | |
| A_25_P00013 447 | hsa-miR-149* | 1161.70 | - | - | 1307.84 | - | - | - | - | |
| A_25_P00013 448 | hsa-miR-149* | 1475.69 | - | 920.26 | 1361.12 | - | - | - | - | |
| A_25_P00013 449 | hsa-miR-149* | 2588.03 | 2206.83 | 1983.15 | 1877.08 | 2103.0 5 | - | - | - | |
| A_25_P00010 490 | hsa-miR-150 | 1639.15 | 1553.32 | 1463.14 | 1554.51 | - | - | - | - | |
| A_25_P00014 846 | hsa-miR-150 | 6937.20 | 5641.82 | 5021.75 | 5087.59 | - | - | - | - | |
| A_25_P00014 847 | hsa-miR-150 | 15575.81 | 14302.65 | 10068.76 | 11380.52 | - | - | - | - | |
| A_25_P00013 450 | hsa-miR-150* | 3676.01 | 2929.55 | 5296.48 | 5448.53 | 3748.8 5 | 2994.5 6 | 2877.0 2 | 3041.6 1 | 0.73 |
| A_25_P00013 451 | hsa-miR-150* | 2897.45 | 2671.97 | 4114.02 | 3965.83 | 2446.1 8 | 1958.1 7 | 2714.2 5 | 2193.3 4 | 0.68 |
| A_25_P00013 452 | hsa-miR-150* | 2864.83 | 2607.10 | 3659.33 | 3655.59 | 1810.4 9 | 1760.8 0 | 2120.0 5 | 2380.3 3 | 0.63 |
| A_25_P00013 453 | hsa-miR-150* | 4110.26 | 3254.61 | 4246.84 | 4287.07 | 1877.6 2 | 2645.7 6 | 1951.8 5 | 1989.4 9 | 0.53 |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00012 376 | hsa-miR-151-5p | 1864.22 | 1750.71 | 1480.46 | 1454.62 | - | - | 1258.7 9 | - | |
| A_25_P00010 467 | hsa-miR-15a | 16787.82 | 13498.30 | 8772.42 | 8336.53 | 2263.6 9 | 2109.2 6 | 2755.8 2 | 2117.6 7 | 0.20 |
| A_25_P00014 817 | hsa-miR-15a | 38555.57 | 30074.19 | 21557.93 | 21375.55 | 7017.7 2 | 5328.4 5 | 5641.8 2 | 5767.8 4 | 0.21 |
| A_25_P00011 101 | hsa-miR-15b | 35580.93 | 27543.59 | 19340.24 | 19904.83 | 6270.9 7 | 4720.9 6 | 4891.3 7 | 4780.1 2 | 0.20 |
| A_25_P00011 102 | hsa-miR-15b | 9542.93 | 7958.86 | 5208.92 | 5228.68 | 1634.9 1 | 1278.8 1 | 1493.4 7 | 1235.3 5 | 0.20 |
| A_25_P00010 599 | hsa-miR-16 | 23090.50 | 12324.12 | 8655.23 | 10161.87 | 5302.7 3 | 3759.5 7 | 4886.0 3 | 2493.6 1 | 0.30 |
| A_25_P00014 818 | hsa-miR-16 | 11726.85 | 5152.57 | 5600.67 | 6490.04 | 1297.6 3 | 665.41 | 1059.2 0 | 892.25 | 0.14 |
| A_25_P00013 271 | hsa-miR-16-2* | 4597.59 | 2821.25 | 2322.62 | 2351.55 | - | - | - | - | |
| A_25_P00011 991 | hsa-miR-17 | 1919.01 | 1772.43 | 1922.02 | 1816.90 | - | - | - | - | |
| A_25_P00013 841 | hsa-miR-17 | 6574.66 | 4754.93 | 5075.26 | 4732.36 | - | - | - | - | |
| A_25_P00014 819 | hsa-miR-17 | 13988.03 | 13139.22 | 9757.08 | 10654.79 | 1590.6 0 | - | - | - | |
| A_25_P00010 285 | hsa-miR-181a | 1544.36 | 1326.13 | 1055.70 | - | - | - | - | - | |
| A_25_P00014 832 | hsa-miR-181a | 1774.66 | 1806.55 | 1477.32 | 1565.46 | - | - | - | - | |
| A_25_P00012 098 | hsa-miR-183 | 1471.37 | - | - | 1204.16 | - | - | - | - | |
| A_25_P00012 238 | hsa-miR-185 | 4127.08 | 2975.37 | 2802.25 | 2689.72 | - | - | - | - | |
| A_25_P00012 239 | hsa-miR-185 | 6422.90 | 6087.57 | 5405.52 | 5151.43 | - | - | - | - | |
| A_25_P00012 243 | hsa-miR-186 | 1812.06 | 1629.36 | 1507.49 | 1290.82 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00013 459 | hsa-miR-186* | 1892.32 | 1757.54 | - | - | - | 11951 6 | - | - | |
| A_25_P00013 324 | hsa-miR-187* | - | - | 1494.38 | 1638.91 | - | - | 1577.2 1 | - | |
| A_25_P00013 325 | hsa-miR-187* | 1616.16 | 1618.14 | 1910.85 | 1886.39 | 1362.5 1 | 1473.1 5 | 1992.6 0 | 1634.3 4 | 0.92 |
| A_25_P00013 326 | hsa-miR-187* | 1749.03 | 1546.41 | 1701.46 | 1784.88 | - | - | - | - | |
| A_25_P00013 327 | hsa-miR-187* | 1852.63 | 1728.90 | 1847.10 | 1803.45 | 1249.3 4 | - | 1459.9 3 | - | 0.80 |
| A_25_P00012 246 | hsa-miR-188-5p | 5451.94 | 4605.59 | 4134.62 | 4030.72 | 1974.8 5 | 17156 5 | 2075.9 4 | 2039.8 8 | 0.43 |
| A_25_P00012 247 | hsa-miR-188-5p | 5788.52 | 4914.99 | 4481.47 | 4114.23 | 2216.3 5 | 1690.9 5 | 1975.5 2 | 1687.0 9 | 0.39 |
| A_25_P00013 569 | hsa-miR-18b* | 1036.56 | 1374.22 | - | - | - | - | - | - | |
| A_25_P00015 315 | hsa-miR-1911* | 1425.04 | 1000.98 | - | - | - | - | - | 866.60 | |
| A_25_P00015 304 | hsa-miR-1914* | 4246.39 | 3773.70 | 2342.97 | 2391.82 | 1924.5 8 | 1887.8 3 | 1522.5 4 | 1475.5 8 | 0.53 |
| A_25_P00015 305 | hsa-miR-1914* | 3319.19 | 3069.81 | 2152.48 | 2109.45 | 1840.7 8 | 1412.5 6 | 1888.5 5 | 1531.1 9 | 0.63 |
| A_25_P00015 302 | hsa-miR-1915 | 33665.82 | 33427.92 | 24285.86 | 24101.99 | 7419.6 0 | 6537.5 9 | 6895.4 8 | 6472.8 4 | 0.24 |
| A_25_P00015 303 | hsa-miR-1915 | 39618.34 | 40177.03 | 27089.27 | 25612.34 | 8321.0 5 | 7134.9 5 | 5922.1 3 | 7037.9 9 | 0.21 |
| A_25_P00010 868 | hsa-miR-192 | 1881.22 | 1851.10 | 1619.18 | 1582.14 | - | - | - | - | |
| A_25_P00010 869 | hsa-miR-192 | 1339.87 | 1016.27 | - | - | - | - | - | - | |
| A_25_P00013 597 | hsa-miR-193b* | 1910.14 | 1523.81 | - | - | - | - | - | - | |
| A_25_P00010 769 | hsa-miR-195 | 2266.03 | 1846.21 | 1486.64 | 1599.48 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00012 052 | hsa-miR-196a | 1563.64 | 1362.93 | - | - | - | - | - | - | |
| A_25_P00010 835 | hsa-miR-197 | 1440.76 | 1682.21 | 1383.84 | 1565.46 | 1381.3 6 | - | 1316.0 2 | - | 0.95 |
| A_25_P00012 058 | hsa-miR-198 | 1493.01 | - | 1201.16 | 1187.26 | - | - | - | - | |
| A_25_P00012 059 | hsa-miR-198 | 2771.61 | 2250.31 | 2237.52 | 2428.89 | - | - | - | - | |
| A_25_P00012 063 | hsa-miR-199a-3p | 1837.88 | 1717.55 | 1389.86 | 1405.68 | 1454.7 6 | - | 1446.9 5 | - | 0.94 |
| A_25_P00012 064 | hsa-miR-199a-3p | 2705.47 | 2041.04 | 1833.28 | 1882.88 | 1886.5 3 | 1576.6 8 | - | 1451.4 9 | 0.78 |
| A_25_P00010 997 | hsa-miR-19a | 10803.90 | 10775.40 | 6690.15 | 6485.16 | 1674.0 4 | 1591.6 5 | 1698.3 5 | 1426.9 0 | 0.18 |
| A_25_P00010 998 | hsa-miR-19a | 7193.29 | 6525.33 | 4081.86 | 4220.43 | - | - | - | - | |
| A_25_P00010 999 | hsa-miR-19b | 32110.29 | 34539.31 | 20067.80 | 20610.08 | 4238.1 5 | 2707.9 6 | 3608.0 5 | 4204.6 7 | 0.14 |
| A_25_P00011 000 | hsa-miR-19b | 14799.77 | 11868.80 | 7772.19 | 7195.12 | 1651.7 3 | 1348.2 7 | - | 1971.0 0 | 0.17 |
| A_25_P00010 612 | hsa-miR-20a | 22276.33 | 20031.80 | 14344.64 | 14572.25 | 2841.2 1 | 1372.1 2 | 1801.7 3 | 1892.4 2 | 0.11 |
| A_25_P00010 613 | hsa-miR-20a | 14483.73 | 12937.37 | 9256.24 | 10163.12 | 1621.9 1 | - | 1507.5 8 | 1301.4 0 | 0.14 |
| A_25_P00010 614 | hsa-miR-20b | 10535.38 | 8579.68 | 6549.16 | 6249.98 | - | - | - | - | |
| A_25_P00010 615 | hsa-miR-20b | 3233.25 | 2729.40 | 2495.78 | 2488.70 | - | - | - | - | |
| A_25_P00010 975 | hsa-miR-21 | 41195.24 | 54778.63 | 25717.32 | 26869.90 | 12912. 91 | 8535.0 7 | 11054. 43 | 10848. 91 | 0.29 |
| A_25_P00010 976 | hsa-miR-21 | 19131.87 | 16072.42 | 10280.53 | 8539.80 | 5337.4 8 | 4045.3 2 | 5022.6 6 | 5076.2 0 | 0.36 |
| A_25_P00010 204 | hsa-miR-22 | 25276.16 | 25334.53 | 16656.18 | 16206.64 | 9589.2 0 | 6125.3 3 | 8602.9 5 | 9375.4 5 | 0.40 |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00010 205 | hsa-miR-22 | 9325.84 | 7612.29 | 6458.21 | 5863.04 | 3021.8 7 | 2537.6 4 | 3519.1 1 | 3247.0 1 | 0.42 |
| A_25_P00010 690 | hsa-miR-221 | 2484.93 | 2152.10 | 1718.38 | 1630.38 | 1393.9 4 | 1523.3 7 | - | - | 0.74 |
| A_25_P00012 130 | hsa-miR-223 | 55159.34 | 42864.08 | 20761.73 | 23023.46 | 9111.4 4 | 6868.0 3 | 5149.6 4 | 6051.9 2 | 0.19 |
| A_25_P00012 131 | hsa-miR-223 | 6307.73 | 5659.90 | 3190.24 | 4016.76 | 2338.2 3 | 1287.9 8 | 705.99 | 829.67 | 0.27 |
| A_25_P00010 843 | hsa-miR-23a | 5909.30 | 4851.15 | 4392.02 | 4248.46 | 3290.2 2 | 2341.2 0 | 3078.8 1 | 2939.3 4 | 0.60 |
| A_25_P00014 820 | hsa-miR-23a | 17587.72 | 15432.58 | 12310.75 | 11870.67 | 8662.8 2 | 6319.0 1 | 6133.4 3 | 7506.9 6 | 0.50 |
| A_25_P00010 881 | hsa-miR-23b | 1364.82 | 1082.62 | - | 1465.80 | - | - | - | - | |
| A_25_P00010 676 | hsa-miR-24 | 6148.67 | 6002.10 | 4644.62 | 4517.29 | 3471.7 6 | 1906.0 8 | 2926.5 3 | 2838.7 7 | 0.52 |
| A_25_P00010 677 | hsa-miR-24 | 5598.03 | 5356.04 | 4284.01 | 4173.66 | 3222.9 7 | 1931.2 4 | 2661.1 8 | 2598.8 8 | 0.54 |
| A_25_P00010 989 | hsa-miR-25 | 37300.08 | 36473.75 | 28948.43 | 28754.63 | 4773.7 1 | 3474.7 4 | 4357.4 8 | 4948.4 9 | 0.13 |
| A_25_P00010 990 | hsa-miR-25 | 16149.80 | 13715.62 | 12788.39 | 12597.53 | 1827.8 1 | - | 1732.7 6 | 1810.7 3 | 0.14 |
| A_25_P00011 998 | hsa-miR-26a | 1600.43 | - | - | 841.10 | - | - | - | - | |
| A_25_P00011 999 | hsa-miR-26a | 2913.61 | 2504.67 | 1777.29 | 1755.21 | 1606.8 9 | - | - | 1111.3 6 | 0.68 |
| A_25_P00012 001 | hsa-miR-26b | 9147.38 | 7020.54 | 4219.11 | 4081.86 | - | - | - | - | |
| A_25_P00012 002 | hsa-miR-26b | 11962.35 | 10296.38 | 6071.32 | 6163.54 | 1545.1 3 | 1061.9 2 | - | - | 0.20 |
| A_25_P00010 797 | hsa-miR-27a | 2497.82 | 2052.05 | 2034.14 | 2038.33 | 1476.0 9 | - | - | 1514.2 0 | 0.72 |
| A_25_P00014 821 | hsa-miR-27a | 5081.66 | 4540.98 | 3604.13 | 3294.24 | 2531.3 0 | 1826.1 1 | 2521.1 1 | 2152.9 | 0.55 |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00010 761 | hsa-miR-27b | 1452.80 | - | - | 1322.99 | - | - | - | - | |
| A_25_P00014 837 | hsa-miR-27b | 2294.59 | 1765.12 | 1885.80 | 1891.76 | 2137.4 9 | 1610.3 8 | 1757.9 9 | 1676.1 | 0.92 |
| A_25_P00013 978 | hsa-miR-296-5p | - | - | 1473.74 | 1536.83 | - | - | - | - | |
| A_25_P00012 012 | hsa-miR-29a | 861.17 | - | 1467.46 | - | 2495.4 2 | - | - | - | |
| A_25_P00012 013 | hsa-miR-29a | 3928.83 | 2821.25 | 2623.11 | 2651.16 | 1510.9 5 | 1327.4 0 | 1612.1 1 | 1266.3 6 | 0.48 |
| A_25_P00010 053 | hsa-miR-29b | 1397.54 | 1335.22 | - | - | - | - | - | - | |
| A_25_P00012 274 | hsa-miR-29c | 3196.00 | 2306.25 | 2100.61 | 2012.98 | 1433.6 4 | 1398.5 8 | - | - | 0.63 |
| A_25_P00012 275 | hsa-miR-29c | 3809.34 | 2747.05 | 2512.35 | 2332.81 | - | - | 1422.4 0 | - | |
| A_25_P00010 839 | hsa-miR-301a | 1664.94 | 1437.38 | - | - | - | - | - | - | |
| A_25_P00014 838 | hsa-miR-30b | 1677.49 | 1441.86 | - | 1353.43 | - | - | - | - | |
| A_25_P00013 489 | hsa-miR-30c-1* | 1371.74 | 1231.15 | 1671.04 | 1763.35 | - | - | - | - | |
| A_25_P00010 682 | hsa-miR-30d | 5038.42 | 4966.31 | 4720.96 | 4350.14 | 1720.3 6 | - | 1560.5 9 | 1860.95 | 0.37 |
| A_25_P00010 683 | hsa-miR-30d | 4275.98 | 3956.16 | 3705.46 | 3479.28 | 1143.0 9 | 1495.0 1 | 1680.12 | - | 0.39 |
| A_25_P00012 300 | hsa-miR-30e | 1553.98 | 1394.64 | 1556.55 | 1257.47 | - | - | - | - | |
| A_25_P00012 301 | hsa-miR-30e | 3587.89 | 2907.69 | 2824.38 | 2708.46 | - | - | - | 993.75 | |
| A_25_P00012 261 | hsa-miR-320a | 2333.56 | 2137.01 | 3165.39 | 3054.23 | 1524.8 9 | - | - | - | |
| A_25_P00012 262 | hsa-miR-320a | 4404.56 | 3298.17 | 6223.37 | 6004.16 | 1575.7 1 | - | 1757.9 9 | 1726.96 | 0.35 |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00015 034 | hsa-miR-320b | 2245.39 | 2083.53 | 3029.78 | 3019.11 | - | - | 2789.34 | - | |
| A_25_P00015 035 | hsa-miR-320b | 7877.66 | 6805.61 | 7949.97 | 8058.85 | 3644.6 1 | 2202.8 4 | 9975.50 | 2500.96 | 0.36 |
| A_25_P00015 036 | hsa-miR-320c | 58697.63 | 37632.37 | 41712.36 | 39530.19 | 11635. 93 | 10231.01 | 9573.1 | 9889.64 | 0.24 |
| A_25_P00015 037 | hsa-miR-320c | - | 58697.63 | 45295.50 | 41566.22 | 11999. 23 | 9885.12 | 4 | 10262.05 | 0.22 |
| A_25_P00015 270 | hsa-miR-320d | 6016.13 | 5873.46 | 4900.55 | 4910.85 | 2881.9 1 | 1999.4 7 | 2248.9 4 | 2543.5 8 | 0.45 |
| A_25_P00015 271 | hsa-miR-320d | 13011.90 | 14042.91 | 11939.00 | 11705.41 | 4014.1 1 | 2799.0 7 | 3405.4 7 | 3894.0 8 | 0.28 |
| A_25_P00010 539 | hsa-miR-324-3p | 1884.48 | 1906.08 | 1570.87 | 1679.18 | - | - | - | - | |
| A_25_P00010 540 | hsa-miR-324-3p | 1526.49 | - | 1025.77 | 1029.92 | - | - | - | - | |
| A_25_P00012 396 | hsa-miR-338-3p | - | 897.89 | 1640.26 | - | - | - | - | - | |
| A_25_P00012 402 | hsa-miR-339-3p | 2001.86 | 1489.36 | - | 1991.60 | - | - | - | - | |
| A_25_P00012 403 | hsa-miR-339-3p | 1653.23 | 1514.01 | - | 1082.51 | - | - | - | - | |
| A_25_P00012 404 | hsa-miR-339-3p | 1714.06 | 1482.27 | - | - | - | - | - | - | |
| A_25_P00012 357 | hsa-miR-342-3p | 1178.46 | 1623.76 | 1376.36 | 1426.23 | - | - | - | - | |
| A_25_P00012 358 | hsa-miR-342-3p | 3299.68 | 2886.76 | 2670.72 | 2726.70 | - | - | - | - | |
| A_25_P00010 953 | hsa-miR-363 | 5262.75 | 4228.33 | 3484.83 | 3518.51 | - | - | - | - | |
| A_25_P00010 954 | hsa-miR-363 | 3217.47 | 2490.92 | 2262.54 | 2179.48 | - | - | - | - | |
| A_25_P00013 991 | hsa-miR-369-3p | - | - | - | - | - | 666.22 | - | 1074.8 2 | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00012 323 | hsa-miR-371-5p | 1589.21 | - | 1528.99 | 1333.03 | - | - | - | - | |
| A_25_P00012 324 | hsa-miR-371-5p | 1118.69 | - | 1396.23 | 1115.42 | - | - | - | - | |
| A_25_P00012 418 | hsa-miR-423-5p | 1448.42 | - | 1677.33 | 1624.12 | - | - | - | - | |
| A_25_P00012 419 | hsa-miR-423-5p | 3261.31 | 2469.88 | 3522.71 | 3413.13 | 1497.4 4 | - | - | - | |
| A_25_P00011 109 | hsa-miR-424 | 1755.21 | 1588.84 | - | 1231.03 | - | - | - | - | |
| A_25_P00010 977 | hsa-miR-425 | 5683.42 | 4683.19 | 3967.45 | 3885.39 | - | - | - | - | |
| A_25_P00014 045 | hsa-miR-425 | 3082.05 | 2523.15 | 2532.06 | 2503.81 | - | - | - | - | |
| A_25_P00012 446 | hsa-miR-451 | Saturation | Saturation | Saturation | Saturation | 9787.8 4 | 9268.9 6 | 9094.77 | 6829.9 3 | |
| A_25_P00012 459 | hsa-miR-483-5p | 13506.07 | 12092.45 | 10809.71 | 10416.88 | 2799.6 7 | 2380.3 3 | 2596.2 9 | 2452.3 7 | 0.22 |
| A_25_P00014 861 | hsa-miR-483-5p | 20355.81 | 17717.45 | 14693.80 | 14258.34 | 5053.2 4 | 4266.7 9 | 4535.8 4 | 4277.5 8 | 0.27 |
| A_25_P00010 431 | hsa-miR-484 | 1960.49 | 1560.59 | 2023.26 | 2026.51 | - | - | - | - | |
| A_25_P00010 432 | hsa-miR-484 | - | - | 1451.27 | 1439.40 | - | - | - | - | |
| A_25_P00014 063 | hsa-miR-486-5p | 8932.75 | 7344.23 | 6490.04 | 7509.94 | 1773.7 6 | 788.28 | 1549.1 6 | 1706.1 0 | 0.16 |
| A_25_P00014 064 | hsa-miR-486-5p | 3914.86 | 4746.15 | 4581.74 | 5059.55 | 717.52 | 614.80 | 764.03 | 769.61 | 0.19 |
| A_25_P00010 688 | hsa-miR-498 | 1681.05 | 1255.10 | - | - | - | - | - | - | |
| A_25_P00012 624 | hsa-miR-499-5p | 1077.91 | - | 946.91 | - | 1223.9 5 | - | 1476.1 8 | - | 1.13 |
| A_25_P00012 625 | hsa-miR-499-5p | 1444.06 | 1211.48 | - | - | - | - | 1358.9 7 | 1440.8 9 | 1.01 |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00014 918 | hsa-miR-509-3-5p | 1986.67 | 1838.30 | 1182.93 | 1439.40 | - | - | - | - | |
| A_25_P00012 660 | hsa-miR-513a-3p | 1052.51 | 1466.23 | - | - | - | - | - | - | |
| A_25_P00012 618 | hsa-miR-516a-5p | 1331.00 | - | 1972.98 | - | - | - | - | - | |
| A_25_P00014 151 | hsa-miR-516a-5p | 3392.88 | 3228.95 | 1882.17 | 1938.29 | - | - | - | - | |
| A_25_P00010 563 | hsa-miR-520b | 1520.87 | 1143.87 | - | - | - | - | - | - | |
| A_25_P00012 692 | hsa-miR-532-3p | - | 670.31 | 898.92 | - | - | - | - | - | |
| A_25_P00010 344 | hsa-miR-557 | 1693.99 | 1425.30 | 1363.65 | 1479.12 | - | - | - | - | |
| A_25_P00010 345 | hsa-miR-557 | 1874.76 | - | 1267.82 | - | - | - | - | - | |
| A_25_P00011 096 | hsa-miR-572 | 8932.92 | 8716.79 | 8609.28 | 7834.06 | 1180.0 4 | - | - | - | |
| A_25_P00011 097 | hsa-miR-572 | 1258.29 | - | 1539.94 | - | - | - | - | - | |
| A_25_P00012 724 | hsa-miR-574-5p | 2401.47 | 1736.22 | 2785.41 | 2781.46 | 5895.8 2 | 3552.5 7 | 9049.7 1 | 4489.7 5 | 2.37 |
| A_25_P00012 725 | hsa-miR-574-5p | 4507.02 | 2540.08 | 4345.26 | 3702.58 | 7831.2 5 | 5912.8 6 | 10428. 21 | 6222.0 9 | 2.01 |
| A_25_P00010 808 | hsa-miR-575 | 7370.09 | 6445.98 | 4819.29 | 4394.78 | - | - | - | - | |
| A_25_P00014 896 | hsa-miR-575 | 7574.04 | 6915.84 | 5139.00 | 4835.06 | - | - | - | - | |
| A_25_P00010 891 | hsa-miR-583 | 2065.00 | 1793.69 | - | - | - | - | - | - | |
| A_25_P00010 892 | hsa-miR-583 | 2545.08 | 2169.56 | 1457.41 | - | - | 1808.1 7 | - | - | |
| A_25_P00010 634 | hsa-miR-584 | 1286.30 | - | 1112.20 | - | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00010 640 | hsa-miR-601 | 4670.72 | 4147.94 | 1794.68 | 1839.77 | - | - | - | - | |
| A_25_P00010 641 | hsa-miR-601 | 3450.20 | 3539.43 | 1898.73 | 1700.52 | - | - | - | - | |
| A_25_P00010 642 | hsa-miR-601 | 3634.29 | 3467.86 | 1935.19 | 1808.76 | - | - | - | - | |
| A_25_P00010 643 | hsa-miR-601 | 2842.98 | 4124.56 | 1959.54 | 2049.87 | - | - | - | - | |
| A_25_P00010 805 | hsa-miR-622 | 1486.23 | 1292.34 | 1875.33 | 1962.31 | - | - | - | - | |
| A_25_P00010 806 | hsa-miR-622 | 1705.92 | - | 2119.69 | 2067.03 | - | - | - | - | |
| A_25_P00010 807 | hsa-miR-622 | 1568.78 | 1368.66 | 2078.21 | 1975.95 | - | - | - | - | |
| A_25_P00010 226 | hsa-miR-623 | 1146.94 | 1269.05 | - | 1343.47 | - | - | - | - | |
| A_25_P00010 227 | hsa-miR-623 | 3768.51 | 3051.49 | 2714.75 | 2543.21 | - | - | - | - | |
| A_25_P00010 228 | hsa-miR-623 | 1899.87 | 1688.01 | 1747.28 | 1795.19 | - | - | - | - | |
| A_25_P00010 229 | hsa-miR-623 | 1980.89 | 1678.27 | 1816.30 | 1617.97 | - | - | - | - | |
| A_25_P00010 248 | hsa-miR-630 | - | - | 58697.63 | 54383.61 | 10359. 80 | 9391.5 3 | 8159.6 4 | 8845.4 9 | 0.19 |
| A_25_P00010 249 | hsa-miR-630 | - | - | 54428.09 | 58697.63 | 9962.5 4 | 8886.4 3 | 7706.9 2 | 8500.7 8 | 0.18 |
| A_25_P00010 402 | hsa-miR-638 | 12537.73 | 6451.63 | 5059.55 | 5600.67 | 4237.1 3 | 4987.3 1 | 2385.9 2 | 1985.1 1 | 0.46 |
| A_25_P00010 403 | hsa-miR-638 | 51388.09 | 50575.71 | 31736.55 | 29844.41 | 10901. 74 | 8025.6 6 | 6348.1 3 | 6764.5 2 | 0.20 |
| A_25_P00012 834 | hsa-miR-652 | 1845.44 | 1593.59 | 1308.95 | 1307.84 | - | - | - | - | |
| A_25_P00010 459 | hsa-miR-660 | 1553.98 | 1478.56 | 1371.66 | 1476.23 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00010 799 | hsa-miR-663 | 10362.67 | 10492.30 | 13259.60 | 13393.78 | - | - | - | - | |
| A_25_P00010 800 | hsa-miR-663 | 4224.00 | 3623.73 | 5824.41 | 5721.62 | - | - | - | - | |
| A_25_P00013 004 | hsa-miR-665 | 1225.56 | 940.74 | - | - | - | - | - | - | |
| A_25_P00012 860 | hsa-miR-671-5p | 2936.63 | 2626.13 | 2476.65 | 2371.75 | 2010.3 1 | 1742.9 8 | 1815.7 7 | 1830.0 6 | 0.71 |
| A_25_P00012 861 | hsa-miR-671-5p | 4444.95 | 3819.02 | 2985.43 | 2879.20 | 1778.7 3 | 1678.2 7 | 1476.1 8 | 1761.0 6 | 0.47 |
| A_25_P00012 078 | hsa-miR-7 | 1626.30 | - | - | 1471.32 | - | - | - | - | |
| A_25_P00012 971 | hsa-miR-708 | 1778.81 | 1495.71 | - | - | - | - | - | - | |
| A_25_P00015 264 | hsa-miR-720 | - | - | - | - | 1560.5 9 | 1131.4 3 | - | 2289.4 9 | |
| A_25_P00015 265 | hsa-miR-720 | 3160.42 | 2561.40 | 3251.15 | 3223.63 | 5171.3 8 | 3659.3 3 | 4106.7 0 | 4393.5 2 | 1.42 |
| A_25_P00011 341 | hsa-miR-765 | 2186.03 | 2019.36 | 1631.37 | 1526.11 | - | - | - | - | |
| A_25_P00011 342 | hsa-miR-765 | 5330.33 | 4504.49 | 3059.04 | 2768.80 | 1406.7 5 | - | - | - | |
| A_25_P00011 232 | hsa-miR-769-3p | 1271.76 | 1456.59 | 2932.70 | 2837.84 | - | - | - | - | |
| A_25_P00012 918 | hsa-miR-874 | 1645.76 | 1431.14 | - | - | - | - | - | - | |
| A_25_P00012 919 | hsa-miR-874 | 1735.89 | 1881.85 | 1329.19 | 1449.00 | - | - | - | - | |
| A_25_P00012 997 | hsa-miR-877 | 1414.65 | 1471.44 | 1096.13 | - | - | - | - | - | |
| A_25_P00012 998 | hsa-miR-877 | 1740.95 | 1507.91 | 1055.70 | 1242.29 | - | - | - | - | |
| A_25_P00012 999 | hsa-miR-877 | 1685.28 | 1611.61 | 1659.56 | 1505.54 | - | - | - | - | |

(continued)

| Probe ID | miRNA name | No RMDG 1 | No RMDG 2 | No RMDG 3 | No RMDG 4 | RMDG 1 | RMDG 2 | RMDG 3 | RMDG 4 | Fold change RMDG/no RMDG |
|---|---|---|---|---|---|---|---|---|---|---|
| A_25_P00013 001 | hsa-miR-887 | 2021.91 | 1916.38 | - | - | - | - | - | - | |
| A_25_P00012 927 | hsa-miR-891b | 806.71 | 1601.58 | - | - | - | - | - | - | |
| A_25_P00013 050 | hsa-miR-923 | 15307.00 | 14563.76 | 7509.94 | 8655.23 | 7126.5 6 | 6620.7 1 | 6458.5 5 | 5115.5 9 | 0.55 |
| A_25_P00012 030 | hsa-miR-92a | 44095.86 | 46336.34 | 34753.18 | 33433.28 | 6776.3 3 | 5641.8 2 | 6566.1 8 | 7939.3 6 | 0.17 |
| A_25_P00012 031 | hsa-miR-92a | 5612.61 | 4270.14 | 4016.76 | 4581.74 | 1185.5 1 | 734.70 | 891.64 | 1330.9 0 | 0.22 |
| A_25_P00010 610 | hsa-miR-93 | 13318.34 | 11448.21 | 9926.59 | 10978.79 | 1953.2 7 | 1661.1 1 | 1780.9 7 | 1567.6 7 | 0.15 |
| A_25_P00010 611 | hsa-miR-93 | 2511.98 | 2029.10 | 2579.95 | 2567.14 | - | - | - | - | |
| A_25_P00013 074 | hsa-miR-936 | 2791.84 | 2185.32 | 2171.70 | 2269.72 | - | - | - | - | |
| A_25_P00013 086 | hsa-miR-939 | 13696.81 | 11724.10 | 7565.71 | 6900.10 | 1465.7 5 | 1455.7 1 | - | - | 0.18 |
| A_25_P00013 087 | hsa-miR-939 | 23825.81 | 20669.21 | 15093.11 | 14966.81 | 3074.0 4 | 2449.5 6 | 2155.4 2 | 1945.6 9 | 0.13 |
| A_25_P00013 089 | hsa-miR-940 | 1621.59 | 1419.63 | 1825.11 | 1900.92 | - | 22421. 07 | - | - | |
| A_25_P00013 090 | hsa-miR-940 | 2660.57 | 2327.78 | 3077.13 | 2903.48 | 1443.9 5 | 1480.4 6 | - | 1398.3 3 | 0.55 |
| A_25_P00012 035 | hsa-miR-96 | 1458.34 | 1067.99 | 1318.79 | 986.69 | - | - | - | - | |

Table 6. List of 13 remodelling-associated proteins (=seed proteins=remodelling proteins) and their 26 interacting proteins (=interactor).

| Seed | Interactor |
|---|---|
| ADRB1 | MAGI2 |
| CRP | FCGR2A |
| CRP | FCGR2C |
| HGF | HGFAC |
| HGF | MET |
| MDK | RPL18A |
| MDK | UBQLN4 |
| MMP2 | HSP90AA1 |
| MMP2 | LAMC2 |
| MMP2 | TIMP2 |
| MMP9 | ITGA5 |
| MMP9 | LCN2 |
| NOS3 | ESR1 |
| NPPA | UBQLN4 |
| NPPB | EWSR1 |
| STUB1 | AKT1 |
| STUB1 | HSPA8 |
| STUB1 | KHDRBS1 |
| STUB1 | MAP3K14 |
| STUB1 | MAP3K3 |
| STUB1 | MAPT |
| STUB1 | MPP1 |
| STUB1 | TRAF2 |
| TFAM | IKBKE |
| TFAM | MCC |
| TFAM | TFB2M |
| TFAM | TNIK |

**Table 7.** The 265 miRNAs targeting both seed proteins and their interactors

| Gene | miRNAs | Gene | mRNA | Gene | mRNA | Gene | miRNA |
|---|---|---|---|---|---|---|---|
| ACE | hsa-miR-890 | HSPA8 | hsa-miR-26a | MAP3K14 | hsa-miR-665 | MMP9 | hsa-miR-204 |
| ACE | hsa-miR-138 | HSPA8 | hsa-miR-301a | MAP3K14 | hsa-miR-874 | MMP9 | hsa-miR-296-3p |
| ACE | hsa-miR-199b-5p | HSPA8 | hsa-miR-338-5p | MAP3K3 | hsa-miR-96 | MMP9 | hsa-miR-330-3p |
| ACE | hsa-miR-22 | HSPA8 | hsa-miR-33a | MAP3K3 | hsa-let-7b | MMP9 | hsa-miR-483-3p |
| ACE | hsa-miR-24 | HSPA8 | hsa-miR-340 | MAP3K3 | hsa-miR-103 | MMP9 | hsa-miR-491-5p |
| ACE | hsa-miR-27a | HSPA8 | hsa-miR-365 | MAP3K3 | hsa-miR-133a | MPP1 | hsa-miR-892b |
| ACE | hsa-miR-323-5p | HSPA8 | hsa-miR-448 | MAP3K3 | hsa-miR-141 | MPP1 | hsa-miR-105 |
| ACE | hsa-miR-432 | HSPA8 | hsa-miR-499-5p | MAP3K3 | hsa-miR-145 | MPP1 | hsa-miR-137 |

(continued)

| Gene | miRNAs | Gene | mRNA | Gene | mRNA | Gene | miRNA |
|---|---|---|---|---|---|---|---|
| ACE | hsa-miR-551a | HSPA8 | hsa-miR-519a | MAP3K3 | hsa-miR-181a | MPP1 | hsa-miR-296-5p |
| ACE | hsa-miR-593 | HSPA8 | hsa-miR-519d | MAP3K3 | hsa-miR-182 | MPP1 | hsa-miR-363 |
| ACE | hsa-miR-635 | HSPA8 | hsa-miR-580 | MAP3K3 | hsa-miR-193b | MPP1 | hsa-miR-423-5p |
| ACE | hsa-miR-876-3p | HSPA8 | bsa-miR-587 | MAP3K3 | hsa-miR-194 | MPP1 | hsa-miR-500 |
| ADRB1 | hsa-miR-937 | HSPA8 | hsa-miR-590-3p | MAP3K3 | hsa-miR-204 | MPP1 | hsa-miR-501-5p |
| ADRB1 | hsa-miR-101 | HSPA8 | hsa-miR-641 | MAP3K3 | hsa-miR-206 | MPP1 | hsa-miR-515-5p |
| ADRB1 | hsa-miR-141 | IKBKE | hsa-miR-769-3p | MAP3K3 | hsa-miR-22 | MPP1 | hsa-miR-519d-5p |
| ADRB1 | hsa-miR-142-3p | IKBKE | bsa-let-7b | MAP3K3 | hsa-miR-23a | MPP1 | hsa-miR-576-3p |
| ADRB1 | hsa-miR-150 | IKBKE | hsa-let-7c | MAP3K3 | hsa-miR-891b | MPP1 | hsa-miR-582-5p |
| ADRB1 | hsa-miR-188-5p | IKBKE | hsa-miR-155 | MAP3K3 | hsa-miR-9 | MPP1 | hsa-miR-592 |
| ADRB1 | hsa-miR-30a | IKBKE | hsa-miR-192 | MAP3K3 | hsa-miR-93 | MPP1 | hsa-miR-607 |
| ADRB1 | hsa-miR-30b | IKBKE | hsa-miR-24 | MAPT | hsa-miR-563 | MPP1 | hsa-miR-886-3p |
| ADRB1 | hsa-miR-30c | IKBKE | hsa-miR-455-5p | MAPT | hsa-miR-34a | NOS3 | hsa-miR-986-3p |
| ADRB1 | hsa-miR-30d | IKBKE | hsa-miR-485-5p | MCC | hsa-miR-628-5p | NOS3 | hsa-miR-155 |
| ADRB1 | hsa-miR-331-3p | IKBKE | hsa-miR-492 | MCC | hsa-miR-136 | NOS3 | hsa-miR-220b |
| ADRB1 | hsa-miR-526b | IKBKE | hsa-miR-576-5p | MCC | hsa-miR-215 | NOS3 | hsa-miR-31 |
| ADRB1 | hsa-miR-578 | IKBKE | hsa-miR-604 | MCC | hsa-miR-24 | NOS3 | hsa-miR-362-Sp |
| ADRB1 | hsa-miR-671-5p | IKBKE | hsa-miR-7 | MCC | hsa-miR-296-3p | NOS3 | hsa-miR-492 |
| ADRB1 | hsa-miR-891a | ITGA5 | hsa-miR-936 | MCC | hsa-miR-371-3p | NOS3 | hsa-miR-500 |
| AKT1 | hsa-miR-885-3p | ITGA5 | hsa-miR-148a | MCC | hsa-miR-450b-3p | NOS3 | hsa-miR-502-5p |
| AKT1 | hsa-miR-138 | ITGA5 | hsa-miR-149 | MCC | hsa-miR-450b-5p | NOS3 | hsa-miR-506 |
| AKT1 | hsa-miR-409-3p | ITGA5 | hsa-miR-25 | MCC | hsa-miR-501-3p | NOS3 | hsa-miR-524-3p |
| AKT1 | hsa-miR-501-3p | ITGA5 | hsa-miR-26a | MCC | hsa-miR-600 | NOS3 | hsa-miR-543 |
| AKT1 | hsa-miR-655 | ITGA5 | hsa-miR-27a | MCC | hsa-miR-605 | NOS3 | hsa-miR-576-3p |
| CRP | hsa-miR-939 | ITGA5 | hsa-miR-296-3p | MCC | hsa-miR-625 | NOS3 | hsa-miR-744 |
| CRP | hsa-miR-10a | ITGA5 | hsa-miR-30b | MDK | hsa-miR-940 | NPPA | hsa-miR-922 |

(continued)

| Gene | miRNAs | Gene | mRNA | Gene | mRNA | Gene | miRNA |
|---|---|---|---|---|---|---|---|
| CRP | hsa-miR-133a | ITGA5 | hsa-miR-32 | MDK | hsa-miR-124 | NPPA | hsa-miR-105 |
| CRP | hsa-miR-146b-3p | ITGA5 | hsa-miR-328 | MDK | hsa-miR-188-5p | NPPA | hsa-miR-125a-3p |
| CRP | hsa-miR-150 | ITGA5 | hsa-miR-338-3p | MDK | hsa-miR-219-2-3p | NPPA | hsa-miR-139-5p |
| CRP | hsa-miR-186 | ITGA5 | hsa-miR-367 | MDK | hsa-miR-223 | NPPA | hsa-miR-194 |
| CRP | hsa-miR-27a | ITGA5 | hsa-miR-382 | MDK | hsa-miR-23a | NPPA | hsa-miR-224 |
| CRP | hsa-miR-299-3p | ITGA5 | hsa-miR-384 | MDK | hsa-miR-326 | NPPA | hsa-miR-425 |
| CRP | hsa-miR-323-5p | ITGA5 | hsa-miR-432 | MDK | hsa-miR-491-5p | NPPA | hsa-miR-552 |
| CRP | hsa-miR-362-5p | ITGA5 | bsa-miR-486-3p | MDK | hsa-miR-608 | NPPA | hsa-miR-576-3p |
| CRP | hsa-miR-424 | ITGA5 | hsa-miR-515-5p | MDK | hsa-miR-623 | NPPA | hsa-miR-582-5p |
| CRP | hsa-miR-500 | ITGA5 | hsa-miR-760 | MDK | hsa-miR-625 | NPPA | hsa-miR-607 |
| CRP | hsa-miR-542-5p | ITGA5 | hsa-miR-876-5p | MDK | hsa-miR-760 | NPPA | hsa-miR-622 |
| CRP | hsa-miR-609 | KHDRBS1 | hsa-miR-662 | MET | hsa-miR-876-3p | NPPA | hsa-miR-802 |
| CRP | hsa-miR-624 | KHDRBS1 | hsa-miR-200b | MET | hsa-miR-1 | NPPB | hsa-miR-632 |
| CRP | hsa-miR-631 | KHDRBS1 | hsa-miR-203 | MET | hsa-miR-101 | NPPB | hsa-miR-21 |
| CRP | hsa-miR-661 | KHDRBS1 | hsa-miR-204 | MET | hsa-miR-122 | NPPB | hsa-miR-218 |
| CRP | hsa-miR-7 | KHDRBS1 | hsa-miR-218 | MET | hsa-miR-130a | NPPB | hsa-miR-220c |
| CRP | hsa-miR-802 | KHDRBS1 | hsa-miR-302c* | MET | hsa-miR-133a | NPPB | hsa-miR-296-3p |
| CRP | hsa-miR-920 | LAMC2 | hsa-miR-767-5p | MET | hsa-miR-144 | NPPB | hsa-miR-374a |
| ESR1 | hsa-miR-934 | LAMC2 | hsa-miR-1 | MET | hsa-miR-182 | NPPB | hsa-miR-409-3p |
| ESR1 | hsa-miR-148a | LAMC2 | hsa-miR-124 | MET | hsa-miR-186 | NPPB | hsa-miR-617 |
| ESR1 | hsa-miR-18a | LAMC2 | hsa-miR-193b | MET | hsa-miR-198 | STUB1 | hsa-miR-922 |
| ESR1 | hsa-miR-19a | LAMC2 | hsa-miR-23a | MET | hsa-miR-23a | STUB1 | hsa-miR-198 |
| ESR1 | hsa-miR-204 | LAMC2 | hsa-miR-323-3p | MET | hsa-miR-31 | STUB1 | hsa-miR-212 |
| ESR1 | hsa-miR-22 | LAMC2 | hsa-miR-548b-3p | MET | hsa-miR-335 | STUB1 | hsa-miR-324-3p |
| ESR1 | hsa-miR-222 | LAMC2 | hsa-miR-615-3p | MET | hsa-miR-337-3p | STUB1 | hsa-miR-329 |
| ESR1 | hsa-miR-26a | LAMC2 | hsa-miR-660 | MET | hsa-miR-338-5p | STUB1 | hsa-miR-331-3p |

(continued)

| Gene | miRNAs | Gene | mRNA | Gene | mRNA | Gene | miRNA |
|---|---|---|---|---|---|---|---|
| ESR1 | hsa-miR-324-3p | LCN2 | hsa-miR-939 | MET | hsa-miR-34a | STUB1 | hsa-miR-455-3p |
| ESR1 | hsa-miR-33a | LCN2 | hsa-miR-138 | MET | hsa-miR-34b | STUB1 | hsa-miR-545 |
| ESR1 | hsa-miR-34b | LCN2 | hsa-miR-491-5p | MET | hsa-miR-34c-5p | STUB1 | hsa-miR-608 |
| ESR1 | hsa-miR-650 | LCN2 | hsa-miR-608 | MET | hsa-miR-369-3p | STUB1 | hsa-miR-625 |
| ESR1 | hsa-miR-9 | LCN2 | hsa-miR-646 | MET | hsa-miR-374b | STUB1 | hsa-miR-634 |
| EWSR1 | hsa-miR-768-3p | LCN2 | hsa-miR-675 | MET | hsa-miR-381 | STUB1 | hsa-miR-873 |
| EWSR1 | hsa-miR-299-5p | LCN2 | hsa-miR-923 | MET | hsa-miR-509-3p | TFAM | hsa-miR-769-5p |
| EWSR1 | hsa-miR-409-3p | MAGI2 | hsa-miR-887 | MET | hsa-miR-520g | TFAM | hsa-miR-299-5p |
| EWSR1 | hsa-miR-522 | MAGI2 | hsa-miR-1 | MET | hsa-miR-548d-3p | TFAM | hsa-miR-455-3p |
| EWSR1 | hsa-miR-582-5p | MAGI2 | hsa-miR-101 | MET | hsa-miR-595 | TFAM | hsa-miR-556-5p |
| EWSR1 | hsa-miR-593 | MAGI2 | hsa-miR-134 | MET | hsa-miR-616 | TFAM | hsa-miR-561 |
| EWSR1 | hsa-miR-659 | MAGI2 | hsa-miR-141 | MET | hsa-miR-633 | TFAM | hsa-miR-582-3p |
| FCGR2A | bsa-miR-643 | MAGI2 | hsa-miR-142-5p | MMP2 | hsa-miR-644 | TFAM | hsa-miR-590-3p |
| FCGR2A | hsa-miR-297 | MAGI2 | hsa-miR-144 | MMP2 | hsa-miR-136 | TFB2M | hsa-miR-935 |
| FCGR2A | hsa-miR-331-5p | MAGI2 | hsa-miR-19a | MMP2 | hsa-miR-299-3p | TFB2M | hsa-miR-101 |
| FCGR2A | hsa-miR-337-5p | MAGI2 | hsa-miR-218 | MMP2 | hsa-miR-29a | TFB2M | hsa-miR-144 |
| FCGR2A | hsa-miR-512-5p | MAGI2 | hsa-miR-22 | MMP2 | hsa-miR-486-3p | TFB2M | hsa-miR-19a |
| FCGR2A | hsa-miR-581 | MAGI2 | hsa-miR-221 | MMP2 | hsa-miR-519e | TFB2M | hsa-miR-452 |
| FCGR2A | hsa-miR-640 | MAGI2 | hsa-miR-27a | MMP2 | hsa-miR-564 | TFB2M | hsa-miR-488 |
| FCGR2C | hsa-miR-331-5p | MAGI2 | hsa-miR-28-3p | MMP3 | hsa-miR-874 | TFB2M | hsa-miR-495 |
| HGF | hsa-miR-522 | MAGI2 | hsa-miR-542-3p | MMP3 | hsa-miR-146b-3p | TFB2M | hsa-miR-539 |
| HGF | hsa-let-7d | MAGI2 | hsa-miR-556-5p | MMP3 | hsa-miR-15b | TFB2M | hsa-miR-548c-3p |
| HGF | hsa-miR-190 | MAGI2 | hsa-miR-587 | MMP3 | hsa-miR-17 | TIMP2 | hsa-miR-891a |
| HGFAC | hsa-miR-940 | MAGI2 | hsa-miR-610 | MMP3 | hsa-miR-18a | TIMP2 | hsa-miR-130a |
| HSP90AA1 | hsa-miR-888 | MAGI2 | hsa-miR-629 | MMP3 | hsa-miR-204 | TIMP2 | hsa-miR-483-5p |
| HSP90AA1 | hsa-miR-146b-3p | MAGI2 | hsa-miR-651 | MMP3 | hsa-miR-27a | TRAF2 | hsa-miR-767-3p |

(continued)

| Gene | miRNAs | Gene | mRNA | Gene | mRNA | Gene | miRNA |
|---|---|---|---|---|---|---|---|
| HSP90AA1 | hsa-miR-148a | MAP3K14 | hsa-miR-892a | MMP3 | hsa-miR-31 | TRAF2 | hsa-miR-150 |
| HSP90AA1 | hsa-miR-196a | MAP3K14 | hsa-miR-137 | MMP3 | hsa-miR-365 | TRAF2 | hsa-miR-188-3p |
| HSP90AA1 | hsa-miR-219-2-3p | MAP3K14 | hsa-miR-155 | MMP3 | hsa-miR-516a-3p | TRAF2 | hsa-miR-221 |
| HSP90AA1 | hsa-miR-362-5p | MAP3K14 | hsa-miR-19a | MMP3 | hsa-miR-520f | TRAF2 | hsa-miR-342-3p |
| HSP90AA1 | hsa-miR-411 | MAP3K14 | hsa-miR-27a | MMP3 | hsa-miR-542-3p | TRAF2 | hsa-miR-504 |
| HSP90AA1 | hsa-miR-518d-5p | MAP3K14 | hsa-miR-31 | MMP3 | hsa-miR-574-3p | TRAF2 | hsa-miR-532-3p |
| HSP90AA1 | hsa-miR-550 | MAP3K14 | hsa-miR-372 | MMP3 | hsa-miR-574-5p | TRAF2 | hsa-miR-589 |
| HSP90AA1 | hsa-miR-591 | MAP3K14 | hsa-miR-412 | MMP3 | hsa-miR-577 | TRAF2 | hsa-miR-601 |
| HSPA8 | hsa-miR-646 | MAP3K14 | hsa-miR-492 | MMP3 | hsa-miR-623 | TRAF2 | hsa-miR-647 |
| HSPA8 | hsa-miR-142-5p | MAP3K14 | hsa-miR-514 | MMP3 | hsa-miR-624 | UBQLN4 | hsa-miR-516b |
| HSPA8 | hsa-miR-147 | MAP3K14 | hsa-miR-517a | MMP9 | hsa-miR-892b | UBQLN4 | hsa-miR-342-3p |
| HSPA8 | hsa-miR-222 | MAP3K14 | hsa-miR-621 | MMP9 | hsa-miR-133a | | |
| HSPA8 | hsa-miR-26a | MAP3K14 | hsa-miR-630 | MMP9 | hsa-miR-149 | | |
| HSPA8 | hsa-miR-301a | MAP3K14 | hsa-miR-634 | MMP9 | hsa-miR-183 | | |

References

1. Mitchell PS, Parkin RK, Kroh EM, Fritz BR, Wyman SK, Pogosova-Agadjanyan EL, et al. Circulating microRNAs as stable blood-based markers for cancer detection. Proc Natl Acad Sci U S A. 2008;105:10513-10518.

2. Lopez-Romero P, Gonzalez MA, Callejas S, Dopazo A, Irizarry RA. Processing of Agilent microRNA array data. BMC Res Notes. 2010;3:18.

3. Friedman RC, Farh KK, Burge CB, Bartel DP. Most mammalian mRNAs are conserved targets of microRNAs. Genome Res. 2009;19:92-105.

4. Krek A, Grun D, Poy MN, Wolf R, Rosenberg L, Epstein EJ, et al. Combinatorial microRNA target predictions. Nat Genet. 2005;37:495-500.

5. Griffiths-Jones S, Grocock RJ, van Dongen S, Bateman A, Enright AJ. miRBase: microRNA sequences, targets and gene nomenclature. Nucleic Acids Res. 2006;34:D140-144.

6. Huang da W, Sherman BT, Tan Q, Collins JR, Alvord WG, Roayaei J, et al. The DAVID Gene Functional Classification Tool: a novel biological module-centric algorithm to functionally analyze large gene lists. Genome Biol. 2007;8:R183.

7. Aranda B, Achuthan P, Alam-Faruque Y, Armean I, Bridge A, Derow C, et al. The IntAct molecular interaction database in 2010. Nucleic Acids Res. 2010;38:D525-531.

8. Salwinski L, Miller CS, Smith AJ, Pettit FK, Bowie JU, Eisenberg D. The Database of Interacting Proteins: 2004 update. Nucleic Acids Res. 2004;32:D449-451.

9. Ceol A, Chatr Aryamontri A, Licata L, Peluso D, Briganti L, Perfetto L, et al. MINT, the molecular interaction database: 2009 update. Nucleic Acids Res. 2010;38:D532-539.

10. Cline MS, Smoot M, Cerami E, Kuchinsky A, Landys N, Workman C, et al. Integration of biological networks and gene expression data using Cytoscape. Nat Protoc. 2007;2:2366-2382.

11. Goncalves JP, Graos M, Valente AX. POLAR MAPPER: a computational tool for integrated visualisation of protein interaction networks and mRNA expression data. J R Soc Interface. 2009;6:881-896.

## Claims

1. A kit for monitoring the prognosis of a patient having suffered from acute myocardial ischemia consisting of (a) probes for measuring a panel of miRNA biomarkers in a sample of bodily fluid of said patient, said panel of miRNA biomarkers consisting of:

   (a) miR-16 encoded by SEQ ID NO:1,
   (b) miR-27a encoded by SEQ ID NO:2,
   (c) miR-101 encoded by SEQ ID NO:3, and
   (d) miR-150 encoded by SEQ ID NO:4,

   and optionally (b) detection reagents for measuring Nt-pro-BNP as shown in SEQ ID NO:5 in a sample of bodily fluid of said patient.

2. The kit of claim 1, wherein said probes for measuring said panel of miRNAs are suitable for detection by quantitative RT-PCR and said detection reagents for measuring said Nt-proBNP level are suitable for detection by immunoassay.

3. The use of a kit according to claim 1 or 2 for monitoring the prognosis of a patient having suffered from acute myocardial ischemia.

4. A method for predicting and/or monitoring the prognosis of left ventricular remodeling in a patient, wherein the patient has suffered from an acute myocardial infarction, comprising:

   i) determining the levels of miR-16, miR-27a, miR-101 and miR-150 in a sample of bodily fluid from said patient, and
ii) correlating the levels of said miRNAs with:

a) levels observed in a population of control patients who have either not suffered from an AMI or have suffered from an AMI and have preserved left ventricular contractility, wherein a statistically significant increase in levels of miR-16 and mi-R-27a and a statistically significant decrease in levels of miR-150 and miR-101 by comparison with the control is indicative of impaired left ventricular contractility, or progress towards impaired left ventricular contractility and optionally also determining an increase in levels of Nt-pro-BNP by comparison with the control, or
b) with a previously established classification model wherein said model was developed by fitting data from a study of a population of patients and said fitted data comprises levels of said biomarkers and conversion to the development of left ventricular remodeling in said selected population of patients and optionally correlating the levels of Nt-pro-BNP with the classification model and

iii) obtaining a prognostic score for being at risk of developing left ventricular remodeling by establishing the odds ratios of only said miRNAs, optionally in combination with levels of Nt-pro-BNP.

**5.** A method according to claims 4 , wherein said patient has a WMIS score between 1 and 1.4.

**6.** A method for assessing the efficacy of a treatment for a patient having suffered from an acute myocardial infarction and having a likelihood of developing a reduced left ventricular contractility, wherein the method comprises i) determining the levels of miR-16, miR-27a, miR-101 and miR-150 in a sample of bodily fluid from said patient, ii) determining the Nt-pro-BNP level in a sample of bodily fluid from said patient, iii) determining the levels of miR-16, miR-27a, miR-101 and miR-150 and the level of Nt-pro-BNP in a sample of bodily fluid from said patient after treatment, iv) comparing only the results of i) and ii) with the results of iii), wherein a difference between the results of i), ii) and iii) indicates an effect of the treatment.

**7.** A method according to claim 6, wherein said patient has a WMIS score between 1 and 1.4.

**8.** A method according to any one of claims 4 to 7, wherein said body fluid is blood, serum, plasma, cerebrospinal fluid, saliva or urine, preferably blood, plasma or serum.

**9.** A method of establishing a classification model comprising

i) establishing the levels of a biomarker panel consisting of miR-16, miR-27a, miR-101 and miR-150 in a sample of bodily fluid from a population of MI patients, and optionally establishing the level of Nt-pro-BNP and
ii) conversion of the levels of this biomarker panel and optionally the level of Nt-pro-BNP to the development of left ventricular remodeling in said selected population of patients,

wherein a prognostic for being at risk of developing left ventricular remodeling is established by establishing the odds ratios of said biomarker panel of miRNAs and optionally of Nt-pro-BNP.

**10.** A method according to claim 9, wherein the probability P of developing left ventricular remodeling is calculated by:

$$P = \exp(X) / (1+\exp(X))$$

wherein X= miR150 x ln 0.08 + miR101 x ln 0.19 + miR27a x ln 15.9 + miR16 x ln 4.18 + Nt-pro-BNP x ln 3.97 + territory x ln 2.29 + STEM/NSTEMI x In 1.68 + Prior MI x ln 8.87 + Hypercholesterolemia x ln 1.63 + Hypertension x In 1.00 + Diabetes x ln 0.70 + Smoking habit x In 1.49 + Gender x ln 1.29 + Age x ln 1.00 + ln 8.51x10E-5 and wherein if P > 0.5 then there is a significant risk of remodeling (WMIS >1.2) and wherein if P <= 0.5 then there is a low or null risk of remodeling (WMIS <=1.2).

## Patentansprüche

**1.** Kit zum Überwachen der Prognose eines Patienten, der an akuter Myokardischämie gelitten hat, bestehend aus (a) Sonden zum Messen einer Gruppe von miRNA-Biomarkern in einer Körperflüssigkeitsprobe des Patienten, wobei die Gruppe der miRNA-Biomarker besteht aus:

(a) miR-16, das durch SEQ ID NO: 1 codiert wird,
(b) miR-27a, das durch SEQ ID NO: 2 codiert wird,
(c) miR-101, das durch SEQ ID NO: 3 codiert wird, und
(d) miR-150, das durch SEQ ID NO: 4 codiert wird,

und gegebenenfalls (b) Nachweisreagenzien zum Messen von Nt-pro-BNP wie in SEQ ID NO: 5 gezeigt, in einer Körperflüssigkeitsprobe des Patienten.

**2.** Kit nach Anspruch 1, wobei die Sonden zum Messen der Gruppe von miRNAs zum Nachweis durch quantitative RT-PCR geeignet sind
und die Nachweisreagenzien zum Messen des Nt-proBNP-Spiegels für den Nachweis durch Immunassay geeignet sind.

**3.** Verwendung eines Kits nach Anspruch 1 oder 2 zum Überwachen der Prognose eines Patienten, der an akuter Myokardischämie gelitten hat.

**4.** Verfahren zur Vorhersage und/oder Überwachung der Prognose des linksventrikulären Remodeling bei einem Patienten, wobei der Patient an einem akuten Myokardinfarkt gelitten hat, umfassend

i) Bestimmen der Spiegel von miR-16, miR-27a, miR-101 und miR-150 in einer Körperflüssigkeitsprobe aus dem Patienten und
ii) Korrelieren der miRNA-Spiegel mit:

a) Spiegeln, die in einer Population von Kontrollpatienten beobachtet werden, die entweder nicht an einer AMI gelitten haben oder die an einer AMI gelitten haben und die linksventrikuläre Kontraktilität bewahrt haben, wobei ein statistisch signifikanter Anstieg der miR-16- und mi-R27a-Spiegel und eine statistisch signifikante Abnahme der miR-150- und miR-101-Spiegel im Vergleich zur Kontrolle eine beeinträchtigte linksventrikuläre Kontraktilität oder einen Verlauf zu beeinträchtigter linksventrikulärer Kontraktilität anzeigen und gegebenenfalls ebenfalls Bestimmen eines Anstiegs der Nt-pro-BNP-Spiegel durch Vergleich mit der Kontrolle, oder
b) mit einem zuvor eingesetzten Klassifikationsmodell, wobei das Modell entwickelt wurde durch Anpassen von Daten aus einer Studie einer Population von Patienten, und die angepassten Daten Biomarkerspiegel umfassen und Umwandlung zur Entwicklung von linksventrikulärem Remodeling in der ausgewählten Population von Patienten und gegebenenfalls Korrelieren der Nt-pro-BNP-Spiegel mit dem Klassifikationsmodell, und

iii) Gewinnen eines prognostischen Werts, der das Risiko für die Entwicklung eines linksventrikulären Remodeling angibt, indem die Chancen-Verhältnisse von nur miRNAs gegebenenfalls in Kombination mit den Nt-pro-BNP-Spiegeln eingesetzt werden.

**5.** Verfahren nach Anspruch 4, wobei der Patient einen WMIS-Wert zwischen 1 und 1,4 hat.

**6.** Verfahren zum Bewerten der Wirksamkeit einer Behandlung für einen Patienten, der an einem akuten Myokardinfarkt gelitten hat und eine Wahrscheinlichkeit hat, eine reduzierte linksventrikuläre Kontraktilität zu entwickeln, wobei das Verfahren umfasst: i) Bestimmen der Spiegel von miR-16, miR-27a, miR-101 und miR-150 in einer Körperflüssigkeitsprobe des Patienten, ii) Bestimmen des Nt-pro-BNP-Spiegels in einer Körperflüssigkeitsprobe aus dem Patienten, iii) Bestimmen der Spiegel von miR-16, miR-27a, miR-101 und miR-150 und des Nt-pro-BNP-Spiegels in einer Körperflüssigkeitsprobe aus dem Patienten nach der Behandlung, iv) Vergleichen der Ergebnisse nur von i) und ii) mit den Ergebnissen von iii), wobei ein Unterschied zwischen den Ergebnissen von i), ii) und iii) eine Wirkung der Behandlung anzeigt.

**7.** Verfahren nach Anspruch 6, wobei der Patient einen WMIS-Wert zwischen 1 und 1,4 aufweist.

**8.** Verfahren nach einem der Ansprüche 4 bis 7, wobei die Körperflüssigkeit Blut, Serum, Plasma, Cerebrospinalflüssigkeit, Speichel oder Urin, vorzugsweise Blut, Plasma oder Serum ist.

**9.** Verfahren zum Einsetzen eines Klassifikationsmodells, umfassend

i) Einsetzen der Spiegel einer Biomarkergruppe, bestehend aus miR-16, miR-27a, miR-101 und miR-150 in einer Körperflüssigkeitsprobe aus einer Population von MI-Patienten, und gegebenenfalls Einsetzen des Nt-pro-BNP-Spiegels und
ii) Umwandeln der Spiegel dieser Biomarkergruppe und gegebenenfalls des Nt-pro-BNP-Spiegels zur Entwicklung von linksventrikulärem Remodeling in der ausgewählten Patientenpopulation,

wobei eine Prognose für ein Risiko zur Entwicklung von linksventrikulärem Remodeling durch Einsetzen der Chancenverhältnisse der Biomarkergruppe von miRNAs und gegebenenfalls von Nt-pro-BNP erstellt wird.

**10.** Verfahren nach Anspruch 9, wobei die Wahrscheinlichkeit P für die Entwicklung von linksventrikulären Remodeling berechnet wird durch:

$$P = \exp(X)/(1 + \exp(X)),$$

wobei X = miR150 x ln 0,08 + miR101 x ln 0,19 + miR27a x ln 15,9 + miR16 x ln 4,18 + Nt-pro-BNP x ln 3,97 + Bereich x ln 2,29 + STEM/NSTEMI x ln 1,68 + vorheriger MI x ln 8,87 + Hypercholesterinämie x ln 1,63 + Hypertonie x ln 1,00 + Diabetes x ln 0,70 + Rauchgewohnheit x ln 1,49 + Geschlecht x ln 1,29 + Alter x ln 1,00 + ln 8,51x10E-5 und wobei, wenn P > 0,5 ist, dann ein signifikantes Risiko des Remodelings besteht (WMIS> 1,2) und wenn P <= 0,5 ist, dann ein geringes oder gar kein Risiko der Remodeling besteht (WMIS <= 1,2).

## Revendications

**1.** Trousse destinée à suivre le pronostic d’un patient ayant souffert d’une ischémie aiguë du myocarde, constituée par (a) des sondes destinées à mesurer un panel de biomarqueurs à ARNmi dans un échantillon de liquide corporel provenant dudit patient, ledit panel de biomarqueurs à ARNmi étant constitué par :

(a) un miR-16 codé par la SEQ ID n° : 1,
(b) un miR-27a codé par la SEQ ID n° : 2,
(c) un miR-101 codé par la SEQ ID n° : 3 et
(d) un miR-150 codé par la SEQ ID n° : 4,

et, en option, (b) des réactifs de détection destinés à mesurer un Nt-pro-BNP, tel qu’il est présenté dans la SEQ ID n° : 5, dans un échantillon de liquide corporel provenant dudit patient.

**2.** Trousse de la revendication 1, dans laquelle lesdites sondes destinées à mesurer ledit panel d’ARNmi sont appropriées pour une détection par ACP-TI quantitative et lesdits réactifs de détection destinés à mesurer ledit taux de Nt-proBNP sont appropriés pour une détection par immuno-dosage.

**3.** Utilisation d’une trousse, selon les revendications 1 ou 2, pour un suivi du pronostic d’un patient ayant souffert d’une ischémie aigüe du myocarde.

**4.** Procédé destiné à prédire et/ou à suivre le pronostic d’un remodelage du ventricule gauche chez un patient, dans lequel le patient a souffert d’un infarctus aigu du myocarde, comprenant les étapes consistant à :

i) déterminer les taux de miR-16, miR-27a, miR-101 et miR-150 dans un échantillon de liquide corporel provenant dudit patient, et
ii) faire une corrélation des taux desdits ARNmi avec :

a) des taux observés dans une population de patients témoins laquelle soit n’a pas souffert d’une IAM, soit a souffert d’une IAM et a conservé une contractilité du ventricule gauche, dans lequel une augmentation statistiquement significative des taux de miR-16 et miR-27a, ainsi qu’une diminution statistiquement significative des taux de miR-150 et miR-101, en comparaison avec le témoin, est une indication d’une contractilité ventriculaire gauche altérée ou d’une progression vers une contractilité ventriculaire gauche altérée et, en option, déterminer également une augmentation des taux de Nt-pro-BNP par une comparaison avec le témoin, ou

b) avec un modèle de classification préalablement établi, ledit modèle ayant été développé par des données de réglages provenant d'une étude d'une population de patients et lesdites données de réglages comprennent des taux desdits biomarqueurs, ainsi que la conversion au développement d'un remodelage du ventricule gauche dans ladite population choisie de patients et, en option, effectuer une corrélation des taux de Nt-pro-BNP avec le modèle de classification, et

iii) obtenir un score de pronostic pour le fait d'être à risque de développer un remodelage du ventricule gauche, en établissant les rapports de cotes desdits ARNmi seulement, en option en combinaison avec les taux de Nt-pro-BNP.

**5.** Procédé selon la revendication 4, dans lequel ledit patient a un score WMIS compris entre 1 et 1,4.

**6.** Procédé destiné à évaluer l'efficacité d'un traitement pour un patient ayant souffert d'un infarctus aigu du myocarde et ayant une vraisemblance de développer une contractilité réduite du ventricule gauche, le procédé comprenant les étapes consistant à i) déterminer les taux de miR-16, miR-27a, miR-101 et miR-150 dans un échantillon de liquide corporel provenant dudit patient, ii) déterminer le taux de Nt-pro-BNP dans un échantillon de liquide corporel provenant dudit patient, iii) déterminer les taux de miR-16, miR-27a, miR-101 et miR-150 ainsi que le taux de Nt-pro-BNP dans un échantillon de liquide corporel provenant dudit patient après un traitement, iv) comparer seulement les résultats des paragraphes i) et ii) avec les résultats du paragraphe iii), dans lequel une différence entre les résultats des paragraphes i), ii) et iii) indique un effet du traitement.

**7.** Procédé selon la revendication 6, dans lequel ledit patient a un score WMIS compris entre 1 et 1,4.

**8.** Procédé selon l'une quelconque des revendications 4 à 7, dans lequel ledit liquide corporel est du sang, du sérum, du plasma, du liquide céphalorachidien, de la salive ou de l'urine, de préférence du sang, du plasma ou du sérum.

**9.** Procédé d'établissement d'un modèle de classification comprenant les étapes consistant à :

i) établir les taux d'un panel de biomarqueurs, constitué par les miR-16, miR-27a, miR-101 et miR-150, dans un échantillon de liquide corporel provenant d'une population de patients à IM et, en option, établir le taux de Nt-pro-BNP, et

ii) convertir les taux de ce panel de biomarqueurs et, en option, le taux de Nt-pro-BNP au développement d'un remodelage du ventricule gauche dans ladite population choisie de patients,

dans lequel un pronostic d'être à risque de développer un remodelage du ventricule gauche est établi en établissant les rapports de cotes dudit panel de biomarqueurs d'ARNmi et, en option, de Nt-pro-BNP.

**10.** Procédé selon la revendication 9, dans lequel la probabilité P de développer un remodelage du ventricule gauche est calculée par :

$$P = \exp(X)/(1+\exp(X))$$

dans laquelle X= miR150 x ln 0,08 + miR101 x ln 0,19 + miR27a x ln 15,9 + miR16 x ln 4,18 + Nt-pro-BNP x ln 3,97 + territoire x ln 2,29 + STEM/NSTEMI x ln 1,68 + MI préalable x ln 8,87 + Hypercholestérolémie x ln 1,63 + Hypertension x ln 1,00 + Diabète x ln 0,70 + Habitudes tabagiques x ln 1,49 + Genre x ln 1,29 + Âge x ln 1,00 + ln 8,51xl0E-5 et dans laquelle si P > 0,5 alors il y a un risque significatif de remodelage (WMIS >1,2)
et dans laquelle si P <= 0,5 alors il y a un risque faible ou nul de remodelage (WMIS <=1,2).

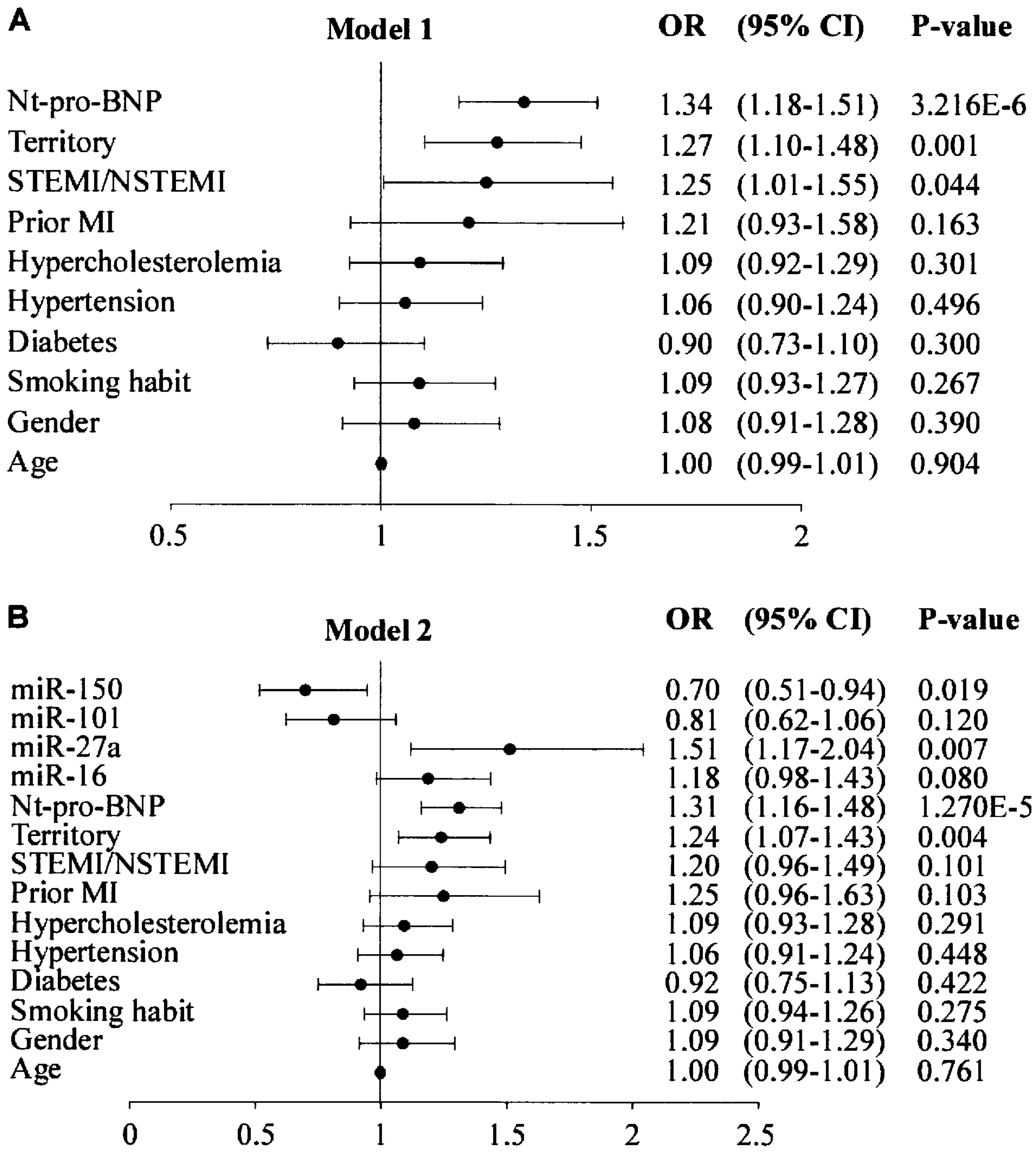
A
Model 1
OR (95% CI) P-value
Nt-pro-BNP 1.34 (1.18-1.51) 3.216E-6
Territory 1.27 (1.10-1.48) 0.001
STEMI/NSTEMI 1.25 (1.01-1.55) 0.044
Prior MI 1.21 (0.93-1.58) 0.163
Hypercholesterolemia 1.09 (0.92-1.29) 0.301
Hypertension 1.06 (0.90-1.24) 0.496
Diabetes 0.90 (0.73-1.10) 0.300
Smoking habit 1.09 (0.93-1.27) 0.267
Gender 1.08 (0.91-1.28) 0.390
Age 1.00 (0.99-1.01) 0.904
0.5
1
1.5
2
B
Model 2
OR (95% CI) P-value
miR-150 0.70 (0.51-0.94) 0.019
miR-101 0.81 (0.62-1.06) 0.120
miR-27a 1.51 (1.17-2.04) 0.007
miR-16 1.18 (0.98-1.43) 0.080
Nt-pro-BNP 1.31 (1.16-1.48) 1.270E-5
Territory 1.24 (1.07-1.43) 0.004
STEMI/NSTEMI 1.20 (0.96-1.49) 0.101
Prior MI 1.25 (0.96-1.63) 0.103
Hypercholesterolemia 1.09 (0.93-1.28) 0.291
Hypertension 1.06 (0.91-1.24) 0.448
Diabetes 0.92 (0.75-1.13) 0.422
Smoking habit 1.09 (0.94-1.26) 0.275
Gender 1.09 (0.91-1.29) 0.340
Age 1.00 (0.99-1.01) 0.761
0
0.5
1
1.5
2
2.5

**Fig. 1**

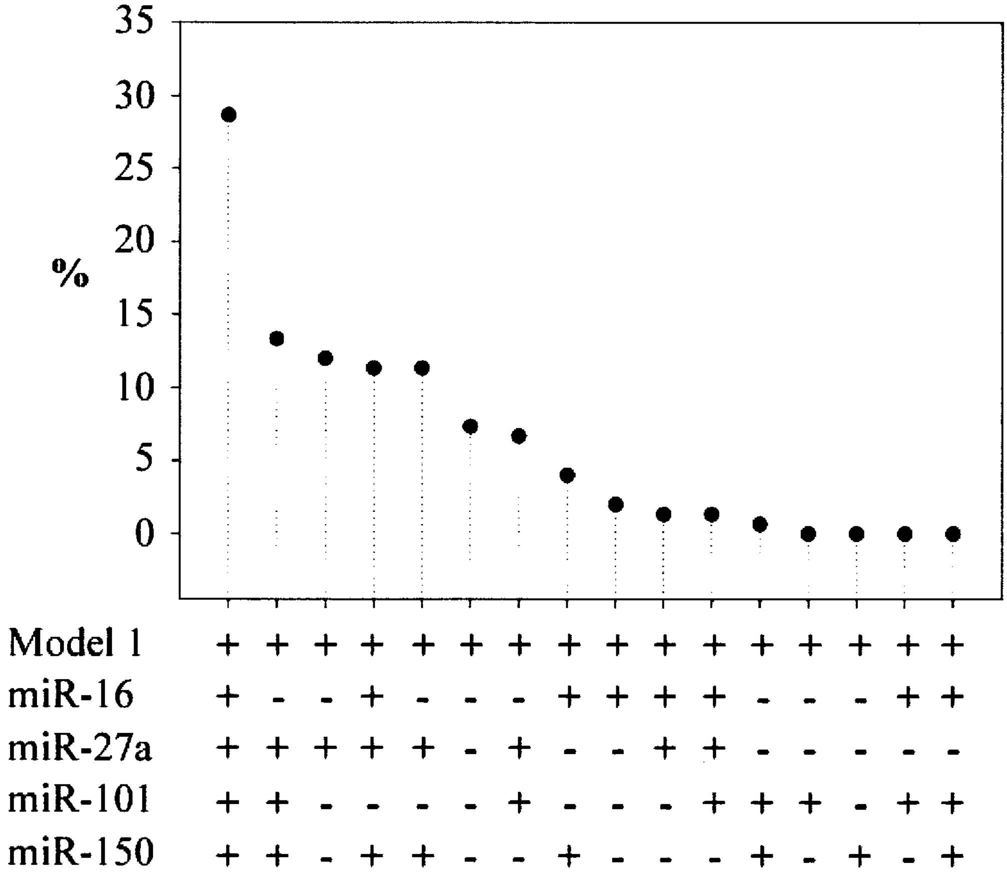


Fig. 2

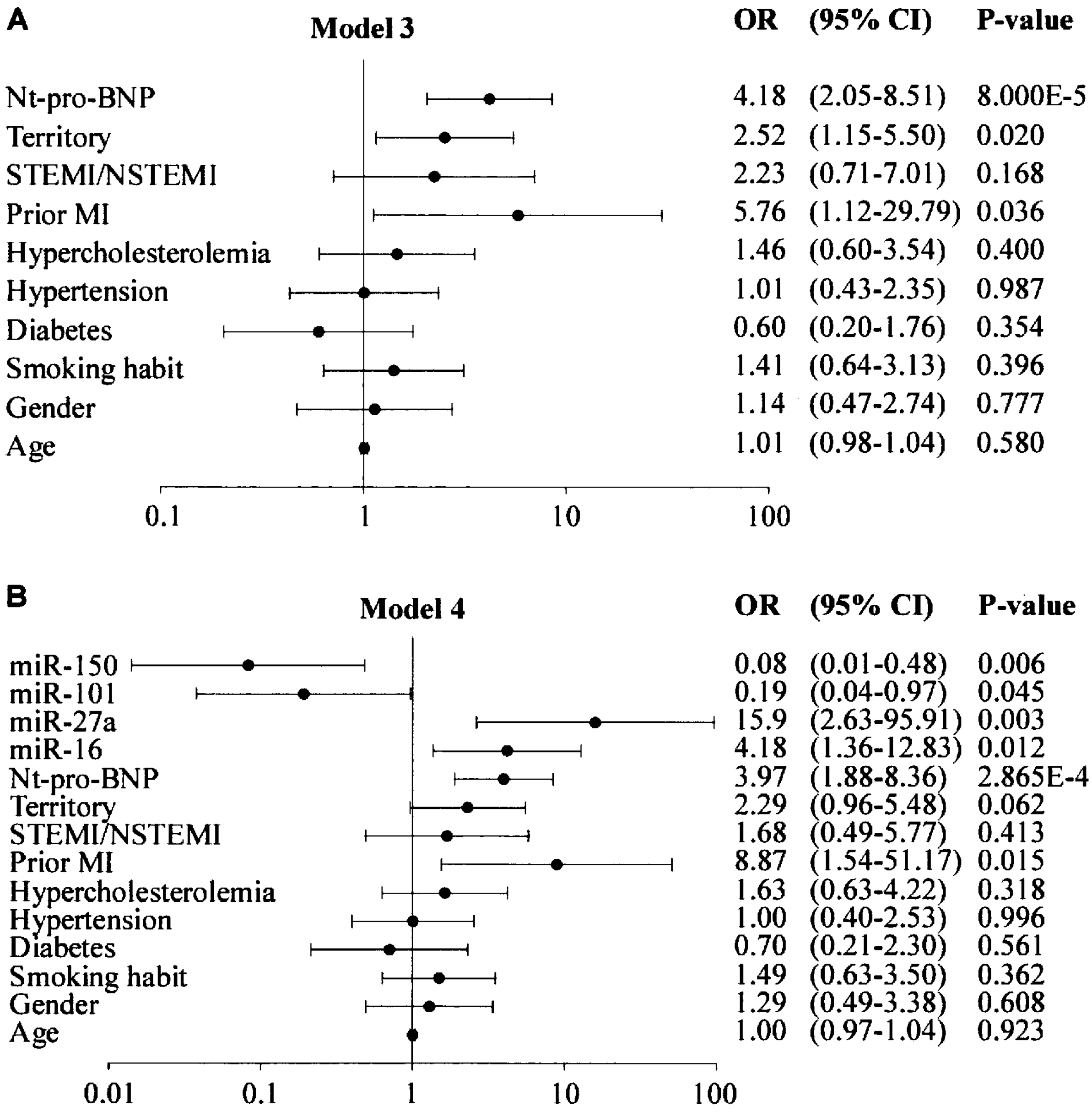
A
Model 3
OR (95% CI) P-value
Nt-pro-BNP 4.18 (2.05-8.51) 8.000E-5
Territory 2.52 (1.15-5.50) 0.020
STEMI/NSTEMI 2.23 (0.71-7.01) 0.168
Prior MI 5.76 (1.12-29.79) 0.036
Hypercholesterolemia 1.46 (0.60-3.54) 0.400
Hypertension 1.01 (0.43-2.35) 0.987
Diabetes 0.60 (0.20-1.76) 0.354
Smoking habit 1.41 (0.64-3.13) 0.396
Gender 1.14 (0.47-2.74) 0.777
Age 1.01 (0.98-1.04) 0.580
0.1
1
10
100
B
Model 4
OR (95% CI) P-value
miR-150 0.08 (0.01-0.48) 0.006
miR-101 0.19 (0.04-0.97) 0.045
miR-27a 15.9 (2.63-95.91) 0.003
miR-16 4.18 (1.36-12.83) 0.012
Nt-pro-BNP 3.97 (1.88-8.36) 2.865E-4
Territory 2.29 (0.96-5.48) 0.062
STEMI/NSTEMI 1.68 (0.49-5.77) 0.413
Prior MI 8.87 (1.54-51.17) 0.015
Hypercholesterolemia 1.63 (0.63-4.22) 0.318
Hypertension 1.00 (0.40-2.53) 0.996
Diabetes 0.70 (0.21-2.30) 0.561
Smoking habit 1.49 (0.63-3.50) 0.362
Gender 1.29 (0.49-3.38) 0.608
Age 1.00 (0.97-1.04) 0.923
0.01
0.1
1
10
100

Fig. 3

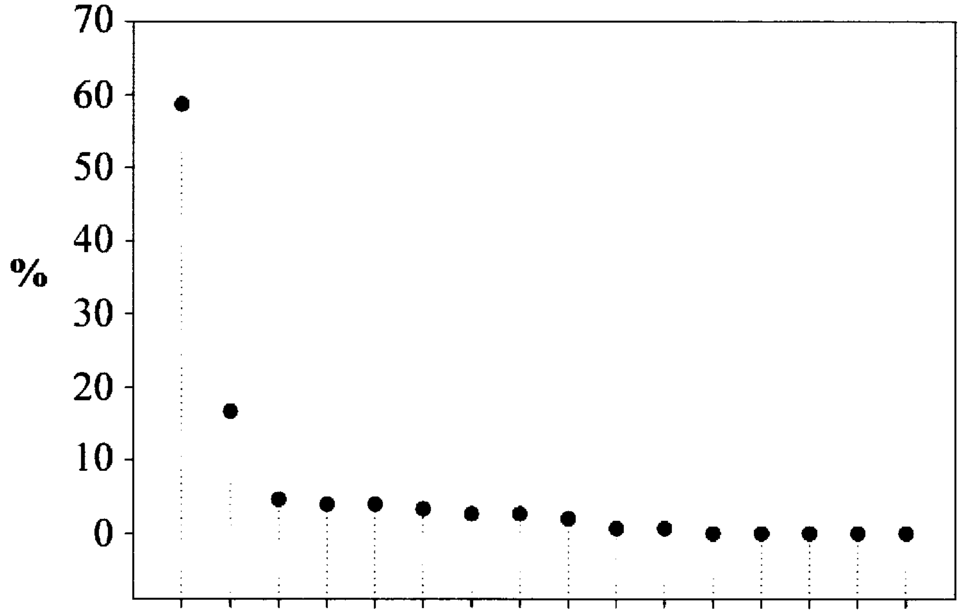


| | | | | | | | | | | | | | | | | |
|---|---|---|---|---|---|---|---|---|---|---|---|---|---|---|---|---|
| Model 3 | + | + | + | + | + | + | + | + | + | + | + | + | + | + | + | + |
| miR-16 | + | + | + | - | - | - | + | - | - | - | + | - | - | + | + | + |
| miR-27a | + | + | - | + | + | - | - | + | + | - | + | - | - | - | - | + |
| miR-101 | + | - | - | - | + | - | - | - | + | + | - | + | - | + | + | + |
| miR-150 | + | + | + | - | - | - | - | + | + | - | - | + | + | - | + | - |

Fig. 4

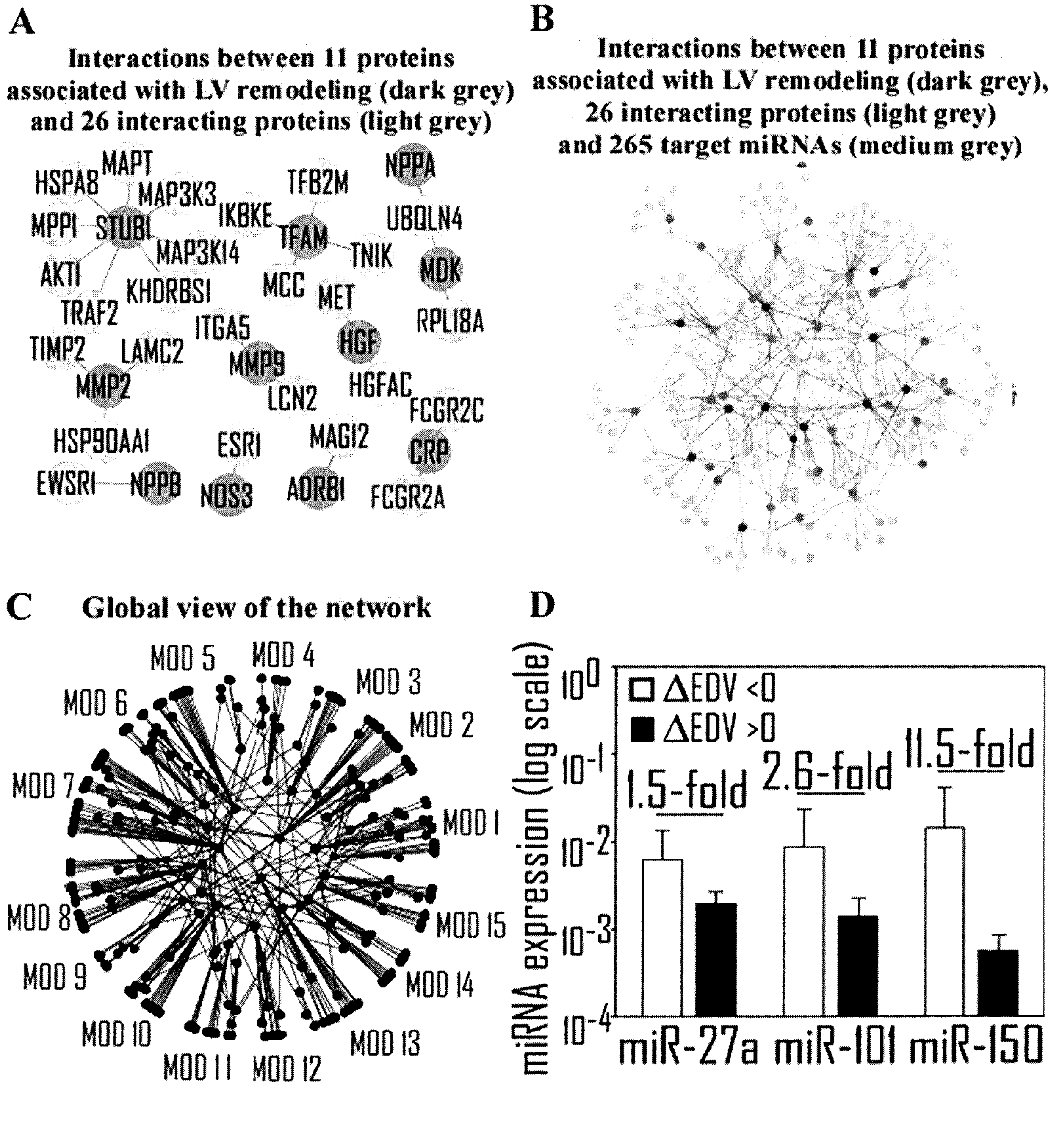
A
Interactions between 11 proteins associated with LV remodeling (dark grey) and 26 interacting proteins (light grey)
HSPA8
MAPT
MAP3K3
TFB2M
NPPA
MPPI
STUBI
IKBKE
TFAM
UBQLN4
MAP3KI4
TNIK
AKTI
MOK
KHDRBSI
MCC
MET
TRAF2
RPLI8A
ITGA5
TIMP2
LAMC2
HGF
MMP9
MMP2
LCN2
HGFAC
FCGR2C
HSP9DAAI
ESRI
MAGI2
CRP
EWSRI
NPPB
NOS3
ADRBI
FCGR2A
B
Interactions between 11 proteins associated with LV remodeling (dark grey), 26 interacting proteins (light grey) and 265 target miRNAs (medium grey)
C
Global view of the network
MOD 1
MOD 2
MOD 3
MOD 4
MOD 5
MOD 6
MOD 7
MOD 8
MOD 9
MOD 10
MOD 11
MOD 12
MOD 13
MOD 14
MOD 15
D
miRNA expression (log scale)
10^0
10^-1
10^-2
10^-3
10^-4
ΔEDV <0
ΔEDV >0
1.5-fold
2.6-fold
11.5-fold
miR-27a
miR-101
miR-150

Fig. 5

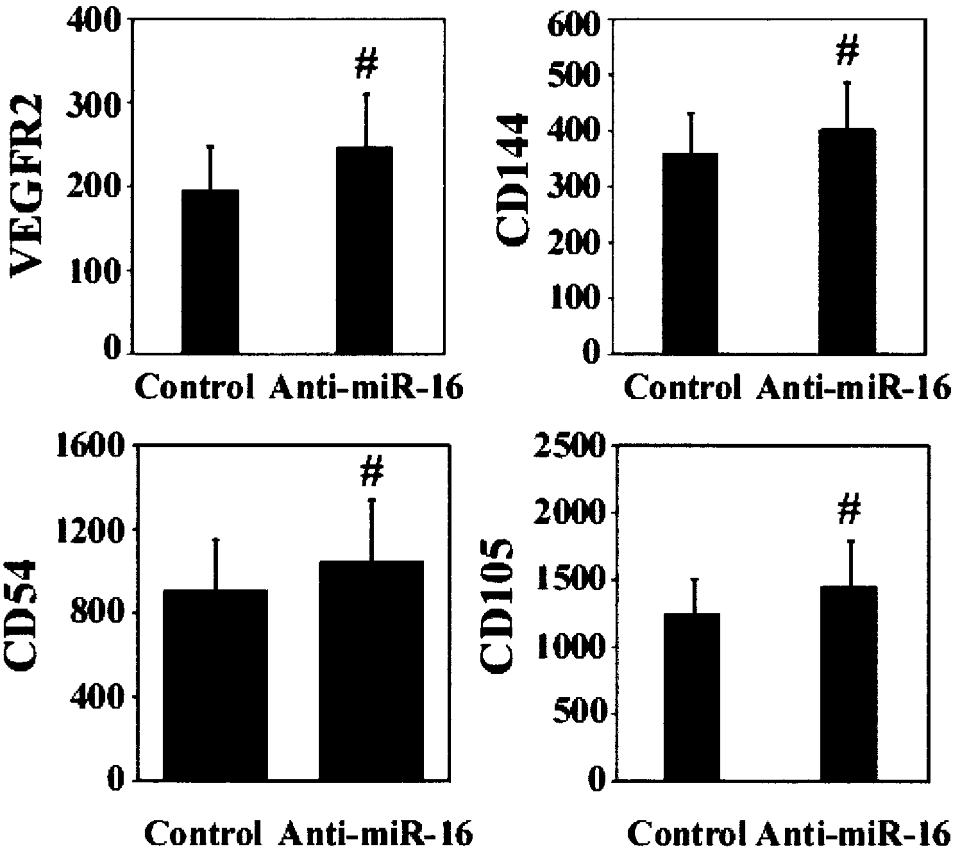
400
300
200
100
0
VEGFR2
#
Control Anti-miR-16
600
500
400
300
200
100
0
CD144
#
Control Anti-miR-16
1600
1200
800
400
0
CD54
#
Control Anti-miR-16
2500
2000
1500
1000
500
0
CD105
#
Control Anti-miR-16

Fig.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008042231 A
- WO 2008043521 A
- US 6143576 A
- US 6113855 A
- US 6019944 A
- US 5631171 A
- US 5955377 A
- US 61730459 B


### Non-patent literature cited in the description


- **TALWAR S et al.** *Eur. Heart J.,* 2000, vol. 21, 1514-1521
- **CREEMERS, ESTHER E. et al.** *Circulation Research,* February 2012, vol. 110 (3), 483-495
- **DI STEFANO, VALERIA et al.** *Vascular Pharmacology,* October 2011, vol. 55 (4), 111-118
- **SAMBROOK et al.** Molecular Cloning: A Laboratory Manual. Cold Spring Harbor Press, 2012
- **AUSUBEL et al.** Current Protocols in Molecular Biology. John Wiley & Sons, 2012
- **IMAI, H. et al.** *J. Virol. Methods,* 1992, vol. 36, 181-184
- **KURTZFELDT, J. et al.** *Nature,* 2005, vol. 438, 685-689
- **EBERT, M.S.** *Nature Methods,* 2007, 12
- Remington's Pharmaceutical Sciences. 2012
- **SONG E et al.** *Nature Biotechnology,* 2005, vol. 23 (6), 709-717
- **TORABI A ; CLELAND JG ; KHAN NK ; LOH PH ; CLARK AL ; ALAMGIR F ; CAPLIN JL ; RIGBY AS ; GOODE K.** The timing of development and subsequent clinical course of heart failure after a myocardial infarction. *Eur Heart J,* 2008, vol. 29, 859-870
- **TALWAR S ; SQUIRE IB ; DOWNIE PF ; MCCULLOUGH AM ; CAMPTON MC ; DAVIES JE ; BARNETT DB ; NG LL.** Profile of plasma N-terminal proBNP following acute myocardial infarction; correlation with left ventricular systolic dysfunction. *Eur Heart J,* 2000, vol. 21, 1514-1521
- **GILAD S ; MEIRI E ; YOGEV Y ; BENJAMIN S ; LEBANONY D ; YERUSHALMI N ; BENJAMIN H ; KUSHNIR M ; CHOLAKH H ; MELAMED N.** Serum microRNAs are promising novel biomarkers. *PLoS One,* 2008, vol. 3, e3148
- **MITCHELL PS ; PARKIN RK ; KROH EM ; FRITZ BR ; WYMAN SK ; POGOSOVA-AGADJANYAN EL ; PETERSON A ; NOTEBOOM J ; O'BRIANT KC ; ALLEN A.** Circulating microRNAs as stable blood-based markers for cancer detection. *Proc Natl Acad Sci U S A,* 2008, vol. 105, 10513-10518
- **CREEMERS EE ; TIJSEN AJ ; PINTO YM.** Circulating MicroRNAs: Novel Biomarkers and Extracellular Communicators in Cardiovascular Disease?. *Circ Res,* 2012, vol. 110, 483-495
- **DEVAUX Y ; VAUSORT M ; GORETTI E ; NAZAROV PV ; AZUAJE F ; GILSON G ; CORSTEN MF ; SCHROEN B ; LAIR ML ; HEYMANS S.** Use of Circulating MicroRNAs to Diagnose Acute Myocardial Infarction. *Clin Chem,* 2012, vol. 58, 559-567
- **WIDERA C ; GUPTA SK ; LORENZEN JM ; BANG C ; BAUERSACHS J ; BETHMANN K ; KEMPF T ; WOLLERT KC ; THUM T.** Diagnostic and prognostic impact of six circulating microRNAs in acute coronary syndrome. *J Mol Cell Cardiol,* 2011, vol. 51, 872-875
- **ZILE MR ; MEHURG SM ; ARROYO JE ; STROUD RE ; DESANTIS SM ; SPINALE FG.** Relationship Between The Temporal Profile of Plasma microRNA and Left Ventricular Remodeling In Patients Following Myocardial Infarction. *Circ Cardiovasc Genet,* 2011, vol. 4, 614-619
- **KELLY D ; COCKERILL G ; NG LL ; THOMPSON M ; KHAN S ; SAMANI NJ ; SQUIRE IB.** Plasma matrix metalloproteinase-9 and left ventricular remodelling after acute myocardial infarction in man: a prospective cohort study. *Eur Heart J,* 2007, vol. 28, 711-718
- **OMLAND T ; PERSSON A ; NG L ; O'BRIEN R ; KARLSSON T ; HERLITZ J ; HARTFORD M ; CAIDAHL K.** N-terminal pro-B-type natriuretic peptide and long-term mortality in acute coronary syndromes. *Circulation,* 2002, vol. 106, 2913-2918
- **TOBIN J.** Estimation of relationships for limited dependent variables. *Econometrica,* 1958, vol. 26, 24-36
- **PENCINA MJ ; D'AGOSTINO RB, SR. ; D'AGOSTINO RB, JR. ; VASAN RS.** Evaluating the added predictive ability of a new marker: from area under the ROC curve to reclassification and beyond. *Stat Med,* 2008, vol. 27, 157-172

- **ZAMPETAKI A ; WILLEIT P ; TILLING L ; DROZDOV I ; PROKOPI M ; RENARD JM ; MAYR A ; WEGER S ; SCHETT G ; SHAH A.** Prospective Study on Circulating MicroRNAs and Risk of Myocardial Infarction. *J Am Coll Cardiol,* 2012, vol. 60, 290-299
- **ZHU H ; FAN GC.** Role of microRNAs in the reperfused myocardium towards post-infarct remodelling. *Cardiovasc Res,* 2012, vol. 94, 284-292
- **ABDELLATIF M.** Differential Expression of MicroRNAs in Different Disease States. *Circ Res,* 2012, vol. 110, 638-650
- **DA COSTA MARTINS PA ; DE WINDT LJ.** MicroRNAs in control of cardiac hypertrophy. *Cardiovasc Res,* 2012, vol. 93, 563-572
- **YAMAMOTO K ; OHKI R ; LEE RT ; IKEDA U ; SHIMADA K.** Peroxisome Proliferator-Activated Receptor y Activators Inhibit Cardiac Hypertrophy in Cardiac Myocytes. *Circulation,* 2001, vol. 104, 1670-1675
- **BELEVYCH AE ; SANSOM SE ; TERENTYEVA R ; HO H-T ; NISHIJIMA Y ; MARTIN MM ; JINDAL HK ; ROCHIRA JA ; KUNITOMO Y ; ABDELLATIF M.** MicroRNA-1 and -133 Increase Arrhythmogenesis in Heart Failure by Dissociating Phosphatase Activity from RyR2 Complex. *PLoS ONE,* 2011, vol. 6, e28324
- **YANG B ; LIN H ; XIAO J ; LU Y ; LUO X ; LI B ; ZHANG Y ; XU C ; BAI Y ; WANG H.** The muscle-specific microRNA miR-1 regulates cardiac arrhythmogenic potential by targeting GJA1 and KCNJ2. *Nat Med,* 2007, vol. 13, 486-491
- **AURORA AB ; MAHMOUD AI ; LUO X ; JOHNSON BA ; VAN ROOIJ E ; MATSUZAKI S ; HUMPHRIES KM ; HILL JA.** Bassel-Duby R, Sadek HA, Olson EN. MicroRNA-214 protects the mouse heart from ischemic injury by controlling Ca2+ overload and cell death. *The Journal of Clinical Investigation,* 2012, vol. 122, 1222-1232
- **NISHI H ; ONO K ; HORIE T ; NAGAO K ; KINOSHITA M ; KUWABARA Y ; WATANABE S ; TAKAYA T ; TAMAKI Y ; TAKANABE-MORI R.** MicroRNA-27a Regulates Beta Cardiac Myosin Heavy Chain Gene Expression by Targeting Thyroid Hormone Receptor β1 in Neonatal Rat Ventricular Myocytes. *Molecular and Cellular Biology,* 2011, vol. 31, 744-755
- **SADEGH MK ; EKMAN M ; RIPPE C ; UVELIUS B ; SWARD K ; ALBINSSON S.** Deletion of Dicer in Smooth Muscle Affects Voiding Pattern and Reduces Detrusor Contractility and Neuroeffector Transmission. *PLoS ONE,* 2012, vol. 7, e35882
- **PAN Z ; SUN X ; SHAN H ; WANG N ; WANG J ; REN J ; FENG S ; XIE L ; LU C ; YUAN Y.** miR-101 Inhibited Post-Infarct Cardiac Fibrosis and Improved Left Ventricular Compliance via FOS/TGFβ1 Pathway. *Circulation,* 2012
- **TIJSEN AJ ; PINTO YM ; CREEMERS EE.** Non-cardiomyocyte microRNAs in heart failure. *Cardiovasc Res,* 2012, vol. 93, 573-582
- **SENN S ; JULIOUS S.** Measurement in clinical trials: a neglected issue for statisticians?. *Stat Med,* 2009, vol. 28, 3189-3209
- **FEDOROV V ; MANNINO F ; ZHANG R.** Consequences of dichotomization. *Pharm Stat,* 2009, vol. 8, 50-61
- **GORETTI et al.** *J. Leukoc. Biol.,* 2013
- **DEVAUX, Y. ; VAUSORT, M ; MCCANN, G. P. ; ZANGRANDO, J. ; KELLY, D. ; RAZVI, N. ; ZHANG, L. ; NG, L.L. ; WAGNER, D.R ; SQUIRE, I.B.** MicroRNA-150. A novel marker of left ventricular remodeling after acute myocardial infarction. *Circ. Cardiovasc Genet.,* 2013, vol. 6, 290-298
- **GORETTI et al.** *J Leukoc Biol,* May 2013, vol. 93 (5), 645-55
- **MITCHELL PS ; PARKIN RK ; KROH EM ; FRITZ BR ; WYMAN SK ; POGOSOVA-AGADJANYAN EL et al.** Circulating microRNAs as stable blood-based markers for cancer detection. *Proc Natl Acad Sci U S A.,* 2008, vol. 105, 10513-10518
- **LOPEZ-ROMERO P ; GONZALEZ MA ; CALLEJAS S ; DOPAZO A ; IRIZARRY RA.** Processing of Agilent microRNA array data. *BMC Res Notes,* 2010, vol. 3, 18
- **FRIEDMAN RC ; FARH KK ; BURGE CB ; BARTEL DP.** Most mammalian mRNAs are conserved targets of microRNAs. *Genome Res.,* 2009, vol. 19, 92-105
- **KREK A ; GRUN D ; POY MN ; WOLF R ; ROSENBERG L ; EPSTEIN EJ et al.** Combinatorial microRNA target predictions. *Nat Genet.,* 2005, vol. 37, 495-500
- **GRIFFITHS-JONES S ; GROCOCK RJ ; VAN DONGEN S ; BATEMAN A ; ENRIGHT AJ.** miRBase: microRNA sequences, targets and gene nomenclature. *Nucleic Acids Res.,* 2006, vol. 34, D140-144
- **HUANG DA W ; SHERMAN BT ; TAN Q ; COLLINS JR ; ALVORD WG ; ROAYAEI J et al.** The DAVID Gene Functional Classification Tool: a novel biological module-centric algorithm to functionally analyze large gene lists. *Genome Biol.,* 2007, vol. 8, R183
- **ARANDA B ; ACHUTHAN P ; ALAM-FARUQUE Y ; ARMEAN I ; BRIDGE A ; DEROW C et al.** The IntAct molecular interaction database in 2010. *Nucleic Acids Res.,* 2010, vol. 38, D525-531
- **SALWINSKI L ; MILLER CS ; SMITH AJ ; PETTIT FK ; BOWIE JU ; EISENBERG D.** The Database of Interacting Proteins: 2004 update. *Nucleic Acids Res.,* 2004, vol. 32, D449-451
- **CEOL A ; CHATR ARYAMONTRI A ; LICATA L ; PELUSO D ; BRIGANTI L ; PERFETTO L et al.** MINT, the molecular interaction database: 2009 update. *Nucleic Acids Res.,* 2010, vol. 38, D532-539

- **CLINE MS ; SMOOT M ; CERAMI E ; KUCHINSKY A ; LANDYS N ; WORKMAN C et al.** Integration of biological networks and gene expression data using Cytoscape. *Nat Protoc.,* 2007, vol. 2, 2366-2382
- **GONCALVES JP ; GRAOS M ; VALENTE AX.** POLAR MAPPER: a computational tool for integrated visualisation of protein interaction networks and mRNA expression data. *J R Soc Interface,* 2009, vol. 6, 881-896

SEQUENCE LISTING

<110> Centre de Recherche Public de la Santé

<120> Compositions and methods for evaluating heart failure

<130> P11499.WO

<140> not yet known
<141> 27 November 2013

<150> LU 92106
<151> 27 November 2012
<150> US 61/730,459
<151> 27 November 2012

<160> 5

<170> PatentIn version 3.5

<210> 1
<211> 89
<212> RNA
<213> Homo sapiens microRNA 16 (MIR16)

<400> 1

```
gtcagcagtg ccttagcagc acgtaaatat tggcgttaag attctaaaat tatctccagt      60

attaactgtg ctgctgaagt aaggttgac                                        89
```

<210> 2
<211> 78
<212> RNA
<213> Homo sapiens microRNA 27a (MIR27A)

<400> 2

ctgaggagca gggcttagct gcttgtgagc agggtccaca ccaagtcgtg ttcacagtgg 60

ctaagttccg ccccccag 78

<210> 3
<211> 75
<212> RNA
<213> Homo sapiens microRNA 101 (MIR101)

<400> 3

tgccctggct cagttatcac agtgctgatg ctgtctattc taaaggtaca gtactgtgat 60

aactgaagga tggca 75

<210> 4
<211> 84
<212> RNA
<213> Homo sapiens microRNA 150 (MIR150)

<400> 4

ctccccatgg ccctgtctcc caacccttgt accagtgctg ggctcagacc ctggtacagg 60

cctgggggac agggacctgg ggac 84

<210> 5
<211> 134
<212> PRT
<213> Homo sapiens natriuretic peptides B preproprotein

<400> 5

```
Met Asp Pro Gln Thr Ala Pro Ser Arg Ala Leu Leu Leu Leu Leu Phe
1               5                   10                  15

Leu His Leu Ala Phe Leu Gly Gly Arg Ser His Pro Leu Gly Ser Pro
            20                  25                  30

Gly Ser Ala Ser Asp Leu Glu Thr Ser Gly Leu Gln Glu Gln Arg Asn
        35                  40                  45

His Leu Gln Gly Lys Leu Ser Glu Leu Gln Val Glu Gln Thr Ser Leu
    50                  55                  60

Glu Pro Leu Gln Glu Ser Pro Arg Pro Thr Gly Val Trp Lys Ser Arg
65                  70                  75                  80

Glu Val Ala Thr Glu Gly Ile Arg Gly His Arg Lys Met Val Leu Tyr
                85                  90                  95

Thr Leu Arg Ala Pro Arg Ser Pro Lys Met Val Gln Gly Ser Gly Cys
            100                 105                 110

Phe Gly Arg Lys Met Asp Arg Ile Ser Ser Ser Ser Gly Leu Gly Cys
        115                 120                 125

Lys Val Leu Arg Arg His
    130
```